(19) Europäisches Patentamt
European Patent Office
Office européen des brevets

(11) Veröffentlichungsnummer: **0 163 286**
**A2**

# (12) EUROPÄISCHE PATENTANMELDUNG

(21) Anmeldenummer: 85106527.6

(22) Anmeldetag: 28.05.85

(51) Int. Cl.[4]: C 07 K 9/00
A 61 K 37/02

(30) Priorität: 29.05.84 CH 2635/84

(43) Veröffentlichungstag der Anmeldung: 04.12.85 Patentblatt 85/49

(84) Benannte Vertragsstaaten: AT BE CH DE FR GB IT LI LU NL SE

(71) Anmelder: CIBA-GEIGY AG
Postfach
CH-4002 Basel(CH)

(72) Erfinder: Baschang, Gerhard, Dr.
Bückenweg 7
CH-4126 Bettingen(CH)

(72) Erfinder: Hartmann, Albert, Dr.
Steingasse 21A
D-7889 Grenzach(DE)

(72) Erfinder: Wacker, Oskar, Dr.
Löwenbergstrasse 60
CH-4059 Basel(CH)

(74) Vertreter: Zumstein, Fritz sen., Dr. et al,
Bräuhausstrasse 4
D-8000 München 2(DE)

## (54) Acylierte Zuckerderivate, Verfahren zu ihrer Herstellung und ihre Verwendung.

(57) Beschrieben sind Zuckerderivate der Formel I,

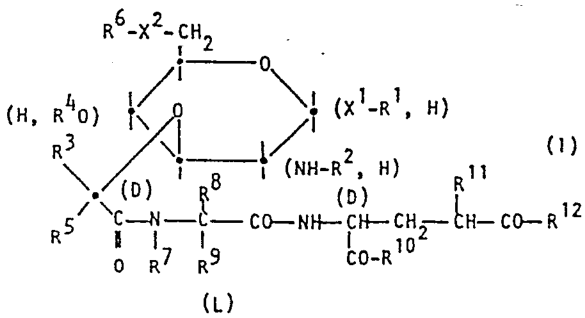

(I)

mit immunistimulierender Wirkung, die als erfinderisches Element mindestens einen Rest $A^1$ oder $A^2$ enthalten. Diese Reste $A^1$ und $A^2$, die Bestandteil der Reste $R^1$, $R^2$, $R^4$, $R^6$, $R^9$, $R^{10}$ oder $R^{12}$ gemäss Formel I sein können, sind folgendermassen difiniert: $A^1$ steht für durch Aryl, Heteroaryl oder Heteroarylthio substituiertes Niederalkanoyl, das zusätzlich durch einen Ethylenrest, der mit dem obengenannten Aryl- oder Heteroarylsubstituenten einen fünfgliedrigen Ring bildet, substituiert sein kann, oder für ortho- oder ortho/meta-substituiertes Aroyl. $A^2$ steht für durch Aryl, Heteroaryl oder Heteroarylthio substituiertes Niederalkoxy.

Beschrieben sind auch Verfahren zur Herstellung der Verbindungen der Formel I sowie neue Zwischenprodukte.

EP 0 163 286 A2

0163286

CIBA-GEIGY AG
Basel (Schweiz)

4-14895/+

# Acylierte Zuckerderivate, Verfahren zu ihrer Herstellung und ihre Verwendung

Die Erfindung betrifft Zuckerderivate der Formel I,

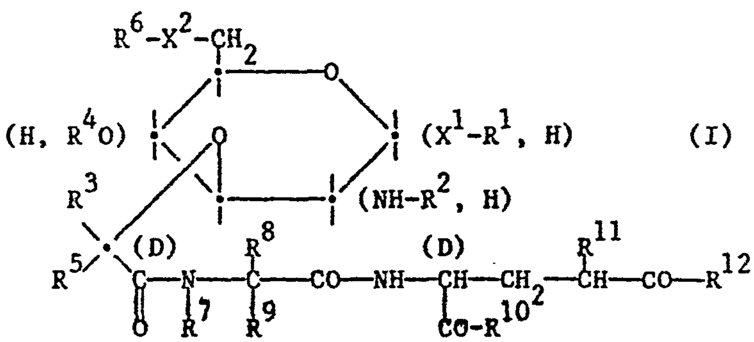

(I)

(L)

worin sich der Zuckerteil von D-Glucose, D-Mannose oder D-Galactose ableitet, $X^1$ Sauerstoff, Schwefel oder die Gruppe NH, $X^2$ Sauerstoff oder die Gruppe NH, $R^1$, $R^4$ und $R^6$ unabhängig voneinander Wasserstoff, Niederalkanoyl, einen Rest der Formel Ia,

$$\left[ \overset{O}{\overset{\|}{C}} - Y^1 - X^3 \right]_n - A^1 \qquad \text{(Ia)}$$

worin n für 0 oder 1, $Y^1$ für unsubstituiertes oder substituiertes Alkylen, das durch Carbonylimino oder Carbonyloxy unterbrochen sein kann, $X^3$ für Sauerstoff oder die Gruppe NH und $A^1$ für durch Aryl, Heteroaryl oder Heteroarylthio substituiertes Niederalkanoyl, das zusätzlich durch einen Ethylenrest, der mit dem obengenannten

Aryl- oder Heteroarylsubstituenten einen fünfgliedrigen Ring bildet, substituiert sein kann, oder für ortho- oder ortho/meta-substituiertes Aroyl stehen, oder einen Rest der Formel Ib,

$$-\left[\overset{O}{\overset{\|}{C}}-Y^1\right]_n-\overset{O}{\overset{\|}{C}}-A^2 \qquad \text{(Ib)}$$

worin n und $Y^1$ die obengenannten Bedeutungen haben und $A^2$ für durch Aryl, Heteroaryl oder Heteroarylthio substituiertes Niederalkoxy steht, oder $R^1$ auch unsubstituiertes oder substituiertes Benzyl, $R^2$ unsubstituiertes oder durch Hydroxy substituiertes Niederalkanoyl, unsubstituiertes oder substituiertes Benzoyl oder einen der obengenannten Reste der Formeln Ia oder Ib, $R^3$ Wasserstoff, Niederalkyl oder Cycloalkyl und $R^5$ Wasserstoff oder $R^3$ und $R^5$ zusammen Niederalkyliden, Cycloalkyliden oder unsubstituiertes oder substituiertes Benzyliden, $R^7$ Wasserstoff oder Niederalkyl oder $R^7$ und $R^9$ zusammen Trimethylen, $R^8$ Wasserstoff oder Niederalkyl, $R^9$ Wasserstoff oder Niederalkyl, das unsubstituiert oder durch Hydroxy, Mercapto, Niederalkylthio, Carboxy, Niederalkoxycarbonyl, Carbamoyl oder einen Rest der Formel Ic, Id, Ie oder If,

$$-X^4-\left[\overset{O}{\overset{\|}{C}}-Y^1-X^3\right]_q-A^1 \qquad \text{(Ic)}$$

$$-X^4-\left[\overset{O}{\overset{\|}{C}}-Y^1-\right]_q\overset{O}{\overset{\|}{C}}-A^2 \qquad \text{(Id)}$$

$$-\overset{O}{\overset{\|}{C}}-X^5-Y^2-X^6-A^1 \qquad \text{(Ie)}$$

$$-\overset{O}{\overset{\|}{C}}-\left[X^5-Y^2-\overset{O}{\overset{\|}{C}}\right]_q-A^2 \qquad \text{(If)}$$

worin q für 0 oder 1, $X^4$ für Sauerstoff oder Schwefel und $X^5$ und $X^6$ unabhängig voneinander für Sauerstoff oder die Gruppe NH stehen und die übrigen Substituenten die obengenannten Bedeutungen haben, substituiert ist, $R^{10}$ und $R^{12}$ unabhängig voneinander Niederalkoxy,

Hydroxy, Amino, Niederalkylamino, das durch Carboxy, Carbamoyl oder Niederalkoxycarbonyl substituiert ist und das zusätzlich durch Amino, Hydroxy, Carboxy, 2-Amino-ethylthio, 2-Amino-ethoxy und/oder die Sulfogruppe $-SO_3H$ substituiert sein kann, einen Rest der Formel Ig,

$$-X^5-Y^2-X^6-A^1 \qquad (Ig)$$

worin $Y^2$ für unsubstituiertes oder substituiertes Alkylen, worin eine Methylengruppe durch Sauerstoff, Schwefel oder Sulfinyl ersetzt sein kann und das durch Carbonylimino oder Carbonyloxy unterbrochen sein kann, steht, und worin $X^5$, $X^6$ und $A^1$ die obengenannten Bedeutungen haben, oder einen Rest der Formel Ih,

$$\left[-X^5-Y^2-\overset{O}{\overset{\|}{C}}\right]_q-A^2 \qquad (Ih)$$

worin q, $X^5$, $Y^2$ und $A^2$ die obengenannten Bedeutungen haben, und $R^{11}$ Wasserstoff, Carboxy, Niederalkoxycarbonyl oder Carbamoyl bedeuten, wobei die Verbindungen der Formel I mindestens einen und höchstens drei Reste $A^1$ und/oder $A^2$ aufweisen, und Salze von solchen Verbindungen mit mindestens einer salzbildenden Gruppe, Verfahren zur Herstellung dieser Verbindungen, diese Verbindungen enthaltende pharmazeutische Präparate und die Verwendung dieser Verbindungen in einem Verfahren zur therapeutischen Behandlung des menschlichen oder tierischen Körpers oder zur Herstellung pharmazeutischer Präparate.

In Formel I sind die Substituenten $-NH-R^2$ und $-OR^4$ α- oder ß-ständig und nehmen die Positionen der entsprechenden Hydroxygruppen von D-Glucose, D-Mannose oder D-Galactose ein.

Die Zuckerderivate der Formel I können in Form von Isomerengemischen oder reinen Isomeren vorliegen. Der Substituent $X^1-R^1$ ist α- und/oder ß-ständig. Die Konfiguration an den Atomen C-$R^3$, C-$R^9$ beziehungsweise C-CO-$R^{10}$ ist im Falle asymmetrischer Substitution (D), (L) beziehungsweise (D), wie in Formel I angegeben.

Niederalkanoyl $R^1$, $R^4$ und $R^6$ ist vorzugsweise Acetyl.

Alkylen ist ein bivalenter gesättigter Kohlenwasserstoffrest, hat vorzugsweise bis einschliesslich 18, in erster Linie bis einschliesslich 12, insbesondere bis einschliesslich 10, hauptsächlich bis einschliesslich 7 C-Atome und ist geradkettig oder verzweigt, wobei die beiden Bindungen von demselben C-Atom oder zwei beliebigen, voneinander verschiedenen C-Atomen, vorzugsweise den beiden endständigen C-Atomen der Kette, ausgehen. Der Begriff umfasst somit auch Alkylidenreste, deren zwei freie Valenzen von demselben C-Atom ausgehen müssen. Alkylen ist z.B. Niederalkyliden, wie Ethyliden, Propyliden oder 2-Methyl-propyliden, oder Mono- oder Oligomethylen, z.B. Methylen, Dimethylen, Trimethylen, Tetramethylen, Pentamethylen oder Hexamethylen.

Substituiertes Alkylen enthält insbesondere freies oder verestertes Carboxy, z.B. Benzyloxycarbonyl oder Niederalkoxycarbonyl, Amino, Niederalkanoylamino, Hydroxy und/oder Niederalkanoyloxy als Substituenten.

Carbonylimino bedeutet vorzugsweise die Gruppe -CO-NH-, aber auch die Gruppe -NH-CO-.

Carbonyloxy bedeutet vorzugsweise die Gruppe -CO-O-, aber auch die Gruppe -O-CO-.

Als Beispiele für substituiertes Alkylen $Y^1$ seien genannt: 2-Carboxy-ethyliden oder 2-Methyl-propyliden. Als Beispiele für substituiertes Alkylen $Y^2$ seien genannt: 1-Carboxy-di-, -tetra- oder -pentamethylen oder 2-Hydroxy-trimethylen. Als Beispiele für

gegebenenfalls substituierte Alkylen $Y^2$, worin eine Methylengruppe durch Sauerstoff, Schwefel oder Sulfinyl ersetzt sein kann und das durch Carbonylimino oder Carbonyloxy unterbrochen sein kann, seien genannt: 1-Carboxy-3-oxa-pentamethylen ($-CH(COOH)-CH_2-O-CH_2-CH_2-$), 1-Benzyloxycarbonyl-3-thia-pentamethylen, 4-(Ethylidencarbonylimino)-n-butyl [$-CH(CH_3)-CONH-(CH_2)_4-$], 3-(Ethylidencarbonylimino)-2-hydroxy-propyl, 2-Acetoxy-3-(ethylidencarbonylimino)-propyl, 3-[(4-Amino-pentyliden)-carbonylimino]-2-hydroxy-propyl oder 1-Benzyloxycarbonyl-2-(ethylen-1-sulfinyl)-dimethylen

$$(-CH(COO-CH_2-C_6H_5)-CH_2-S(=O)-CH_2-CH_2-) .$$

Entsprechend den obigen Definitionen bedeutet durch Carbonylimino unterbrochenes Alkylen z.B. den Rest $-CH(CH_3)-CO-NH-(CH_2)_4-$.

Aryl ist insbesondere substituiertes Phenyl oder Naphthyl, wobei als Substituenten des Phenylrestes insbesondere die folgenden zu nennen sind: Niederalkyl, insbesondere vorzugsweise para-ständiges 2-Methyl-propyl, substituiertes Niederalkyl, z.B. meta-ständiges Hydroxybenzyl, Halogen, z.B. Chlor oder Fluor, Hydroxy, Niederalkoxy, wie insbesondere Methoxy, Cycloalkyl, wie insbesondere Cyclohexyl, Phenyl, Heterocyclyl, wie insbesondere 1-Oxo-2-isoindolinyl oder 2,5-Dihydro-1H-pyrrol-1-yl, unsubstituiertes oder substituiertes Amino, wie insbesondere unsubstituiertes oder substituiertes Phenylamino, z.B. 2,6-Dichlor-phenyl-amino, 2,6-Dichlor-4-fluor-phenyl-amino oder 2,3-Dimethyl-phenyl-amino, und/oder aromatisches Acyl, wie insbesondere Benzoyl. Gegebenenfalls substituiertes Naphthyl ist insbesondere 6-Methoxy-naphth-2-yl.

Heteroaryl ist insbesondere unsubstituiertes oder vorzugsweise substituiertes, maximal ungesättigtes Heterocyclyl mit 6 oder vorzugsweise 5 Ringgliedern und 1 bis 4, vorzugsweise 1 oder 2, Heteroatomen, wie vorzugsweise Stickstoff, Sauerstoff und/oder Schwefel, wobei im Falle eines fünfgliedrigen Heterocyclus mit 2 Heteroatomen die Heteroatome vorzugsweise in 1,3-Stellung zueinander

stehen. Heteroaryl ist auch vorgenanntes Heterocyclyl mit 5 oder 6 Ringgliedern, das ein oder zwei ankondensierte und gegebenenfalls substituierte Phenylreste enthält, z.B. Carbazol-2-yl, insbesondere 6-Chlor-carbazol-2-yl, oder Indol-3-yl, insbesondere 1-(4-Chlor-benzoyl)-5-methoxy-2-methyl-indol-3-yl oder 1-Benzoyl-5-methoxy-2-methyl-indol-3-yl.

Heteroaryl ist ferner vorgenanntes Heterocyclyl mit 5 oder 6 Ringgliedern, das zusätzlich zu einem ankondensierten Phenylrest einen ankondensierten Pyridinrest enthält, z.B. 5H-[1]benzopyrano-[2,3-b]pyridin-7-yl.

Heteroaryl mit einem Heteroatom ist vorzugsweise z.B. Pyrrolyl, das z.B. durch Niederalkyl, wie Methyl, und/oder aromatisches Acyl, z.B. Benzoyl oder 4-Methyl-benzoyl, substituiert sein kann, z.B. 1-Methyl-5-(4-methyl-benzoyl)-pyrrol-2-yl.

Als bevorzugte Heteroarylreste mit zwei Heteroatomen seien unsubstituiertes oder vorzugsweise substituiertes 1,3-Oxazol-2-yl, 1,3-Diazol-2-yl oder 1,3-Thiazol-2-yl genannt. Substituenten der obengenannten Heteroarylreste sind vorzugsweise Phenylreste, die unsustituiert oder, z.B. durch Methoxy, substituiert sind, wie z.B. 4-Methoxy-phenyl, wobei ein Heteroarylrest vorzugsweise durch 2, vorzugsweise zueinander ortho-ständige, solche Phenylreste substituiert ist. Substituiertes Heteroaryl ist folglich z.B. 4,5-Di-(4-methoxy-phenyl)-1,3-oxazol-2-yl, -diazol-2-yl oder -thiazol-2-yl.

Heteroarylthio weist die obengenannten Heteroarylreste auf und ist z.B. wie oben geschildert substituiertes 1,3-Oxazol-2-thio, 1,3-Diazol-2-thio oder 1,3-Thiazol-2-thio, z.B. 4,5-Di-(4-methoxy-phenyl)-1,3-thiazol-2-thio.

Substituiertes Niederalkanoyl $A^1$ ist vorzugsweise substituiertes Acetyl, Propionyl oder 2-Methyl-propionyl, und die Substituenten befinden sich vorzugsweise in 2-Stellung.

Der einen Niederalkanoylrest $A^1$ zusätzlich substituierende Ethylenrest befindet sich ebenfalls vorzugsweise in 2-Stellung.

Zusätzlich durch einen Ethylenrest, der mit dem Arylsubstituenten, wie z.B. Phenylsubstituenten, einen fünfgliedrigen Ring bildet, substituiertes Niederalkanoyl $A^1$ ist vorzugsweise Hydrinden-1-carbonyl (Indan-1-carbonyl), z.B. 5-Cyclohexyl-6-chlor-hydrinden-1-carbonyl.

Zusätzlich durch einen Ethylenrest, der mit dem Heteroarylsubstituenten, wie z.B. Pyrrol-, Furan- oder Thiophensubstituenten, einen fünfgliedrigen Ring bildet, substituiertes Niederalkanoyl $A^1$ ist, z.B. 3H-1,2-dihydropyrrolo[a]-pyrrol-1-carbonyl, z.B. (d,l)-5-Benzoyl-3H-1,2-dihydropyrrolo[a]-pyrrol-1-carbonyl der Formel Iaα.

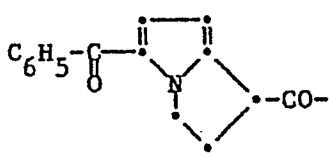

(Iaα)

Ortho-substituiertes Aroyl $A^1$ ist vorzugsweise durch eine unsubstituierte oder, vorzugsweise, substituierte Aminogruppe ortho-substituiertes Benzoyl, wobei die Aminogruppe vorzugsweise durch einen Phenylrest, z.B. einen einen Niederalkylsubstituenten, wie insbesondere Methylsubstituenten, tragenden Phenylrest substituiert ist. Ortho-substituiertes Aroyl ist folglich z.B. 2-(2,3-Dimethylphenyl)-amino-benzoyl. Ortho/meta-substituiertes Aroyl $A^1$ ist vorzugsweise Benzoyl, welches in ortho-Stellung, vorzugsweise 2-Stellung, durch Hydroxy und in meta-Stellung, vorzugsweise 5-Stellung, durch unsubstituiertes oder substituiertes, vorzugsweise durch Halogen, z.B. Fluor, substituiertes Phenyl substituiert ist. Ortho/meta-substituiertes Aroyl ist folglich z.B. 5-(2,4-Difluorphenyl)-2-hydroxy-benzoyl.

Substituiertes Niederalkoxy $A^2$ ist vorzugsweise substituiertes Ethoxy, insbesondere 2-substituiertes Ethoxy, substituiertes Propoxy, insbesondere 3-substituiertes Propoxy, oder substituiertes 2-Methyl-propoxy, insbesondere 2-substituiertes 2-Methyl-propoxy.

Substituiertes Benzyl $R^1$ ist insbesondere im Phenylteil durch Niederalkyl, Hydroxy, Niederalkoxy oder Halogen substituiertes Benzyl.

Niederalkanoyl $R^2$ ist vorzugsweise Acetyl oder Propionyl. Durch Hydroxy substituiertes Niederalkanoyl $R^2$ trägt die Hydroxygruppe vorzugsweise in 2-Stellung und ist insbesondere Glykolyl.

Substituiertes Benzoyl $R^2$ trägt insbesondere Niederalkyl, Niederalkoxy, Halogen, Niederalkanoyloxy und/oder Niederalkanoylamino als Substituenten des Phenylteils.

Bevorzugterweise ist $R^2$ unsubstituiertes Benzoyl.

Niederalkyl $R^3$ ist vorzugsweise $C_{1-3}$-Alkyl, insbesondere Methyl, Ethyl oder n-Propyl.

Niederalkyliden $R^3 + R^5$ ist vorzugsweise unsubstituiertes oder durch $C_{1-3}$-Alkyl substituiertes Methyliden, z.B. Methyliden, Ethyliden, n-Propyliden oder n-Butyliden, oder gegebenenfalls substituiertes Benzyliden, z.B. im Phenylrest halogeniertes oder durch Niederalkyl substituiertes Benzyliden, wie 4-Chlor-benzyliden oder 4-Methylbenzyliden.

Cycloalkyliden $R^3 + R^5$ ist vorzugsweise Cyclopentyliden oder Cyclohexyliden.

Niederalkyl $R^7$ ist vorzugsweise $C_{1-3}$-Alkyl, in erster Linie Methyl.

Niederalkyl $R^9$ ist vorzugsweise Methyl, Ethyl, Isopropyl, 2-Methylpropyl oder sek.-Butyl.

Durch Hydroxy, Mercapto, Niederalkylthio, Carboxy oder Carbamoyl substituiertes Niederalkyl $R^9$ ist vorzugsweise entsprechend substituiertes $C_{1-2}$-Alkyl, z.B. Hydroxymethyl, 1-Hydroxy-ethyl, Mercaptomethyl, 2-Methylthio-ethyl, Carboxy-methyl, 2-Carboxy-ethyl, Carbamoyl-methyl oder 2-Carbamoyl-ethyl.

Niederalkoxycarbonyl $R^{11}$ ist vorzugsweise ($C_{1-4}$-Alkoxy)-carbonyl, z.B. Methoxycarbonyl, Ethoxycarbonyl oder n-Butyloxycarbonyl.

Niederalkoxy $R^{10}$ oder $R^{12}$ ist vorzugsweise $C_{1-4}$-Alkoxy, z.B. Methoxy, Ethoxy oder n-Butoxy.

Durch Carboxy, Carbamoyl oder Niederalkoxycarbonyl substituiertes Niederalkylamino $R^{10}$ oder $R^{12}$, das zusätzlich durch Amino, Hydroxy, Carboxy, 2-Amino-ethylthio, 2-Amino-ethoxy und/oder die Sulfogruppe $-SO_3H$ substituiert sein kann, ist vorzugsweise in 1-Stellung durch Carboxy, Carbamoyl oder Niederalkoxycarbonyl substituiert und ist z.B. derart in 1-Stellung substituiertes Ethylamino, n-Propylamino, 2-Methyl-propyl-amino, 3-Methyl-butyl-amino, 2-Methyl-butyl-amino, 5-Amino-pentyl-amino, 4-Amino-butyl-amino, 2-Hydroxy-ethyl-amino, 2-Carboxy-ethyl-amino, 2-Carboxy-propyl-amino, 6-Amino-6-carboxy-ethyl-amino, 2-(2-Amino-ethylthio)-ethylamino, 2-(2-Amino-ethoxy)-ethyl-amino oder 2-Sulfo-ethyl-amino, wobei die obengenannten substituierten Niederalkylaminoreste $R^{10}$ oder $R^{12}$, die sich von Alanin, α-Amino-buttersäure, Valin, Leucin, Isoleucin, Lysin, Thialysin und Oxalysin ableiten, vorzugsweise die (L)-Konfiguration haben.

Alkylen $Y^2$, worin eine Methylengruppe durch Sauerstoff oder Schwefel ersetzt ist, ist vorzugsweise 3-Oxa-pentamethylen oder 3-Thia-pentamethylen.

Salzbildende Gruppen in einer Verbindung der Formel I sind saure Gruppen, z.B. freie Carboxyl- oder Sulfonsäuregruppen, oder basische Gruppen, wie insbesondere freie Aminogruppen. Je nach der Art der

salzbildenden Gruppe bilden die Verbindungen der Formel I Metall- oder Ammoniumsalze oder Säureadditionssalze. Salze einer Verbindung der Formel I sind vorzugsweise pharmazeutisch-verwendbar und nicht-toxisch, z.B. Alkalimetall- oder Erdalkalimetallsalze, z.B. Natrium-, Kalium-, Magnesium- oder Calciumsalze, oder Salze mit Ammoniak oder geeigneten organischen Aminen, wobei in erster Linie aliphatische, cycloaliphatische, cycloaliphatisch-aliphatische oder araliphatische primäre, sekundäre oder tertiäre Mono-, Di- oder Polyamine, sowie heterocyclische Basen für die Salzbildung in Frage kommen, wie Niederalkylamine, z.B. Triethylamin, Hydroxyniederalkylamine, z.B. 2-Hydroxy-ethylamin, Bis-(2-hydroxyethyl)-amin, 2-Hydroxy-ethyl-diethyl-amin oder Tri-(2-hydroxyethyl)-amin, basische aliphatische Ester von Carbonsäuren, z.B. 4-Amino-benzoesäure-2-diethylaminoethylester, Niederalkylenamine, z.B. 1-Ethylpiperidin, Cycloalkylamine, z.B. Dicyclohexylamin, oder Benzylamine, z.B. N,N'-Dibenzylethylendiamin, ferner Basen vom Pyridintyp, z.B. Pyridin, Collidin oder Chinolin. Verbindungen der Formel I mit mindestens einer basischen Gruppe können Säureadditionssalze, z.B. mit anorganischen Säuren, wie Salzsäure, Schwefelsäure oder Phosphorsäure, oder mit geeigneten organischen Carbon- oder Sulfonsäuren, z.B. Trifluoressigsäure, sowie mit Aminosäuren, wie Arginin und Lysin, bilden. Bei Anwesenheit von mehreren sauren oder basischen Gruppen können Mono-oder Polysalze gebildet werden. Verbindungen der Formel I mit einer sauren, z.B. freien Carboxylgruppe, und einer freien basischen, z.B. einer Aminogruppe, können auch in Form von inneren Salzen, d.h. in zwitterionischer Form vorliegen, oder es kann ein Teil des Moleküls als inneres Salz, und ein anderer Teil als normales Salz vorliegen.

Zur Isolierung oder Reinigung können auch pharmazeutisch ungeeignete Salze Verwendung finden. Zur therapeutischen Anwendung gelangen jedoch nur die pharmazeutisch verwendbaren, nicht-toxischen Salze, die deshalb bevorzugt werden.

Die Aufgabe der vorliegenden Erfindung ist die Bereitstellung neuer, pharmakologisch wirksamer Verbindungen mit einem hinsichtlich der Gesamtheit ihrer pharmakologischen Wirkungen neuartigen Wirkungsprofil.

Die Verbindungen der Formel I besitzen wertvolle pharmakologische Eigenschaften, insbesondere eine ausgeprägte immunomodulierende, in erster Linie immunostimulierende, Wirkung.

Die Verbindungen der Formel I steigern in vivo auch Manifestationen der zellvermittelten Immunität, wie z.B. aus folgendem Test hervorgeht, in dem die Steigerung der Spättyp-Ueberempfindlichkeit (Delayed Type Hypersensitivity) gegen bovines Serumalbumin (BSA) beim Meerschweinchen ermittelt wird:
Pirbright Meerschweinchen werden am Tag 0 mit 1 mg BSA und verschiedenen Mengen einer Verbindung der Formel I in inkomplettem Freund'schen Adjuvans durch Injektion von je 0,1 ml dieses Antigen-Adjuvans-Gemisches in die beiden Hinterpfoten immunisiert. Drei Wochen später werden Hautreaktionen durch intrakutane Injektion von 100 µg BSA in 0,1 ml gepufferter physiologischer Kochsalzlösung (PBS) in die enthaarte Flanke des Tieres ausgelöst und aufgrund des 24-48 Stunden danach anhand der Erythemfläche und der Hautdickenzunahme berechneten Reaktionsvolumens quantifiziert. Nach 24-48 Stunden (Spättypreaktion) beobachtet man eine signifikante antigenspezifische Zunahme des Reaktionsvolumens, die als ein Mass für zellvermittelte Immunität gilt. Die $ED_{50}$-Werte für die Verbindungen der Formel I liegen im obengenannten Test im Bereich zwischen 1 und 300 µg/Tier.

Die Verbindungen der Formel I steigern auch die Mitogenität, wie z.B. aus folgendem Test hervorgeht:

Suspensionen hoch angereicherter B-Lymphozyten (Milzzellen kongenital athymischer nu/nu Mäuse) werden in Gegenwart einer Verbindung der Formel I während 3 Tagen in 0,2 ml-Kulturen in einem $CO_2$-Brutschrank inkubiert. Als Mass für die Proliferationsaktivität

bestimmt man die Inkorporation von $^3$H-Thymidin in die Lymphozyten während der letzten 18 Stunden der Kulturperiode. Daraus ergibt sich, dass Verbindungen der Formel I bereits in Dosen von 200 Nanogramm/ml mitogen sind für B-Lymphozyten.

Die Verbindung der Formel I besitzen ausserdem Antitumoreigenschaften. Diese beruhen auf ihrer Fähigkeit, z.B. inkorporiert in multilamellären Liposomen oder in phosphatgepufferter physiologischer Kochsalzlösung (PBS), Makrophagen derart zu aktivieren, dass diese körpereigenen Abwehrzellen in der Lage sind, Tumorzellen abzutöten (Zytotoxizität) oder an ihrem Wachstum zu hindern (Zytostase). Die Induktion tumorizidaler und tumoristatischer Alveolarmakrophagen der Ratte in vitro und in situ lässt sich z.B. mit folgendem Versuch zeigen:

Alveolarmakrophagen werden durch Lungenspülung mit Kulturmedium erhalten. Diese Makrophagen werden entweder durch Injektion der Testsubstanzen in die Ratten (intravenös oder intranasal, in situ-Aktivierung) oder durch eine 24stündige Vorinkubation mit einer Verbindung der Formel I im $CO_2$-Inkubator (in vitro-Aktivierung) aktiviert. Die so aktivierten Makrophagen werden nun für weitere 72 Stunden mit Tumorzellen inkubiert.

Um tumorizidale Aktivitäten der Makrophagen zu messen, werden die Tumorzellen vor der 72 Stunden-Inkubation mit $^{125}$I-Iododeoxyuridin markiert. Die nicht abgetöteten Tumorzellen können nach Wegwaschen der durch lysierte Tumorzellen freigewordenen Radioaktivität anhand der verbleibenden Radioaktivität gemessen werden.

Um tumoristatische Aktivitäten der Makrophagen zu erfassen, wird den Kulturen 8 Stunden vor dem Ende der 72 Stunden-Inkubation $^3$H-Thymidin zugesetzt und danach die $^3$H-Thymidininkorporation in die Tumorzellen gemessen. In vitro können die Substanzen sowohl gelöst in PBS als auch inkorporiert in Liposomen bereits in Dosen von 20 Nanogramm/0,2 ml Kultur tumorizidale Ratten-Alveolarmakrophagen induzieren. Bei Ratten bewirkt eine einmalige intravenöse Applikation der Verbindungen inkorporiert in Liposomen bei einer Dosis von 160 µg/Tier eine Induktion von tumorizidalen und tumoristatischen Alveolarmakrophagen. Darüberhinaus bewirkt eine einmalige intranasale Applikation der Substanzen in PBS bei einer Dosis von 25 µg/Ratte die Induktion von tumoristatischen Alveolarmakrophagen.

Im Vergleich zu bekannten Muramylpeptiden weisen die Verbindungen der Formel I stark reduzierte unerwünschte Nebenwirkungen auf, insbesondere sind sie viel weniger pyrogen. Dieser Befund ist von grosser Bedeutung, da die Applikation einer pyrogenen Substanz unter Umständen einen thermischen Schock herbeiführen kann, weshalb eine sichere Anwendbarkeit nur unter ständiger ärztlicher Aufsicht gewährleistet ist und bestimmte Verabreichungsformen, wie die intravenöse Gabe, ausser Betracht bleiben müssen.

Die Prüfung auf Pyrogenität kann nach der in Europäische Pharmakopöe, Band 2, Seiten 56-59 (1971) gegebenen Vorschrift an Kaninchen durchgeführt werden. Hiernach ist z.B. N-Propionyl-desmethyl-muramyl-L-alanyl-D-isoglutaminyl-L-alanin-3-{2-[2-(2,6-dichlor-phenyl-amino)-phenyl]-acetylamino}-2-hydroxy-propylamid in einer Dosis von 5 mg/kg i.v. nicht pyrogen.

Die Verbindungen der Formel I können somit insbesondere als Immunstimulantien bei Warmblütern einschliesslich des Menschen verwendet werden, z.B. als Adjuvantien bei der Entwicklung neuer oder der Verbesserung konventioneller Impfstoffe sowie zur Therapie von Tumorerkrankungen.

Diejenigen Verbindungen der Formel I, worin $R^1$, $R^4$ und $R^6$ Nieder-alkanoyl bedeuten, z.B. 1,4,6-Tri-O-acetyl-N-propionyl-desmethyl-muramyl-L-alanyl-D-isoglutaminyl-L-alanin-{2-acetoxy-3-[d-2-(6-methoxy-naphth-2-yl)-propionylamino]-propyl}-amid, oder worin $R^1$ gegebenenfalls substituiertes Benzyl bedeutet, sind ausserdem überraschenderweise auch zur Prophylaxe und Therapie von Virusinfektionen hervorragend geeignet, wie sich aus Tierversuchen ergibt.

In diesen Tierversuchen werden Tiere, wie Mäuse oder Meerschweinchen, mit den verschiedensten Virusarten in einer Dosis, die für alle oder die grosse Mehrzahl der unbehandelten (Kontroll)Tiere letal ist, z.B. $LD_{80-90}$, infiziert und der Infektionsverlauf bei den unbehandelten Kontrolltieren im Vergleich zu Tieren beobachtet, die vor, gleichzeitig mit oder nach der Infektion mit einer der obengenannten Verbindungen oder einem Salz davon behandelt werden.

Dabei zeigt sich, dass ein prophylaktischer Effekt bei Verabreichung der Verbindungen der Formel I, worin $R^1$, $R^4$ und $R^6$ Niederalkanoyl oder $R^1$ gegebenenfalls substituiertes Benzyl bedeuten, schon mehrere Tage bis zu einigen, z.B. vier, Wochen vor der Infektion, und ein therapeutischer Effekt noch bei Verabreichung mehrere Tage, z.B. 1 Woche, nach der Infektion, eintritt.

Diese Verbindungen der Formel I sind im obengenannten Test an der Maus bereits im Dosisbereich zwischen 0,0001 mg/kg und 0,1 mg/kg wirksam.

Bemerkenswert ist auch das breite virale Spektrum, gegen das die obengenannten Verbindungen wirksam sind.

Die Verbindungen der Formel I, worin $R^1$, $R^4$ und $R^6$ Niederalkanoyl oder $R^1$ gegebenenfalls substituiertes Benzyl bedeuten, können insbesondere zur Prophylaxe und Therapie von Krankheiten verwendet werden, die durch die nachstehend näher bezeichneten Viren hervorgerufen werden [zur Nomenklatur vgl. J.L.Melnick, Prog. med. Virol. 26, 214-232 (1980) und 28, 208-221 (1982)]:

DNA-Viren mit kubischer Symmetrie und nacktem Nukleokapsid, DNA-Viren mit umhülltem Virion sowie RNA-Viren mit kubischer und solche mit helikaler Symmetrie des Kapsids.

Bevorzugterweise verwendet man diese Verbindungen der Formel I im Falle von DNA-Viren mit umhülltem Virion und kubischer Symmetrie des Kapsids, im Falle von RNA-Viren mit kubischer Symmetrie des Kapsids und nacktem Virion und im Falle von RNA-Viren mit helikaler Symmetrie des Kapsids, in denen die Nukleokapsidhülle bei der Oberflächenmembran gelegen ist, aber auch im Falle von Adenoviridae, Poxviridae und Coronaviridae, wie insbesondere menschlichen Coronaviren.

In erster Linie verwendet man diese Verbindungen der Formel I im Falle von Herpesviridae, Picornaviridae und Myxoviren, aber auch im Falle von Mastadenoviren, wie insbesondere menschlichen Adenoviren, im Falle von Chordopoxvirinae, wie hauptsächlich Orthopoxviren, wie insbesondere z.B. Vacciniaviren, im Falle von Reoviridae, vornehmlich (insbesondere menschlichen) Rotaviren, sowie im Falle von Caliciviridae und Rhabdoviridae, wie in erster Linie Vesiculoviren des Menschen sowie von Pferden, Rindern und Schweinen.

Hauptsächlich verwendet man diese Verbindungen der Formel I im Falle von Alphaherpesvirinae, wie Varicellaviren, z.B. menschlichen Varicella-Zoster Viren, Rhinoviren, Cardioviren und Orthomyxoviridae, aber auch im Falle von Betaherpesvirinae, wie insbesondere menschlichen Cytomegaloviren, im Falle von Aphthoviren, in erster Linie Apthoviren von Paarhufern, wie hauptsächlich von Rindern, sowie im Falle von Paramyxoviridae, wie in erster Linie Pneumoviren, z.B. respiratorischen Syncitialviren des Menschen, und wie daneben Morbilliviren oder Paramyxoviren, wie Parainfluenzaviren, z.B. menschlichen Parainfluenzaviren, einschliesslich der Sendaiviren sowie im Falle von Arboviren oder Vesiculoviren, z.B. Vesicular stomatitis Viren.

In allererster Linie verwendet man diese Verbindungen der Formel I im Falle von Simplexviren, z.B. menschlichen Herpes simplex Viren der Typen 1 und 2, im Falle von menschlichen Encephalomyocarditisviren, im Falle von Influenzaviren, wie hauptsächlich Influenza A und Influenza B Viren, im Falle von Vaccinia und Parainfluenza Viren und ganz besonders im Falle der in den Beispielen genannten Viren.

Diese Verbindungen der Formel I können zur Prophylaxe und Therapie von Virusinfektionen, insbesondere bei Warmblütern einschliesslich des Menschen, verwendet werden, indem man sie enteral oder parenteral, in erster Linie zusammen mit geeigneten Hilfs- oder Trägerstoffen, appliziert. Bevorzugterweise appliziert man sie auf die Schleimhaut, z.B. intranasal, rektal, vaginal oder auf die Bindehaut des Auges, oder oral. Der antivirale Effekt tritt jedoch auch bei Applikation auf anderen Wegen, z.B. subkutan, intravenös, intramuskulär oder bei Applikation auf die normale Haut ein.

Die Dosierung des Wirkstoffes hängt u.a. von der Warmblüterspezies, der Abwehrlage des Organismus, der Applikationsweise und der Art des Virus ab. Die Dosis-Wirkungsbeziehung ist relativ schwach ausgeprägt.

Zur Vorbeugung appliziert man eine einmalige Dosis von etwa 0,01 mg bis etwa 10 mg, vorzugsweise 0,05 bis 1 mg, z.B. 0,2 mg, Wirkstoff an einen Warmblüter von etwa 70 kg Körpergewicht, z.B. den Menschen. Die prophylaktische Wirkung dieser Dosis erstreckt sich über mehrere Wochen. Bei Bedarf, z.B. in Zeiten erhöhter Ansteckungsgefahr, kann man die Verabreichung dieser Dosis wiederholen.

Die therapeutische Dosis für Warmblüter von etwa 70 kg Körpergewicht liegt zwischen 0,1 mg und 25 mg, vorzugsweise zwischen 0,1 und 1 mg, z.B. bei 0,5 mg, insbesondere bei oraler Applikation. Die Dosierung bei topischer, insbesondere intranasaler Applikation, liegt bis zum Faktor 10 niedriger. Bei Bedarf kann man die Verabreichung dieser Verbindungen der Formel I bis zum Eintritt einer Besserung der Erkrankung wiederholen. Oft genügt jedoch eine einmalige Applikation.

Die Erfindung betrifft insbesondere Zuckerderivate der Formel I, worin sich der Zuckerteil von D-Glucose, D-Mannose oder D-Galactose ableitet, $X^1$ Sauerstoff, Schwefel oder die Gruppe NH, $X^2$ Sauerstoff oder die Gruppe NH, $R^1$, $R^4$ und $R^6$ unabhängig voneinander Wasserstoff, Niederalkanoyl, einen Rest der Formel Ia,

$$-\left[\overset{O}{\overset{\|}{C}}-Y^1-X^3\right]_n-A^1 \qquad (Ia)$$

worin n für 0 oder 1, $Y^1$ für unsubstituiertes oder substituiertes Alkylen, das durch Carbonylimino oder Carbonyloxy unterbrochen sein kann, $X^3$ für Sauerstoff oder die Gruppe NH und $A^1$ für durch Aryl, Heteroaryl oder Heteroarylthio substituiertes Niederalkanoyl, das zusätzlich durch einen Ethylenrest, der mit dem obengenannten Aryl- oder Heteroarylsubstituenten einen fünfgliedrigen Ring bildet, substituiert sein kann, oder für ortho- oder ortho/meta-substituiertes Aroyl stehen, oder einen Rest der Formel Ib,

$$-\left[\overset{O}{\overset{\|}{C}}-Y^1\right]_n-\overset{O}{\overset{\|}{C}}-A^2 \qquad (Ib)$$

worin n und $Y^1$ die obengenannten Bedeutungen haben und $A^2$ für durch Aryl, Heteroaryl oder Heteroarylthio substituiertes Niederalkoxy steht, oder $R^1$ auch unsubstituiertes oder substituiertes Benzyl, $R^2$ unsubstituiertes oder durch Hydroxy substituiertes Niederalkanoyl, unsubstituiertes oder substituiertes Benzyl oder einen der obengenannten Reste der Formeln Ia oder Ib, $R^3$ Wasserstoff, Niederalkyl oder Cycloalkyl und $R^5$ Wasserstoff oder $R^3$ und $R^5$ zusammen Niederalkyliden, Cycloalkyliden oder unsubstituiertes oder substituiertes Benzyliden, $R^7$ Wasserstoff oder Niederalkyl oder $R^7$ und $R^9$ zusammen Trimethylen, $R^8$ Wasserstoff oder Niederalkyl, $R^9$ Wasserstoff oder Niederalkyl, das unsubstituiert oder durch Hydroxy, Mercapto, Niederalkylthio, Carboxy, Niederalkoxycarbonyl, Carbamoyl oder einen Rest der Formel Ic, Id, Ie oder If,

$$-X^4-\left[\overset{O}{\overset{\|}{C}}-Y^1-X^3\right]_q-A^1 \qquad \text{(Ic)}$$

$$-X^4-\left[\overset{O}{\overset{\|}{C}}-Y^1\right]_q-\overset{O}{\overset{\|}{C}}-A^2 \qquad \text{(Id)}$$

$$-\overset{O}{\overset{\|}{C}}-X^5-Y^2-X^6-A^1 \qquad \text{(Ie)}$$

$$-\overset{O}{\overset{\|}{C}}-\left[X^5-Y^2-\overset{O}{\overset{\|}{C}}\right]_q-A^2 \qquad \text{(If)}$$

worin q für 0 oder 1, $X^4$ für Sauerstoff oder Schwefel und $X^5$ und $X^6$ unabhängig voneinander für Sauerstoff oder die Gruppe NH stehen und die übrigen Substituenten die obengenannten Bedeutungen haben, substituiert ist, $R^{10}$ und $R^{12}$ unabhängig voneinander Niederalkoxy, Hydroxy, Amino, Niederalkylamino, das durch Carboxy, Carbamoyl oder Niederalkoxycarbonyl substituiert ist und das zusätzlich durch Amino, Hydroxy, Carboxy, 2-Amino-ethylthio, 2-Amino-ethoxy und/oder die Sulfogruppe $-SO_3H$ substituiert sein kann, einen Rest der Formel Ig,

$$-X^5-Y^2-X^6-A^1 \qquad \text{(Ig)}$$

worin $Y^2$ für unsubstituiertes oder substituiertes Alkylen, worin eine Methylengruppe durch Sauerstoff, Schwefel, oder Sulfinyl ersetzt sein kann und das durch Carbonylimino oder Carbonyloxy unterbrochen sein kann, steht, und worin $X^5$, $X^6$ und $A^1$ die obengenannten Bedeutungen haben, oder einen Rest der Formel Ih,

$$\left[-X^5-Y^2-\overset{O}{\overset{\|}{C}}\right]_q-A^2 \qquad \text{(Ih)}$$

worin q, $X^5$, $Y^2$ und $A^2$ die obengenannten Bedeutungen haben, und $R^{11}$ Wasserstoff, Carboxy, Niederalkoxycarbonyl oder Carbamoyl bedeuten, wobei die Verbindungen der Formel I mindestens einen und höchstens

drei Reste $A^1$ und/oder $A^2$ aufweisen, und Salze von solchen Verbindungen mit mindestens einer salzbildenden Gruppe.

Bevorzugt sind Verbindungen der Formel I, worin sich der Zuckerteil von D-Glucose ableitet, das heisst, worin die Substituenten $-NH-R^2$ und $-OR^4$ α-ständig sind, und Salze von solchen Verbindungen mit mindestens einer salzbildenden Gruppe.

Bevorzugt sind auch die obengenannten Verbindungen der Formel I, worin $R^3$ Wasserstoff, Niederalkyl oder, zusammen mit $R^5$, gegebenenfalls durch $C_{1-3}$-Alkyl substituiertes Methyliden oder gegebenenfalls substituiertes Benzyliden bedeutet, insbesondere derartige Verbindungen, worin $R^2$ für Benzoyl steht.

Die Erfindung betrifft insbesondere die oben genannten Zuckerderivate der Formel I, worin $A^1$ für den Acylrest einer Carbonsäure, ausgewählt aus der Gruppe, bestehend aus 6-Chlor-5-cyclohexyl-indan-1-carbonsäure (Clindanac), 2-[4,5-bis-(4-Methoxy-phenyl)-oxazol-2-yl]-propionsäure, 2-(5-Chlor-4-cyclohexyl-2-hydroxy-phenyl)-esigsäure, 2-[4,5-bis-(4-Methoxy-phenyl)-oxazol-2-yl]-2-methyl-propionsäure, 2-(3-Fluor-4-phenyl-phenyl)-propionsäure (Flurbiprofen), (±)-5-Benzoyl-3H-1,2-dihydropyrrolo[a]-pyrrol-1-carbonsäure, 2-[4-(1,3-Dihydro-1-oxo-2H-isoindol-2-yl)-phenyl]-propionsäure (Indoprofen), 2-{2-[(2,6-Dichlor-phenyl)-amino]-phenyl}-essigsäure (Diclofenac), 2-{2-[(2,6-Dichlor-4-fluor-phenyl)-amino]-5-fluor-phenyl}-essigsäure , 2-(2,3-Dimethyl-phenyl)-amino--benzoesäure, 2-[4,5-bis-(4-Methoxy-phenyl)-imidazol-2-yl]-2-methyl-propionsäure, 2-{2-[(2,6-Dichlor-phenyl)-amino]-5-fluor-phenyl}-essigsäure, 2-(3-Benzoyl-phenyl)-propionsäure (Ketoprofen), 2-S-[4,5-bis-(4-Methoxy-phenyl)-thiazol-2-yl]-mercaptoessigsäure, 3-S-[4,5-bis-(4-Methoxy-phenyl)-thiazol-2-yl]-mercaptopropionsäure, 2-{2-[(2,6-Dichlor-4-fluor-phenyl)-amino]-phenyl}-essigsäure, 5-(2,4-Difluor-phenyl)-2-hydroxy-benzoesäure (Diflunisal), 2-(6-Chlor-9H-carbazol-2-yl)-propionsäure (Carprofen), 2-(4-Isobutyl-phenyl)-propionsäure (Ibuprofen), 1-(4-Chlor-benzoyl)-5-methoxy-2-methyl-indol-3-yl-essigsäure (Indomethacin), 2-(6-Methoxy-naphth-2-yl)-propionsäure (Naproxen), 2-[3-Chlor-4-(3-pyrrolin-1-yl)-phenyl]-propionsäure (Pirprofen), 2-(5H-[1]benzopyrano[2,3-b]-pyridin-7-yl)-propionsäure (Pranoprofen), 5-(4-Methyl-benzoyl)-1-methyl-pyrrol-2-yl-essigsäure (Tolmetin) und 2-[4,5-bis-(4-Methoxy-phenyl)-oxazol-2-yl]-essigsäure, steht, und Salze von solchen Verbindungen mit mindestens einer salzbildenden Gruppe.

Die Erfindung betrifft vornehmlich die obengenannten Verbindungen der Formel I, worin $A^2$ für substituiertes Niederalkoxy, ausgewählt aus der Gruppe, bestehend aus 2-[4,5-bis-(4-Methoxy-phenyl)-thiazol-2-yl-thio]-ethoxy, 2-[4,5-bis-(4-Methoxy-phenyl)-imidazol-2-yl]-2-methyl-propoxy und 3-[4,5-bis-(4-Methoxy-phenyl)-thiazol-2-yl-thio]-propoxy, steht, und Salze von solchen Verbindungen mit mindestens einer salzbildenden Gruppe.

Die Erfindung betrifft hauptsächlich die obengenannten Verbindungen der Formel I, worin $X^1$ und $X^2$ Sauerstoff, $R^2$ unsubstituiertes oder durch Hydroxy substituiertes $C_{2-4}$-Alkanoyl oder einen Rest der wie oben definierten Formeln Ia oder Ib, $R^3$ Wasserstoff oder Niederalkyl, $R^5$, $R^7$ und $R^8$ Wasserstoff, $R^9$ Niederalkyl, das unsubstituiert oder durch Hydroxy, Mercapto, Methylthio oder einen Rest der wie oben definierten Formeln Ic, Id, Ie oder If substituiert ist, $R^{10}$ Hydroxy oder Amino, $R^{11}$ Wasserstoff, $R^{12}$ Niederalkoxy, Hydroxy, Amino oder einen Rest der wie oben definierten Formeln Ig oder Ih bedeuten und die übrigen Substituenten die obengenannten Bedeutungen haben, wobei die Verbindungen der Formel I einen Rest $A^1$ oder $A^2$ aufweisen müssen, und pharmazeutisch verwendbare Salze von solchen Verbindungen mit mindestens einer salzbildenden Gruppe.

Die Erfindung betrifft vornehmlich die obengenannten Verbindungen der Formel I, die einen Rest der Formel Ia, Ib, Ic, Id, Ie, If, Ig oder Ih aufweisen, worin $Y^1$ beziehungsweise $Y^2$ unsubstituiertes oder durch Hydroxy oder Carboxy substituiertes Alkylen mit bis zu 12 C-Atomen, das durch Carbonylimino oder Carbonyloxy unterbrochen

sein kann, bedeuten und die übrigen Substituenten die obengenannten Bedeutungen haben, und pharmazeutisch verwendbare Salze von solchen Verbindungen mit mindestens einer salzbildenden Gruppe.

Die Erfindung betrifft besonders die obengenannten Verbindungen der Formel I, die einen Rest der Formel Ia, Ic, Ie oder Ig aufweisen, worin $A^1$ für den Acylrest von Indomethacin, Ketoprofen, Naproxen, Ibuprofen oder, vorzugsweise, Diclofenac steht, und die übrigen Substituenten die obengenannten Bedeutungen haben, und pharmazeutisch verwendbare Salze von solchen Verbindungen mit mindestens einer salzbildenden Gruppe.

Besonders bevorzugt sind Verbindungen der Formel I, worin sich der Zuckerteil von D-Glucose ableitet, $X^1$ und $X^2$ Sauerstoff, $R^1$ Wasserstoff oder Niederalkanoyl, $R^2$ Niederalkanoyl oder Benzoyl, $R^3$ Wasserstoff oder Niederalkyl oder zusammen mit $R^5$ einen Niederalkylidenrest, der unsubstituiert ist oder durch gegebenenfalls Halogen-substituiertes Phenyl substituiert ist, $R^4$ Wasserstoff oder Niederalkanoyl, $R^5$ Wasserstoff oder zusammen mit $R^3$ einen Niederalkylidenrest, der unsubstituiert ist oder durch gegebenenfalls Halogen-substituiertes Phenyl substituiert ist, $R^6$ Wasserstoff, Niederalkanoyl oder einen Rest der Formel Ia, worin n für 1, $Y^1$ für unsubstituiertes oder durch Carboxy substituiertes Niederalkyliden, $X^3$ für NH und $A^1$ für 2-{2-[(2,6-Dichlor-phenyl)-amino]-phenyl}-acetyl stehen, $R^7$ und $R^8$ Wasserstoff, $R^9$ Niederalkyl, $R^{10}$ Amino, Niederalkoxy oder einen Rest der Formel Ig, worin $X^5$ für NH, $Y^2$ für einen Niederalkylenrest, der durch Carbonylimino unterbrochen und durch Hydroxy substituiert sein kann, $X^6$ für NH und $A^1$ für 2-(6-Methoxy-naphth-2-yl)-propionyl stehen, $R^{11}$ Wasserstoff oder Niederalkoxycarbonyl und $R^{12}$ Niederalkoxy, Hydroxy, Amino, einen Rest der Formel Ig, worin $X^5$ für NH, $Y^2$ für unsubstituiertes oder durch Hydroxy, Niederalkanoyloxy, Amino, Carboxy und/oder Benzyloxycarbonyl substituiertes $C_{2-10}$-Alkylen, worin eine Methylengruppe durch Sauerstoff, Schwefel, Sulfinyl $(-\overset{O}{\overset{\|}{S}}-)$ oder Carbonylimino $(-\overset{O}{\overset{\|}{C}}-NH-)$ ersetzt sein kann, $X^6$ für NH oder Sauerstoff und $A^1$ für

2-{2-[(2,6-Dichlor-phenyl)-amino]-phenyl}-acetyl, 1-Benzoyl-5-methoxy-2-methyl-indol-3-yl-acetyl, 2-(6-Methoxy-naphth-2-yl)-propionyl, 2-(4-Isobutyl-phenyl)-propionyl, 2-[3-(Hydroxybenzyl)-phenyl]-propionyl, 2-(3-Benzoyl-phenyl)-propionyl, 2-[3-Chlor-4-(pyrrol-1-yl)-phenyl]-propionyl, 1-(4-Chlor-benzoyl)-5-methoxy-2-methyl-indol-3-yl-acetyl, 2-[3-Chlor-4-(3-pyrrolin-1-yl)-phenyl]-propionyl oder 2-[4,5-bis-(4-Methoxy-phenyl)-oxazol-2-yl]-propionyl stehen, oder einen Rest der Formel Ih bedeuten, worin q für 1, $X^5$ für NH, $Y^2$ für Niederalkylen und $A^2$ für 2-[4,5-bis-(4-Methoxy-phenyl)-thiazol-2-yl-thio]-ethoxy stehen, mit der Massgabe, dass die Verbindungen einen und nur einen Rest $A^1$ oder $A^2$ enthalten, und pharmazeutisch verwendbare Salze von solchen Verbindungen mit mindestens einer salzbildenden Gruppe.

Hauptsächlich bevorzugt sind die obengenannten Verbindungen, worin $Y^1$ 2-Carboxy-ethyliden oder 2-Methyl-propyliden oder $Y^2$ Di- oder Tetramethylen, Ethyliden, 1-Carboxy-dimethylen, 1-Carboxy-tetramethylen, 1-Carboxy-pentamethylen, 2-Hydroxy-trimethylen, 1-Carboxy-3-oxa-pentamethylen ($-\underset{COOH}{CH}-CH_2-O-CH_2-CH_2-$), 1-Benzyloxycarbonyl-3-thiapentamethylen, 4-(Ethylidencarbonylimino)-n-butyl [$-CH(CH_3)-CONH-(CH_2)_4-$], 3-(Ethylidencarbonylimino)-2-hydroxy-propyl, 2-Acetoxy-3-(ethylidencarbonylimino)-propyl, 3-[(4-Amino-pentyliden)-carbonylimino]-2-hydroxy-propyl oder 1-Benzyloxycarbonyl-2-(ethylen-1-sulfinyl)-dimethylen($-\underset{COO-CH_2-C_6H_5}{CH}-CH_2-\overset{O}{S}-CH_2-CH_2-$) bedeuten, und pharmazeutisch verwendbare Salze von solchen Verbindungen mit mindestens einer salzbildenden Gruppe.

Die Erfindung betrifft vorzugsweise die obengenannten Verbindungen der Formel I, worin der Rest $A^1$ vom Acylrest des Indomethacins verschieden ist.

In allerersster Linie betrifft die Erfindung die in den Beispielen beschriebenen Verbindungen und pharmazeutisch verwendbare Salze von solchen Verbindungen mit mindestens einer salzbildenden Gruppe.

Die Erfindung betrifft auch die neuen, sowohl als Zwischenprodukte als auch als Endprodukte verwendbaren Verbindungen der Formel II,

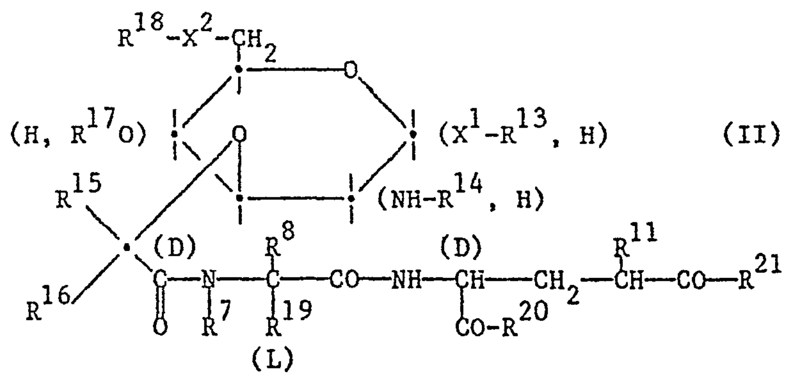

(II)

worin sich der Zuckerteil von D-Glucose, D-Mannose oder D-Galactose ableitet, $X^1$, $X^2$, $R^7$, $R^8$ und $R^{11}$ die obengenannten Bedeutungen haben, $R^{13}$, $R^{17}$ und $R^{18}$ unabhängig voneinander Wasserstoff oder Niederalkanoyl, oder $R^{13}$ auch unsubstituiertes oder substituiertes Benzyl, $R^{14}$ unsubstituiertes oder durch Hydroxy substituiertes Niederalkanoyl oder unsubstituiertes oder substituiertes Benzoyl, $R^{15}$ Cycloalkyl und $R^{16}$ Wasserstoff oder $R^{15}$ und $R^{16}$ zusammen Niederalkyliden, Cycloalkyliden oder unsubstituiertes oder substituiertes Benzyliden, $R^{19}$ Wasserstoff oder Niederalkyl, das unsubstituiert oder durch Hydroxy, Mercapto, Niederalkylthio, Carboxy, Niederalkoxycarbonyl oder Carbamoyl substituiert ist, $R^{20}$ und $R^{21}$ unabhängig voneinander Niederalkoxy, Hydroxy, Amino oder Niederalkylamino, das durch Carboxy, Carbamoyl oder Niederalkoxycarbonyl substituiert ist und das zusätzlich durch Amino, Hydroxy, Carboxy, 2-Amino-ethylthio, 2-Amino-ethoxy und/oder die Sulfogruppe $-SO_3H$ substituiert sein kann, bedeuten, Salze dieser Verbindungen mit mindestens einer salzbildenden Gruppe, Verfahren zu ihrer Herstellung und ihre Verwendung als Zwischenprodukt zur Herstellung der Verbindungen der Formel I sowie als Immunstimulantien.

Die Erfindung betrifft insbesondere diejenigen Verbindungen der Formel II, worin $R^{13}$, $R^{17}$ und $R^{18}$ unabhängig voneinander Wasserstoff oder Niederalkanoyl, $R^{14}$ unsubstituiertes oder durch Hydroxy substituiertes Niederalkanoyl, $R^{15}$ Cycloalkyl und $R^{16}$ Wasserstoff oder $R^{15}$ und $R^{16}$ zusammen Niederalkyliden oder Cycloalkyliden bedeuten.

Die Verbindungen der Formel II werden in an sich bekannter Weise hergestellt, z.B. analog der in der Deutschen Offenlegungsschrift 26 55 500 beschriebenen Weise, wobei man ein mögliches Ausgangsmaterial, z.B. durch Umsetzung einer Verbindung der Formel III,

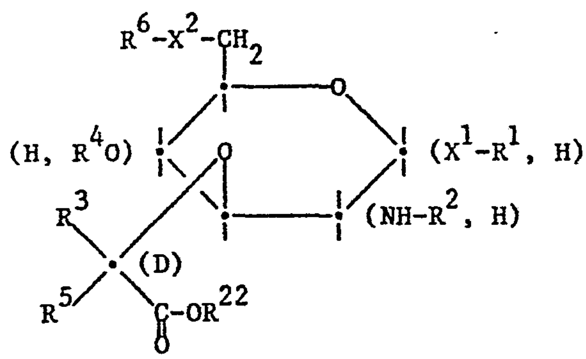

(III)

worin $R^3$ und $R^5$ Wasserstoff und $R^{22}$ einen aliphatischen, aromatischen oder aliphatischen Rest, z.B. Methyl, bedeuten und die übrigen Substituenten die obengenannten Bedeutungen haben, mit einer Base, die in der Lage ist, einen der Reste $R^3$ und $R^5$ als $H^{\oplus}$-Ion abzulösen, z.B. mit Natriumhydroxid, Natriumamid oder Lithiumhydrid, anschliessender Umsetzung des gebildeten Carbanions mit einem Keton, Eliminierung von Wasser aus dem erhaltenen Zwischenprodukt und Anknüpfung der Peptidsequenz erhält.

Die Erfindung betrifft auch das neue, nach Eliminierung von Wasser gemäss vorstehender Sequenz erhaltene Zwischenprodukt.

Die Verbindungen der Formel I werden ebenfalls in an sich bekannter Weise hergestellt.

0163286

Verfahrensvariante a): Verbindungen der Formel I, worin mindestens einer der Reste $R^{10}$ und $R^{12}$ für einen Rest der Formel Ig oder Ih steht, oder Salze von solchen Verbindungen mit mindestens einer salzbildenden Gruppe werden z.B. erhalten, indem man eine Verbindung der Formel IV,

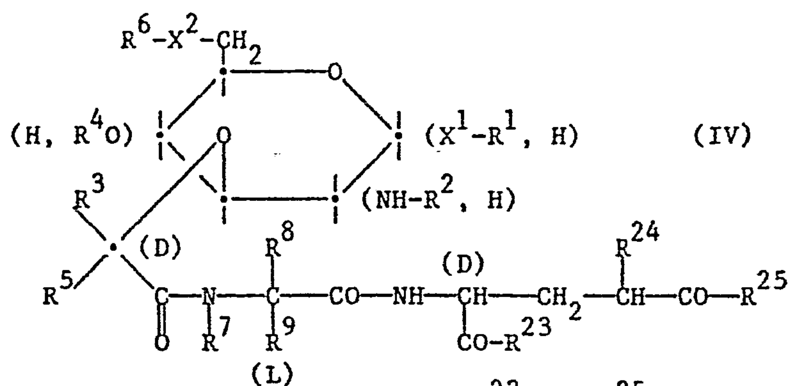 (IV)

worin mindestens einer der Reste $R^{23}$ und $R^{25}$ für Hydroxy steht und der andere der Reste $R^{23}$ und $R^{25}$ durch eine Carboxylschutzgruppe geschütztes Hydroxy, Niederalkoxy, Amino, Niederalkylamino, das durch Niederalkoxycarbonyl oder geschütztes Carboxy substituiert ist und das zusätzlich durch Amino, Hydroxy, 2-Amino-ethylthio, 2-Amino-ethoxy, geschütztes Carboxy oder eine gegebenenfalls in geschützter Form vorliegende Sulfogruppe substituiert sein kann, oder einen wie oben definierten Rest der Formel Ig oder Ih bedeutet, $R^{24}$ Wasserstoff, Carbamoyl oder geschütztes Carboxy bedeutet und die übrigen Substituenten die obengenannten Bedeutungen haben, wobei in einer Verbindung der Formel IV vorhandene Carboxy- und Aminogruppen sowie erforderlichenfalls Hydroxy- und andere funktionelle Gruppen mit Ausnahme der an der Reaktion teilnehmenden Gruppe in geschützter Form vorliegen, oder ein reaktionsfähiges Carbonsäurederivat einer Verbindung der Formel IV in einem Schritt oder stufenweise mit einer Verbindung der Formel V,

$$H-\left[X^5-Y^2-X^7\right]_r-A^3 \qquad (V)$$

worin $X^5$ und $Y^2$ die obengenannten Bedeutungen haben, $A^3$ die obengenannte Bedeutung von $A^1$ oder $A^2$ hat, $X^7$ die obengenannte Bedeutung von $X^6$ hat, wenn $A^3$ für $A^1$ steht, oder $X^7$ eine Carbonylgruppe bedeutet, wenn $A^3$ für $A^2$ steht, und r für 1 steht, wenn $A^3$ für $A^1$ steht, oder r die obengenannte Bedeutung von q hat, wenn $A^3$ für $A^2$ steht, oder mit einem reaktionsfähigen Derivat einer Verbindung der Formel V umsetzt, vorhandene Schutzgruppen abspaltet und, wenn erwünscht, eine erhaltene Verbindung der Formel I mit mindestens einer salzbildenden Gruppe in ein Salz überführt.

Schutzgruppen, ihre Einführung und Abspaltung sind beispielsweise beschrieben in "Protective Groups in Organic Chemistry", Plenum Press, London, New York 1973,und in "Methoden der organischen Chemie", Houben-Weyl, 4. Auflage, Bd. 15/1, Georg-Thieme-Verlag, Stuttgart 1974 sowie in Theodora W. Greene, "Protective Groups in Organic Synthesis, John Wiley & Sons, New York 1981. Charakteristisch für Schutzgruppen ist, dass sie leicht, d.h. ohne dass unerwünschte Nebenreaktionen stattfinden, z.B. solvolytisch, reduktiv, photolytisch oder auch unter physiologischen Bedingungen abspaltbar sind.

Hydroxyschutzgruppen sind z.B. Acylreste, wie gegebenenfalls, z.B. durch Halogen, substituiertes Niederalkanoyl, wie 2,2-Dichloracetyl, oder Acylreste von Kohlensäurehalbestern, insbesondere tert.-Butyloxycarbonyl, gegebenenfalls substituiertes Benzyloxycarbonyl, z.B. 4-Nitro-benzyloxycarbonyl, oder Diphenylmethoxycarbonyl, oder 2-Halogen-niederalkoxycarbonyl, wie 2,2,2-Trichlorethoxycarbonyl, ferner Trityl oder Formyl, oder organische Silyl- oder Stannylreste, ferner leicht abspaltbare verethernde Gruppen, wie tert.-Niederalkyl, z.B. tert.-Butyl, 2-oxa-oder 2-thia-aliphatische oder -cycloaliphatische Kohlenwasserstoffreste, in erster Linie 1-Niederalkoxyniederalkyl oder 1-Niederalkylthio-niederalkyl, z.B. Methoxymethyl, 1-Methoxy-ethyl, 1-Ethoxy-ethyl, Methylthiomethyl, 1-Methylthioethyl oder 1-Ethylthioethyl, oder 2-Oxa- oder 2-Thiacycloalkyl mit 5-6 Ringatomen, z.B. Tetrahydrofuryl oder 2-Tetrahydropyranyl oder entsprechende Thiaanaloge, sowie gegebenenfalls

substituiertes 1-Phenylniederalkyl, wie gegebenenfalls substituiertes Benzyl oder Diphenylmethyl, wobei als Substituenten der Phenylreste z.B. Halogen, wie Chlor, Niederalkoxy, wie Methoxy und/oder Nitro in Frage kommen.

Carboxylgruppen sind üblicherweise in veresterter Form geschützt, wobei solche Estergruppierungen unter schonenden Bedingungen leicht spaltbar sind. In dieser Art geschützte Carboxylgruppen enthalten als veresternde Gruppen in erster Linie in 1-Stellung verzweigte oder in 1- oder 2-Stellung geeignet substituierte Niederalkylgruppen. Bevorzugte, in veresterter Form vorliegende Carboxylgruppen sind u.a. tert.-Niederalkoxycarbonyl, z.B. tert.-Butyloxycarbonyl, Arylmethoxycarbonyl mit einem oder zwei Arylresten, wobei diese gegebenenfalls z.B. durch Niederalkyl, wie tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellen, wie gegebenenfalls, z.B. wie oben erwähnt, substituiertes Benzyloxycarbonyl, z.B. 4-Methoxybenzyloxycarbonyl, oder 4-Nitrobenzyloxycarbonyl, oder gegebenenfalls, z.B. wie oben erwähnt, substituiertes Diphenylmethoxycarbonyl, z.B. Diphenylmethoxycarbonyl oder Di-(4-methoxyphenyl)-methoxycarbonyl, 1-Niederalkoxyniederalkoxycarbonyl, wie Methoxymethoxycarbonyl, 1-Methoxyethoxycarbonyl oder 1-Ethoxymethoxycarbonyl, 1-Niederalkylthioniederalkoxycarbonyl, wie 1-Methylthiomethoxycarbonyl oder 1-Ethylthioethoxycarbonyl, Aroylmethoxycarbonyl, worin die Aroylgruppe gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2-Trichlorethoxycarbonyl, 2-Bromethoxycarbonyl oder 2-Iodethoxycarbonyl, oder 2-(trisubstituiertes Silyl)-ethoxycarbonyl, worin die Substituenten unabhängig voneinander je einen gegebenenfalls substituierten, z.B. durch Niederalkyl, Niederalkoxy, Aryl, Halogen, und/oder Nitro substituierten, aliphatischen, araliphatischen, cycloaliphatischen oder aromatischen Kohlenwasserstoffrest, wie entsprechendes, gegebenenfalls substituiertes Niederalkyl, Phenylniederalkyl, Cycloalkyl oder Phenyl, bedeuten,

z.B. 2-Triniederalkylsilylethoxycarbonyl, 2-Trimethylsilylethoxycarbonyl oder 2-(Di-n-butyl-methyl-silyl)-ethoxycarbonyl, oder 2-Triarylsilylethoxycarbonyl, wie 2-Triphenylsilylethoxycarbonyl.

Die oben und im folgenden erwähnten organischen Silyl- oder Stannylreste enthalten vorzugsweise Niederalkyl, insbesondere Methyl, als Substituenten der Silizium- oder Zinn-Atome. Entsprechende Silyl- oder Stannylgruppen sind in erster Linie Triniederalkylsilyl, insbesondere Trimethylsilyl, ferner Dimethyl-tert.-butyl-silyl, oder entsprechend substituiertes Stannyl, z.B. Tri-n-butylstannyl.

Bevorzugte geschützte Carboxylgruppen sind tert.-Niederalkoxycarbonyl, wie tert.-Butoxycarbonyl, und in erster Linie gegebenenfalls, z.B. wie oben erwähnt, substituiertes Benzyloxycarbonyl, wie 4-Nitrobenzyloxycarbonyl, oder Diphenylmethoxycarbonyl, vor allem 2-(Trimethylsilyl)-ethoxycarbonyl.

Eine geschützte Aminogruppe kann z.B. in Form einer leicht spaltbaren Acylamino-, Arylmethylamino-, verätherten Mercaptoamino-, 2-Acyl-niederalk-1-en-yl-amino-, Silyl- oder Stannylaminogruppe oder als Azidogruppe vorliegen.

In einer entsprechenden Acylaminogruppe ist Acyl beispielsweise der Acylrest einer organischen Carbonsäure mit z.B. bis zu 18 Kohlenstoffatomen, insbesondere einer gegebenenfalls,z.B. durch Halogen oder Aryl, substituierten Alkancarbonsäure oder gegebenenfalls, z.B. durch Halogen, Niederalkoxy oder Nitro, substituierten Benzoesäure, oder eines Kohlensäurehalbesters. Solche Acylgruppen sind beispielsweise Niederalkanoyl, wie Formyl, Acetyl oder Propionyl, Halogenniederalkanoyl, wie 2-Halogenacetyl, insbesondere 2-Chlor-, 2-Brom-, 2-Jod-, 2,2,2-Trifluor- oder 2,2,2-Trichloracetyl, gegebenenfalls, z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl, insbesondere tert.-Niederalkoxycarbonyl, z.B. tert.-Butyloxycarbonyl,

Arylmethoxycarbonyl mit einem oder zwei Arylresten, die vorzugsweise gegebenenfalls, z.B. durch Niederalkyl, insbesondere tert.-Niederalkyl, wie tert.Butyl, Niederalkoxy, wie Methoxy, Hydroxy, Halogen, z.B. Chlor, und/oder Nitro,mono- oder polysubstituiertes Phenyl darstellen, wie gegebenenfalls substituiertes Benzyloxycarbonyl, z.B. 4-Nitro-benzyloxycarbonyl, oder substituiertes Diphenylmethoxycarbonyl, z.B. Benzhydryloxycarbonyl oder Di-(4-methoxyphenyl)-methoxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2-Trichlorethoxycarbonyl, 2-Bromethoxycarbonyl oder 2-Iodethoxycarbonyl, oder 2-(trisubstituiertes Silyl)-ethoxycarbonyl, worin die Substituenten unabhängig voneinander je einen gegebenenfalls substituierten, z.B. durch Niederalkyl, Niederalkoxy, Aryl, Halogen oder Nitro substituierten, aliphatischen, araliphatischen, cycloaliphatischen oder aromatischen Kohlenwasserstoffrest mit bis zu 15 C-Atomen, wie entsprechendes, gegebenenfalls substituiertes Niederalkyl, Phenylniederalkyl, Cycloalkyl oder Phenyl, bedeuten, z.B. 2-Triniederalkylsilylethoxycarbonyl, wie 2-Trimethylsilylethoxycarbonyl oder 2-(Di-n-butyl-methyl-silyl)-ethoxycarbonyl, oder 2-Triarylsilylethoxycarbonyl, wie 2-Triphenylsilylethoxycarbonyl.

Weitere, als Aminoschutzgruppen in Frage kommende Acylreste sind auch entsprechende Reste organischer Phosphor-, Phosphon- oder Phosphinsäuren, wie Diniederalkylphosphoryl, z.B. Dimethylphosphoryl, Diethylphosphoryl, Di-n-propylphosphoryl oder Diisopropylphosphoryl, Dicycloalkylphosphoryl, z.B. Dicyclohexylphosphoryl, gegebenenfalls substituiertes Diphenylphosphoryl, z.B. Diphenylphosphoryl, gegebenenfalls, z.B. durch Nitro substituiertes Di-(phenylniederalkyl)-phosphoryl, z.B. Dibenzylphosphoryl oder Di-(4-nitrobenzyl)-phosphoryl, gegebenenfalls substituiertes Phenyloxy-phenyl-phosphonyl, z.B. Phenyloxyphenyl-phosphonyl, Diniederalkylphosphinyl, z.B. Diethylphosphinyl, oder gegebenenfalls substituiertes Diphenylphosphinyl, z.B. Diphenylphosphinyl.

In einer Arylmethylaminogruppe, die eine Mono-, Di- oder insbesondere Triarylmethylaminogruppe darstellt, sind die Arylreste insbesondere gegebenenfalls substituierte Phenylreste. Solche Gruppen sind beispielsweise Benzyl-, Diphenylmethyl- und insbesondere Tritylamino.

Eine verätherte Mercaptogruppe in einer mit einem solchen Rest geschützten Aminogruppe ist in erster Linie Arylthio oder Arylniederalkylthio, worin Aryl insbesondere gegebenenfalls, z.B. durch Niederalkyl, wie Methyl oder tert.-Butyl, Niederalkoxy, wie Methoxy, Halogen, wie Chlor, und/oder Nitro substituiertes Phenyl ist. Eine entsprechende Aminoschutzgruppe ist z.B. 4-Nitrophenylthio.

In einem als Aminoschutzgruppe verwendbaren 2-Acyl-niederalk-1-enl-yl-rest ist Acyl z.B. der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters. Entsprechende Schutzgruppen sind in erster Linie 1-Niederalkanoyl-prop-1-en-2-yl, z.B. 1-Acetyl-prop-1-en-2-yl, oder 1-Niederalkoxycarbonyl-prop-1-en-2-yl, z.B. 1-Ethoxycarbonyl-prop-1-en-2-yl.

Eine Aminogruppe kann auch in protonierter Form geschützt werden; als entsprechende Anionen kommen in erster Linie diejenigen von starken anorganischen Säuren, wie von Halogenwasserstoffsäuren, z.B. das Chlor- oder Bromanion, oder von organischen Sulfonsäuren, wie p-Toluolsulfonsäure, in Frage.

Bevorzugte Aminoschutzgruppen sind Acylreste von Kohlensäurehalbestern, insbesondere tert.-Butyloxycarbonyl, gegebenenfalls, z.B. wie angegeben, substituiertes Benzyloxycarbonyl, z.B. 4-Nitrobenzyloxycarbonyl, oder Diphenylmethoxycarbonyl, oder 2-Halogenniederalkoxycarbonyl, wie 2,2,2-Trichlorethoxycarbonyl, ferner Trityl oder Formyl.

Eine Mercaptogruppe, wie z.B. in Cystein, kann insbesondere durch S-Alkylierung mit gegebenenfalls substituierten Alkylresten, Thioacetalbildung, S-Acylierung oder durch das Erstellen asymmetrischer Disulfid-Gruppierungen geschützt werden. Bevorzugte Mercaptoschutzgruppen sind z.B. gegebenenfalls im Phenylrest, z.B. durch Methoxy oder Nitro, substituiertes Benzyl, wie 4-Methoxybenzyl, gegebenenfalls im Phenylteil, z.B. durch Methoxy, substituiertes Diphenylmethyl, wie 4,4'-Dimethoxydiphenyl-methyl, Triphenylmethyl, Trimethylsilyl, Benzyl-thiomethyl, Tetrahydropyranyl, Acylaminomethyl, Benzoyl, Benzyloxycarbonyl oder Aminocarbonyl, wie Ethylaminocarbonyl.

Vorzugsweise wird die Reaktion so durchgeführt, dass man die Verbindung der Formel IV in Form eines aktivierten Carbonsäurederivats mit der Verbindung der Formel V umsetzt, wobei die Aktivierung der Carbonsäure der Formel IV auch in situ in Gegenwart der Verbindung der Formel V erfolgen kann.

Aktivierte Carbonsäurederivate einer Verbindung der Formel IV sind in erster Linie reaktionsfähige aktivierte Ester oder reaktionsfähige Anhydride, ferner reaktionsfähige cyclische Amide; dabei können reaktionsfähige Derivate von Säuren der Formel IV auch in situ gebildet werden.

Aktivierte Ester von Säuren sind insbesondere am Verknüpfungskohlenstoffatom des veresternden Restes ungesättigte Ester, z.B. vom Vinylester-Typ, wie eigentliche Vinylester (die man z.B. durch Umesterung eines entsprechenden Esters mit Vinylacetat erhalten kann; Methode des aktivierten Vinylesters), Carbamoylvinylester (die man z.B. durch Behandeln der entsprechenden Säure mit einem Isoxazoliumreagens erhalten kann; 1,2-Oxazolium- oder Woodward-Methode), oder 1-Niederalkoxyvinylester (die man z.B. durch Behandeln der entsprechenden Säure mit einem Niederalkoxyacetylen erhalten kann; Ethoxyacetylen-Methode), oder Ester vom Amidinotyp, wie N,N'-disubstituierte Amidinoester (die man z.B. durch Behandeln

der entsprechenden Säure mit einem geeigneten N,N'-disubstituierten Carbodiimid, z.B. N,N'-Dicyclohexylcarbodiimid, erhalten kann; Carbodiimid-Methode), oder N,N-disubstituierte Amidinoester (die man z.B. durch Behandeln der entsprechenden Säure mit einem N,N-disubstituierten Cyanamid erhalten kann; Cyanamid-Methode), geeignete Arylester, insbesondere durch Elektronen-anziehende Substituenten geeignet substituierte Phenylester (die man z.B. durch Behandeln der entsprechenden Säure mit einem geeignet substituierten Phenol, z.B. 4-Nitrophenol, 4-Methylsulfonyl-phenol, 2,4,5-Trichlorphenol, 2,3,4,5,6-Pentachlor-phenol oder 4-Phenyldiazophenol, in Gegenwart eines Kondensationsmittels, wie N,N'-Dicyclohexyl-carbodiimid, erhalten kann; Methode der aktivierten Arylester), Cyanmethylester (die man z.B. durch Behandeln der entsprechenden Säure mit Chloracetonitril in Gegenwart einer Base erhalten kann; Cyanmethylester-Methode), Thioester, insbesondere gegebenenfalls, z.B. duch Nitro, substituierte Phenyl-thioester (die man z.B. durch Behandeln der entsprechenden Säure mit gegebenenfalls, z. B. durch Nitro, substituierten Thiophenolen, u.a. mit Hilfe der Anhydrid-oder Carbodiimid-Methode, erhalten kann; Methode der aktivierten Thiolester), Amino-oder Amidoester (die man z.B. durch Behandeln der entsprechenden Säure mit einer N-Hydroxy-amino- bzw. N-Hydroxy-amido-Verbindung, z.B. N-Hydroxy-succinimid, N-Hydroxy-piperidin, N-Hydroxyphthalimid oder 1-Hydroxy-benztriazol, z.B. nach der Anhydrid- oder Carbodiimid-Methode, erhalten kann; Methode der aktivierten N-Hydroxyester) oder Silylester (die man z.B. durch Behandeln der entsprechenden Säure mit einem Silylierungsmittel, z.B. Hexamethyldisilazan, erhalten kann).

Anhydride von Säuren der Formel IV können symmetrische oder vorzugsweise gemischte Anhydride dieser Säuren sein, so z.B. Anhydride mit anorganischen Säuren, wie Säurehalogenide, insbesondere Säurechloride (die man z.B. durch Behandeln der entsprechenden Säure mit Thionylchlorid, Phosphorpentachlorid oder Oxalylchlorid erhalten kann; Säurechloridmethode), Azide (die man z.B. aus einem entsprechenden Säureester über das entsprechende Hydrazid und dessen Behandlung mit salpetriger Säure erhalten kann; Azidmethode),

Anhydride mit Kohlensäurehalbderivaten, wie mit entsprechenden Estern, z.B. Kohlensäureniederalkylhalbestern (die man z.B. durch Behandeln der entsprechenden Säure mit Halogen-, wie Chlorameisensäure-niederalkylestern oder mit einem 1-Niederalkoxycarbonyl-2-niederalkoxy-1,2-dihydro-chinolin, z.B. 1-Niederalkoxycarbonyl-2-ethoxy-1,2-dihydrochinolin, erhalten kann; Methode der gemischten O-Alkyl-kohlensäureanhydride), oder Anhydride mit dihalogenierter, insbesondere dichlorierter Phosphorsäure (die man z.B. durch Behandeln der entsprechenden Säure mit Phosphoroxychlorid erhalten kann; Phosphoroxychloridmethode), oder Anhydride mit organischen Säuren, wie gemischte Anhydride mit organischen Carbonsäuren (die man z.B. durch Behandeln der entsprechenden Säure mit einem gegebenenfalls substituierten Niederalkan- oder Phenylalkancarbonsäurehalogenid, z.B. Phenylessigsäure-, Pivalinsäure- oder Trifluoressigsäurechlorid erhalten kann; Methode der gemischten Carbonsäureanhydride) oder mit organischen Sulfonsäuren (die man z.B. durch Behandeln eines Salzes, wie eines Alkalimetallsalzes, der entsprechenden Säure, mit einem geeigneten organischen Sulfonsäurehalogenid, wie Niederalkan- oder Aryl-, z.B. Methan- oder p-Toluolsulfonsäurechlorid, erhalten kann; Methode der gemischten Sulfonsäureanhydride), sowie symmetrische Anhydride (die man z.B. durch Kondensation der entsprechenden Säure in Gegenwart eines Carbodiimids oder von 1-Diethylaminopropin erhalten kann; Methode der symmetrischen Anhydride).

Geeignete cyclische Amide sind insbesondere Amide mit fünfgliedrigen Diazacyclen aromatischen Charakters, wie Amide mit Imidazolen, z.B. Imidazol (die man z.B. durch Behandeln der entsprechenden Säure mit N,N'-Carbonyldiimidazol erhalten kann; Imidazolid-Methode), oder Pyrazolen, z.B. 3,5-Dimethyl-pyrazol (die man z.B. über das Säurehydrazid durch Behandeln mit Acetylaceton erhalten kann; Pyrazolid-Methode).

Wie erwähnt können Derivate von Säuren der Formel IV auch in situ gebildet werden. So kann man z.B. N,N'-disubstituierte Amidinoester in situ bilden, indem man das Gemisch des Ausgangsmaterials der

Formel V und der Säure der Formel IV in Gegenwart eines geeigneten N,N-disubstituierten Carbodiimids, z.B. N,N'-Dicyclohexylcarbodiimid, zur Reaktion bringt. Ferner kann man Amino- oder Amidoester von Säuren der Formel IV in Gegenwart des zu acylierenden Ausgangsmaterials der Formel V bilden, indem man das Gemisch der entsprechenden Säure- und Amino-Ausgangsstoffe in Gegenwart eines N,N'-disubstituierten Carbodiimids, z.B. N,N'-Dicyclohexyl-carbodiimid, und eines N-Hydroxy-amins oder N-Hydroxy-amids, z.B. N-Hydroxysuccinimid, gegebenenfalls in Anwesenheit einer geeigneten Base, z.B. 4-Dimethylamino-pyridin, umsetzt.

Alternativ kann Verfahrensvariante a) auch so durchgeführt werden, dass man die Säure IV mit einem reaktionsfähigen Derivat einer Verbindung der Formel V umsetzt.

Ein Derivat einer Verbindung der Formel V, worin der Rest $H-X^5$ für die Gruppe NH steht und worin die an der Reaktion teilnehmende Aminogruppe in reaktionsfähiger Form vorliegt, kann z.B. durch Umsetzung mit einem Phosphit, z.B. Diethylchlorphosphit, 1,2-Phenylen-chlorphosphit, Ethyl-dichlor-phosphit, Ethylen-chlorphosphit oder Tetraethylpyrophosphit, hergestellt werden. Eine reaktionsfähige Form einer Verbindung der Formel V ist z.B. auch ein Carbaminsäurehalogenid oder ein Isocyanat, wobei die an der Reaktion teilnehmende Aminogruppe in einer Verbindung der Formel V an Halogencarbonyl, z.B. Chlorcarbonyl, gebunden ist beziehungsweise als Isocyanatgruppe vorliegt, wobei im letzteren Falle nur Verbindungen der Formel I zugänglich sind, die am Stickstoffatom der durch die Reaktion gebildeten Amidgruppe ein Wasserstoffatom tragen.

Ein Derivat einer Verbindung der Formel V, worin die Gruppe $H-X^5$ für in reaktionsfähiger Form vorliegendes Hydroxy steht, ist z.B. ein Halogenid. In diesem Fall kann man z.B. auch ein Metallsalz, wie Alkalimetall-, vorzugsweise Cäsiumsalz, einer Carbonsäure der Formel V mit dem genannten Halogenid umsetzen.

0162226

Die Reaktion zwischen den Verbindungen der Formeln IV und V kann, wenn r für 1 steht, wie oben ausgeführt ist, auch stufenweise durchgeführt werden, wobei man die Verbindung der Formel IV zunächst mit einer Verbindung der Formel Va,

$$H\text{-}X^5\text{-}Y^2\text{-}X^6\text{-}H \qquad (Va)$$

worin die Substituenten die obengenannten Bedeutungen haben, oder mit einem Derivat dieser Verbindungen, worin die Gruppe $H\text{-}X^5\text{-}$ in reaktionsfähiger Form vorliegt, wobei die Gruppe $X^6\text{-}H$ erforderlichenfalls durch eine Schutzgruppe geschützt ist, umsetzt, eine vorhandene Schutzgruppe für die Gruppe $X^6\text{-}H$ abspaltet und dann das erhaltene Zwischenprodukt der Formel IVa,

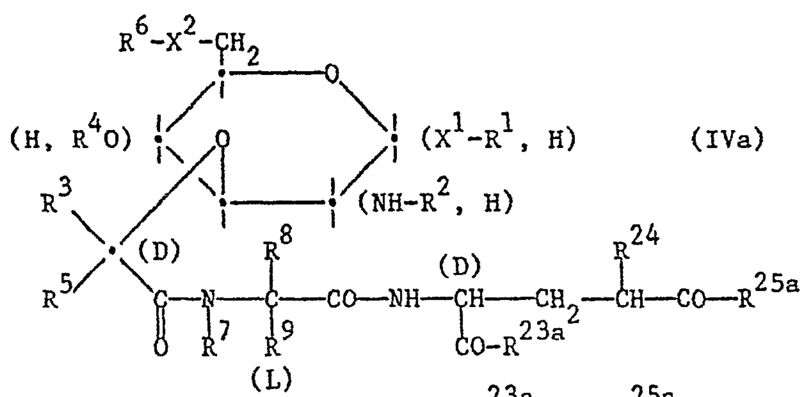

(IVa)

worin mindestens einer der Reste $R^{23a}$ und $R^{25a}$ für einen Rest der Formel $-X^5\text{-}Y^2\text{-}X^6\text{-}H$ steht, worin die Substituenten die obengenannten Bedeutungen haben, und der andere der Reste $R^{23a}$ und $R^{25a}$ die obengenannte Bedeutung von $R^{23}$ beziehungsweise $R^{25}$ hat, und die übrigen Substituenten die obengenannten Bedeutungen haben, wobei in einer Verbindung der Formel IVa vorhandene Amino- und/oder Hydroxygruppen mit Ausnahme der an der Reaktion teilnehmenden Gruppe in geschützter Form vorliegen, oder ein reaktionsfähiges Derivat einer Verbindung der Formel IVa mit einer Carbonsäure der Formel $A^1\text{-}OH$, worin $A^1$ die obengenannte Bedeutung hat, wobei darin vorhandene Amino- und/oder Hydroxygruppen sowie erforderlichenfalls andere funktionelle Gruppen mit Ausnahme der an der Reaktion teilnehmenden Gruppe in geschützter Form vorliegen, oder mit einem reaktionsfähigen Carbonsäurederivat einer Carbonsäure der Formel $A^1$-OH umsetzt, vorhandene Schutzgruppen abspaltet und, wenn erwünscht, eine erhaltene Verbindung der Formel I mit mindestens einer salzbildenden Gruppe in ein Salz überführt.

Alternativ kann man eine Verbindung der Formel IV auch zunächst mit einer Verbindung der Formel Vb,

$$H-X^5-Y^2-\overset{\overset{O}{\|}}{C}-OH \qquad (Vb)$$

worin die Substituenten die obengenannten Bedeutungen haben, wobei in einer Verbindung der Formel Vb vorhandene Carboxylgruppen vorzugsweise in geschützter Form vorliegen, oder mit einem Derivat dieser Verbindungen, worin die Gruppe $H-X^5-$ in reaktionsfähiger Form vorliegt, umsetzen und dann das erhaltene Zwischenprodukt der Formel IVb,

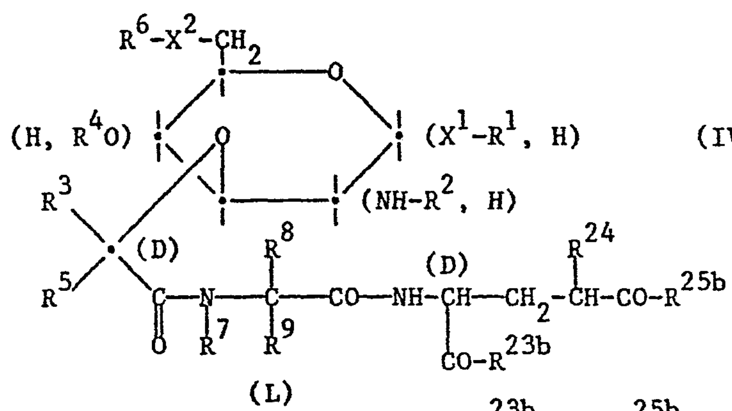

(IVb)

worin mindestens einer der Reste $R^{23b}$ und $R^{25b}$ für einen Rest der Formel $-X^5-Y^2-\overset{\overset{O}{\|}}{C}-OH$ steht, worin die Substituenten die obengenannten Bedeutungen haben, und der andere der Reste $R^{23b}$ und $R^{25b}$ die obengenannte Bedeutung von $R^{23}$ beziehungsweise $R^{25}$ hat, und die übrigen Substituenten die obengenannten Bedeutungen haben, wobei in einer Verbindung der Formel IVb vorhandene funktionelle Gruppen, wie insbesondere Amino-, Hydroxy- und/oder Carboxygruppen, mit Ausnahme der an der Reaktion teilnehmenden Gruppe erforderlichenfalls in geschützter Form vorliegen, oder ein reaktionsfähiges Carbonsäurederivat einer Verbindung der Formel IVb mit einer Verbindung der

Formel H-$A^2$, worin $A^2$ die obengenannte Bedeutung hat, wobei darin vorhandene funktionelle Gruppen, wie insbesondere Amino-, Hydroxy und/oder Carboxygruppen, mit Ausnahme der an der Reaktion teilnehmenden Gruppe erforderlichenfalls in geschützter Form vorliegen, oder mit einem reaktionsfähigen Derivat davon umsetzt, vorhandene Schutzgruppen abspaltet und, wenn erwünscht, eine erhaltene Verbindung der Formel I mit mindestens einer salzbildenden Gruppe in ein Salz überführt.

Die Erfindung betrifft auch diejenigen Ausführungsformen des Verfahrens, wobei man von einer auf beliebige Weise erhaltenen Verbindung der Formel IVa oder IVb ausgeht.

Die Abspaltung der Schutzgruppen, z.B. der Carboxyl-, Amino-, Hydroxy- oder Mercaptoschutzgruppen, erfolgt in an sich bekannter Weise, z.B. mittels Solvolyse, insbesondere Hydrolyse, Alkoholyse oder Acidolyse, oder mittels Reduktion, insbesondere Hydrogenolyse oder chemische Reduktion, gegebenenfalls stufenweise oder gleichzeitig, wobei auch enzymatische Methoden verwendet werden können.

So kann man tert.-Niederalkoxycarbonyl oder in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl z.B. durch Behandeln mit einer geeigneten Säure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, freigesetzt werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zink, oder Metallsalz, wie einem Chrom-II-salz, z.B. Chrom-II-chlorid, üblicherweise in Gegenwart eines Wasserstoff-abgebenden Mittels, das zusammen mit dem

Metall nascierenden Wasserstoff zu erzeugen vermag, wie einer Säure, in erster Linie einer geeigneten Carbonsäure, wie einer gegebenenfalls, z.B. durch Hydroxy, substituierten Niederalkancarbonsäure, z.B. Essigsäure, Ameisensäure, Glycolsäure, Diphenylglycolsäure, Milchsäure, Mandelsäure, 4-Chlor-mandelsäure oder Weinsäure, oder eines Alkohols oder Thiols, wobei man vorzugsweise Wasser zugibt, in freies Carboxyl überführen. Durch Behandeln mit einem reduzierenden Metall oder Metallsalz, wie oben beschrieben, kann man auch 2-Halogen-niederalkoxycarbonyl (gegebenenfalls nach Umwandlung einer 2-Brom-niederalkoxycarbonylgruppe in eine entsprechende 2-Iodniederalkoxycarbonylgruppe) oder Aroylmethoxycarbonyl in freies Carboxyl umwandeln, wobei Aroylmethoxycarbonyl ebenfalls durch Behandeln mit einem nucleophilen, vorzugsweise salzbildenden Reagens, wie Natriumthiophenolat oder Natriumjodid, gespalten werden kann. Substituiertes 2-Silylethoxycarbonyl kann auch durch Behandeln mit einem, das Fluoridanion liefernden Salz der Fluorwaserstoffsäure, wie einem Alkalimetallfluorid, z.B. Natrium-oder Kaliumfluorid, in Anwesenheit eines macrocyclischen Polyethers ("Kronenether"), oder mit einem Fluorid einer organischen quaternären Base, wie Tetra-niederalkylammoniumfluorid oder Triniederalkyl-arylammoniumfluorid, z.B. Tetraethylammoniumfluorid oder Tetrabutylammoniumfluorid, in Gegenwart eines aprotischen polaren Lösungsmittels, wie Dimethylsulfoxid oder N,N-Dimethylacetamid, in freies Carboxyl übergeführt werden. Mit einer organischen Silylgruppe, wie Triniederalkylsilyl z.B. Trimethylsilyl, verestertes Carboxyl kann in üblicher Weise solvolytisch, z.B. durch Behandeln mit Wasser, einem Alkohol oder Säure freigesetzt werden.

Eine geschützte Aminogruppe setzt man in an sich bekannter und je nach Art der Schutzgruppen in verschiedenartiger Weise, vorzugsweise mittels Solvolyse oder Reduktion, frei. 2-Halogen-niederalkoxycarbonylamino (gegebenenfals nach Umwandlung einer 2-Brom-niederalkoxycarbonylaminogruppe in eine 2-Iod-niederalkoxycarbonylaminogruppe), Aroylmethoxycarbonylamino oder 4-Nitrobenzyloxycarbonylamino kann z.B. durch Behandeln mit einem geeigneten chemischen Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagens, wie Natriumthiophenolat, und 4-Nitro-benzyloxycarbonylamino auch durch Behandeln mit einem Alkalimetall-, z.B. Natriumdithionit, gespalten werden. Gegebenenfalls substituiertes Diphenylmethoxycarbonylamino, tert.-Niederalkoxycarbonylamino oder 2-trisubstituiertes Silylethoxycarbonylamino kann durch Behandeln mit einer geeigneten Säure, z.B. Ameisen- oder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierkatalysators, wie eines Palladiumkatalysators, gegebenenfalls substituiertes Triarylmethylamino oder Formylamino z.B. durch Behandeln mit einer Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser, und eine mit einer organischen Silylgruppe geschützte Aminogruppe z.B. mittels Hydrolyse oder Alkoholyse freigesetzt werden. Eine durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat, des Thioharnstoffs und anschliessende Solvolyse, wie Alkoholyse oder Hydrolyse, des entstandenen Kondensationsprodukts freigesetzt werden. Eine durch 2-substituiertes Silylethoxycarbonyl geschützte Aminogruppe kann auch durch Behandeln mit einem Fluoridanionen liefernden Salz der Fluorwaserstoffsäure, wie oben im Zusammenhang mit der Freisetzung einer entsprechend geschützten Carboxylgruppe angegeben, in die freie Aminogruppe übergeführt werden.

In Form einer Azidogruppe geschütztes Amino wird z.B. durch Reduktion in freies Amino übergeführt, beispielsweise durch katalytische Hydrierung mit Wasserstoff in Gegenwart eines Hydrierkatalysators, wie Platinoxid, Palladium oder Raney-Nickel, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z.B.

Methylenchlorid, oder auch in Wasser oder einem Gemisch von Wasser und einem organischen Lösungsmittel, wie einem Alkohol oder Dioxan, bei etwa 20°C bis 25°C, oder auch unter Kühlen oder Erwärmen, durchgeführt.

Eine durch eine geeignete Acylgruppe, eine organische Silylgruppe oder durch gegebenenfalls substituiertes 1-Phenylniederalkyl geschützte Hydroxy- oder Mercaptogruppe wird analog einer entsprechend geschützten Aminogruppe freigesetzt. Eine durch 2,2-Dichloracetyl geschützte Hydroxy- bzw. Mercaptogruppe wird z.B. durch basische Hydrolyse, eine durch tert.-Niederalkyl oder durch einen 2-oxa- oder 2-thia-aliphatischen oder -cycloaliphatischen Kohlenwasserstoffrest veretherte Hydroxy- bzw. Mercaptogruppe durch Acidolyse, z.B. durch Behandeln mit einer Mineralsäure oder einer starken Carbonsäure, z.B. Trifluoressigsäure, freigesetzt. Zwei Hydroxygruppen, die zusammen mittels einer vorzugsweise substituierten Methylengruppe, wie durch Niederalkyliden, z.B. Isopropyliden, Cycloalkyliden, z.B. Cyclohexyliden, oder Benzyliden, geschützt sind, können durch saure Solvolyse, besonders in Gegenwart einer Mineralsäure oder einer starken organischen Säure, freigesetzt werden.

Beim Vorhandensein von mehreren geschützten funktionellen Gruppen können, wenn erwünscht, die Schutzgruppen so gewählt werden, dass gleichzeitig mehr als eine solche Gruppe abgespalten werden kann, beispielsweise acidolytisch, wie durch Behandeln mit Zink und Essigsäure, oder mit Wasserstoff und einem Hydrierkatalysator, wie einem Palladium-Kohle-Katalysator.

Salze von Verbindungen der Formel I mit mindestens einer salzbildenden Gruppe können in an sich bekannter Weise hergestellt werden. So kann man Salze von Verbindungen der Formel I z.B. durch Behandeln mit Metallverbindungen, wie Alkalimetallsalzen von geeigneten organischen Carbonsäuren, z.B. dem Natriumsalz der α-Aethyl-capronsäure, oder mit anorganischen Alkali- oder Erdalkalimetallverbindungen, wie entsprechenden Hydroxiden, Carbonaten

und Hydrogencarbonaten, wie Natrium- oder Kaliumhydroxid, -carbonat oder -hydrogencarbonat, oder entsprechenden Calciumverbindungen oder mit Ammoniak oder geeigneten organischen Aminen bilden, wobei man vorzugsweise stöchiometrische Mengen oder einen kleinen Ueberschuss des salzbildenden Mittels verwendet. Säureadditionssalze von Verbindungen der Formel I, die salzbildende basische Gruppen enthalten, erhält man in üblicher Weise, z.B. durch Behandeln mit einer Säure oder einem geeigneten Anionenaustauschreagens. Innere Salze von Verbindungen der Formel I, welche saure und basische salzbildende Gruppen enthalten, können z.B. durch Neutralisieren von Salzen, wie Säureadditionssalzen, auf den isoelektrischen Punkt, z.B. mit schwachen Basen, oder durch Behandeln mit flüssigen Ionenaustauschern gebildet werden.

Salze können in üblicher Weise in die freien Verbindungen übergeführt werden, Metall- und Ammoniumsalze z.B. durch Behandeln mit geeigneten Säuren, und Säureadditionssalze z.B. durch Behandeln mit einem geeigneten basischen Mittel.

Gemische von Isomeren können in an sich bekannter Weise, z.B. durch fraktionierte Kristallisation, Chromatographie etc. in die einzelnen Isomeren, Racemate z.B. unter Bildung von Derivaten mit optisch aktiven Verbindungen und Trennung der so erhältlichen Diastereomerengemische in die optisch aktiven Antipoden, aufgetrennt werden.

<u>Verfahrensvariante b):</u>

Verbindungen der Formel I, worin mindestens einer der Reste $R^1$, $R^2$, $R^4$ und $R^6$ für einen Rest der Formel Ia oder Ib steht, werden z.B. erhalten, indem man eine Verbindung der Formel VI

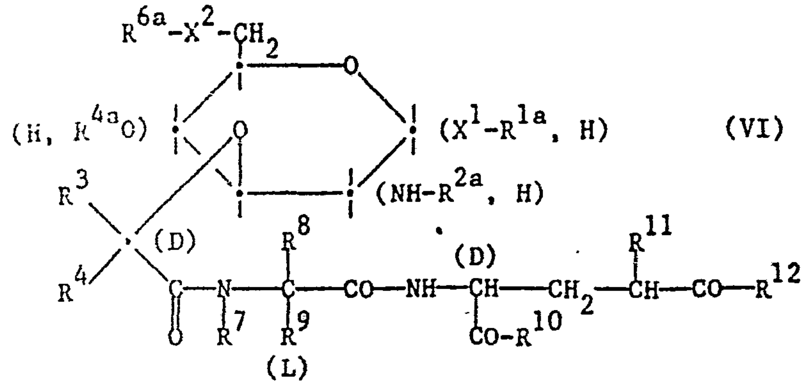

(VI)

worin mindestens einer und höchstens drei der Reste $R^{1a}$, $R^{2a}$, $R^{4a}$ und $R^{6a}$ für Wasserstoff stehen und die anderen der Reste $R^{1a}$, $R^{2a}$, $R^{4a}$ und $R^{6a}$ die obengenannten Bedeutungen der Reste $R^1$, $R^2$, $R^4$ beziehungsweise $R^6$ haben und die übrigen Substituenten die obengenannten Bedeutungen haben, wobei in einer Verbindung der Formel VI vorhandene Hydroxy, Amino oder Mercaptogruppen mit Ausnahme der an der Reaktion teilnehmenden Gruppe(n) sowie erforderlichenfalls auch andere funktionelle Gruppen in geschützter Form vorliegen, oder ein reaktionsfähiges Derivat einer Verbindung der Formel VI in einem Schritt oder stufenweise mit einer Carbonsäure der Formel VII,

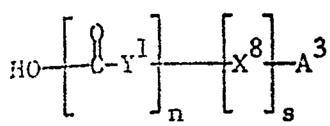

(VII)

worin $Y^1$ und n die obengenannten Bedeutungen haben, $A^3$ die obengenannte Bedeutung von $A^1$ oder $A^2$ hat, $X^8$ die obengenannte Bedeutung von $X^3$ hat, wenn $A^3$ für $A^1$ steht, oder $X^8$ eine Carbonylgruppe bedeutet, wenn $A^3$ für $A^2$ steht, und s die obengenannte Bedeutung von n hat, wenn $A^3$ für $A^1$ steht, oder s für 1 steht, wenn $A^3$ für $A^2$ steht, wobei in einer Verbindung der Formel VII vorhandene funktionelle Gruppen mit Ausnahme der an der Reaktion teilnehmenden Gruppe erforderlichenfalls in geschützter Form vorliegen, oder mit einem reaktionsfähigen Derivat einer Verbindung der Formel VII

umsetzt, vorhandene Schutzgruppen abspaltet und, wenn erwünscht, eine erhaltene Verbindung der Formel I mit mindestens einer salzbildenden Gruppe in ein Salz überführt.

Schutzgruppen für die genannten funktionellen Gruppen sind die bei Verfahrensvariante a) beschriebenen. Der Schutz von Carboxylgruppen in einer Verbindung der Formel VI ist z.B. erforderlich, wenn man ein Gemisch der Verbindungen der Formeln VI und VII in situ aktiviert, z.B. durch Zugabe von Dicyclohexylcarbodiimid.

Ein reaktionsfähiges Derivat einer Verbindung der Formel VI ist z.B. eine Verbindung, worin die an der Reaktion teilnehmende Hydroxy- oder Aminogruppe in reaktionsfähiger Form vorliegt, z.B. analog wie bei Verfahrensvariante a) beschrieben.

Funktionelle Gruppen in einer Verbindung der Formel VII, deren Schutz erforderlich sein kann, sind z.B. Amino-, Hydroxy-, Mercapto- und weitere Carboxygrupen. Ob ein Schutz erforderlich ist, hängt von der Reaktionsdurchführung im Einzelfall ab (vgl. unten).

Ein reaktionsfähiges Derivat einer Carbonsäure der Formel VII ist ein Carbonsäurederivat analog den bei Verfahrensvariante a) für Verbindung IV beschriebenen. Ein Schutz von funktionellen Gruppen, wie insbesondere Aminogruppen, in einer Verbindung der Formel VII ist in der Regel erforderlich, wenn diese Verbindung z.B. in Form eines aktivierten Esters, Anhydrids oder Amids mit einer Verbindung der Formel VI zur Reaktion gebracht wird.

Die Reaktion einer Verbindung der Formel VI mit einer Verbindung der Formel VII kann, wie oben geschildert ist, auch stufenweise durchgeführt werden. Dabei kann man die Verbindung der Formel VI zunächst mit einer Verbindung der Formel VIIa,

$$HO-\overset{O}{\overset{\|}{C}}-Y^1-X^3-H \qquad (VIIa)$$

worin die Substituenten die obengenannten Bedeutungen haben, wobei in einer Verbindung der Formel VIIa vorhandene funktionelle Gruppen mit Ausnahme der an Reaktion teilnehmenden Carboxylgruppe erforderlichenfalls in geschützter Form vorliegen, oder mit einem reaktionsfähigen Säurederivat davon umsetzen, eine geschützte Gruppe $-X^3-H$ in Freiheit setzen, und das erhaltene Zwischenprodukt der Formel VIa

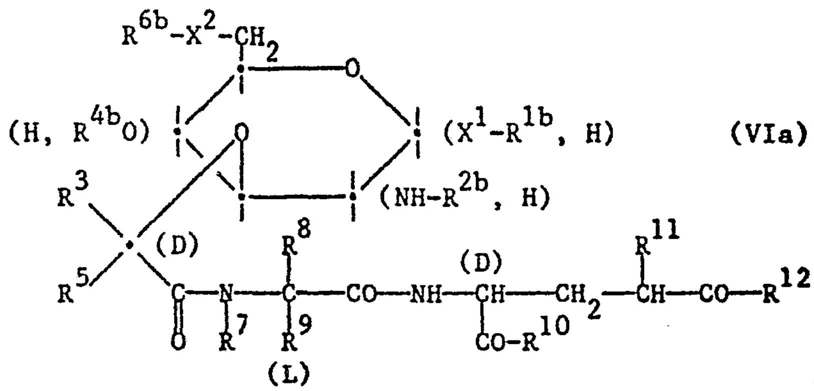

(VIa)

worin mindestens einer und höchstens drei der Reste $R^{1b}$, $R^{2b}$, $R^{4b}$ und $R^{6b}$ für einen Rest der Formel

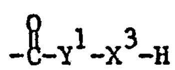

stehen und die anderen der Reste $R^{1b}$, $R^{2b}$, $R^{4b}$ und $R^{6b}$ die oben angegebenen Bedeutungen der Reste $R^1$, $R^2$, $R^4$ beziehungsweise $R_6$ haben und die übrigen Substituenten die obengenannten Bedeutungen haben, wobei in einer Verbindung der Formel VIa vorhandene Hydroxy, Amino oder Mercaptogruppen mit Ausnahme der an der Reaktion teilnehmenden Gruppe(n) sowie erforderlichenfalls auch andere funktionelle Gruppen in geschützter Form vorliegen, oder ein reaktionsfähiges Derivat einer Verbindung der Formel VIa mit einer Carbonsäure der Formel $HO-A^1$, worin $A^1$ die obengenannte Bedeutung hat, wobei darin vorhandene funktionelle Gruppen mit Ausnahme der an Reaktion teilnehmenden Carboxylgruppe erforderlichenfalls in geschützter Form vorliegen, oder mit einem reaktionfähigen Säurederivat davon umsetzen, vorhandene Schutzgruppen abspalten und, wenn erwünscht, eine erhaltene Verbindung der Formel I mit mindestens einer salzbildenden Gruppe in ein Salz überführen.

 

Alternativ kann man die Verbindungen der Formel VI zunächst mit einer Verbindung der Formel VIIb,

$$HO-\overset{O}{\overset{\|}{C}}-Y^1-\overset{O}{\overset{\|}{C}}-OH \qquad \text{(VIIb)}$$

worin $Y^1$ die obengenannte Bedeutung hat und worin die rechte Carboxylgruppe erforderlichenfalls (z.B. wenn die Verbindung der Formel VIIb asymmetrisch ist) in geschützter Form vorliegt, oder mit einem reaktionsfähigen Säurederivat davon umsetzen, eine geschützte Carboxylgruppe freisetzen und anschliessend das erhaltene Zwischenprodukt der Formel VIb,

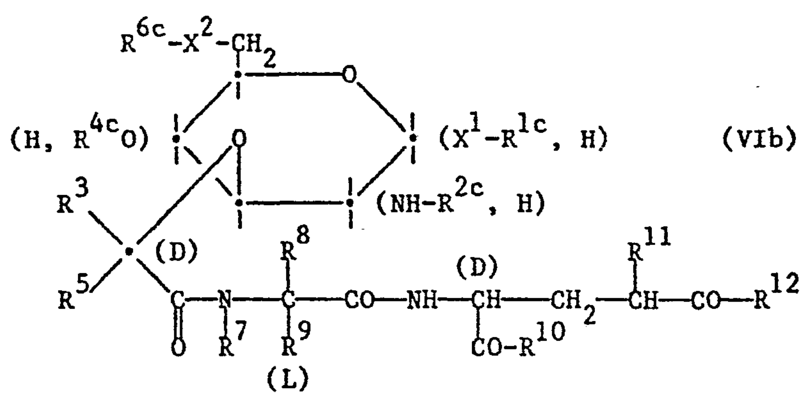

(VIb)

worin mindestens einer und höchstens drei der Reste $R^{1c}$, $R^{2c}$, $R^{4c}$ und $R^{6c}$ für den Rest

$-\overset{O}{\overset{\|}{C}}-Y^1-\overset{O}{\overset{\|}{C}}-OH$ , worin $Y^1$, die obengenannte Bedeutung hat, stehen und die anderen der Reste $R^{1c}$, $R^{2c}$, $R^{4c}$ und $R^{6c}$ die obengenannten Bedeutungen der Reste $R^1$, $R^2$, $R^4$ beziehungsweise $R^6$ haben und die übrigen Substituenten die obengenannten Bedeutungen haben, wobei in einer Verbindung der Formel VIb vorhandene funktionelle Gruppen mit Ausnahme der an der Reaktion teilnehmenden Gruppe(n) erforderlichenfalls in geschützter Form vorliegen, oder ein reaktionsfähiges Säurederivat einer Verbindung der Formel VIb mit einer Verbindung der Formel $H-A^2$, worin $A^2$ die obengenannte Bedeutung hat, wobei

darin vorhandene funktionelle Gruppen mit Ausnahme der an der Reaktion teilnehmenden Gruppe erforderlichenfalls in geschützter Form vorliegen, oder mit einem reaktionsfähigen Derivat davon umsetzen, vorhandene Schutzgruppen abspalten und, wenn erwünscht, eine erhaltene Verbindung der Formel I mit mindestens einer salzbildenden Gruppe in ein Salz überführen.

Die Erfindung betrifft auch diejenigen Ausführungsformen des Verfahrens, wobei man von einer auf beliebige Weise erhaltenen Verbindung der Formel VIa oder VIb ausgeht. Die obengenannten Reaktionen werden in analoger Weise, wie bei Verfahrensvariante a) beschrieben, durchgeführt.

Verfahrensvariante c:

Verbindungen der Formel I, worin der Rest $R^9$ Niederalkyl bedeutet, das durch einen wie oben definierten Rest der Formel Ic oder Id substituiert ist, oder ihre Salze werden z.B. erhalten, indem man eine Verbindung der Formel I, worin der Rest $R^9$ für durch Hydroxy oder Mercapto substituiertes Niederalkyl steht, wobei in einer Verbindung der Formel I vorhandene Hydroxy-, Mercapto- und Aminogruppen sowie, wenn erwünscht, andere funktionelle Gruppen mit Ausnahme der an der Reaktion teilnehmenden funktionellen Gruppe in geschützter Form vorliegen, oder ein reaktionsfähiges Derivat davon, in einem Schritt oder stufenweise mit einer Carbonsäure der Formel VIII,

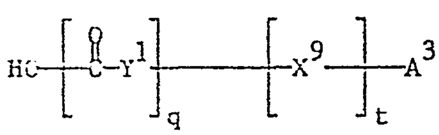

(VIII)

worin $Y^1$ und q die obengenannten Bedeutungen haben, $A^3$ die obengenannte Bedeutung von $A^1$ oder $A^2$ hat, $X^9$ die obengenannte Bedeutung von $X^3$ hat, wenn $A^3$ für $A^1$ steht, oder $X^9$ eine Carbonylgruppe bedeutet, wenn $A^3$ für $A^2$ steht, und t die obengenannte Bedeutung von q hat, wenn $A^3$ für $A^1$ steht, oder q für 1 steht, wenn $A^3$ für $A^2$ steht, wobei in einer Verbindung der Formel VIII vorhandene funktionelle Gruppen mit Ausnahme der an der Reaktion teilnehmenden Gruppe erforderlichenfalls in geschützter Form vorliegen, oder mit einem reaktionsfähigen Derivat einer Verbindung der Formel VIII umsetzt, vorhandene Schutzgruppen abspaltet und, wenn erwünscht, eine erhaltene Verbindung der Formel I mit mindestens einer salzbildenden Gruppe in ein Salz überführt.

Ein reaktionsfähiges Derivat einer Verbindung der Formel I, worin der Rest $R^9$ für durch Hydroxy substituiertes Niederalkyl steht, ist z.B. ein Halogenid, z.B. Chlorid, Bromid oder Iodid, das z.B. mit dem Cäsiumsalz einer Verbindung der Formel VIII umgesetzt werden kann.

Die Reaktion kann, wie oben geschildert ist, auch stufenweise durchgeführt werden. Dabei kann man die Verbindung der Formel I, worin der Rest $R^9$ für durch Hydroxy oder Mercapto substituiertes Niederalkyl steht, wobei in einer Verbindung der Formel I vorhandene Hydroxy-, Mercapto- und Aminogruppen sowie, wenn erwünscht, andere funktionelle Gruppen mit Ausnahme der an der Reaktion teilnehmenden funktionellen Gruppe in geschützter Form vorliegen, oder ein reaktionsfähiges Derivat davon zunächst mit einer Verbindung der Formel VIIa,

$$HO-\overset{O}{\overset{\|}{C}}-Y^1-X^3-H \qquad \text{(VIIa)}$$

worin die Substituenten die obengenanten Bedeutungen haben, wobei in einer Verbindung der Formel VIIa vorhandene funktionelle Gruppen mit Ausnahme der an Reaktion teilnehmenden Carboxylgruppe erforderlichenfalls in geschützter Form vorliegen, oder mit einem reaktionsfähigen Säurederivat davon umsetzen, eine geschtzte Gruppe $-X^3-H$ in Freiheit setzen, und das erhaltene Zwischenprodukt der Formel IXa,

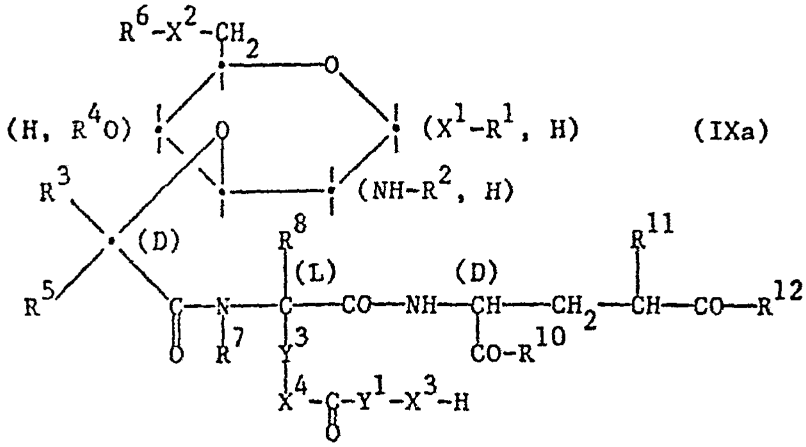

(IXa)

worin $Y^3$ Niederalkylen bedeutet und die übrigen Substituenten die obengenannten Bedeutungen haben, wobei in einer Verbindung der Formel IXa vorhandene funktionelle Gruppen mit Ausnahme der an der Reaktion teilnehmenden Gruppe erforderlichenfalls in geschützter Form vorliegen, oder ein reaktionsfähiges Derivat einer Verbindung der Formel IXa mit einer Carbonsäure der Formel $HO-A^1$, worin $A^1$ die obengenannte Bedeutung hat, wobei darin vorhandene funktionelle Gruppen mit Ausnahme der an Reaktion teilnehmenden Carboxylgruppe erforderlichenfalls in geschützter Form vorliegen, oder mit einem reaktionsfähigen Säurederivat davon umsetzen, vorhandene Schutzgruppen abspalten und, wenn erwünscht, eine erhaltene Verbindung der Formel I mit mindestens einer salzbildenden Gruppe in ein Salz überführen.

Alternativ kann man die Verbindung der Formel I, worin der Rest $R^9$ für durch Hydroxy oder Mercapto substituiertes Niederalkyl steht, wobei in einer Verbindung der Formel I vorhandene Hydroxy-, Mercapto- und Aminogruppen sowie, wenn erwünscht, andere funktionelle Gruppen mit Ausnahme der an der Reaktion teilnehmenden funktionellen Gruppe in geschützter Form vorliegen, oder ein reaktionsfähiges Derivat davon zunächst mit einer Verbindung der Formel VIIb,

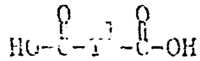

(VIIb)

worin $Y^1$ die obengenannte Bedeutung hat und worin die rechte Carboxylgruppe erforderlichenfalls (z.B. wenn die Verbindung der Formel VIIb asymmetrisch ist) in geschützter Form vorliegt, oder mit einem reaktionsfähigen Säurederivat davon umsetzen, eine geschützte Carboxylgruppe freisetzen und anschliessend das erhaltene Zwischenprodukt der Formel IXb,

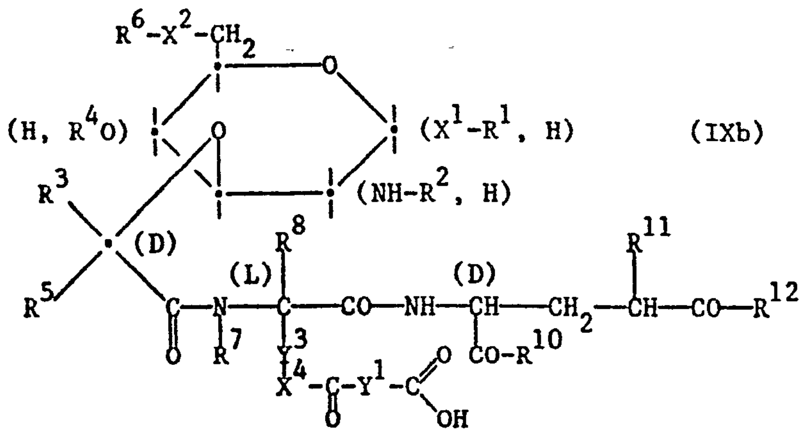

(IXb)

worin $Y^3$ Niederalkylen bedeutet und die übrigen Substituenten die obengenannten Bedeutungen haben, wobei in einer Verbindung der Formel IXb vorhandene funktionelle Gruppen mit Ausnahme der an der Reaktion teilnehmenden Gruppe erforderlichenfalls in geschützter Form vorliegen, oder ein reaktionsfähiges Carbonsäurederivat einer Verbindung der Formel IXb mit einer Verbindung der Formel $H-A^2$, worin $A^2$ die obengenannte Bedeutung hat, wobei darin vorhandene funktionelle Gruppen mit Ausnahme der an der Reaktion teilnehmenden Gruppe erforderlichenfalls in geschützter Form vorliegen, oder mit einem reaktionsfähigen Derivat davon umsetzen, vorhandene Schutzgruppen abspalten und, wenn erwünscht, eine erhaltene Verbindung der Formel I mit mindestens einer salzbildenden Gruppe in ein Salz überführen.

Die Erfindung betrifft auch diejenigen Ausführungsformen des Verfahrens, wobei man von einer auf beliebige Weise erhaltenen Verbindung der Formel IXa oder IXb ausgeht. Die obengenannten Reaktionen werden in analoger Weise, wie bei Verfahrensvariante a) beschrieben, durchgeführt.

Verfahrensvariante d):

Verbindungen der Formel I, worin der Rest $R^9$ durch einen wie oben definierten Rest der Formeln Ie oder If substituiertes Niederalkyl bedeutet, oder ihre Salze werden z.B. erhalten, indem man eine Verbindung der Formel I, worin der Rest $R^9$ durch Carboxy substituiertes Niederalkyl bedeutet, wobei in einer Verbindung der Formel I vorhandene Carboxygruppen sowie, wenn erwünscht, andere funktionelle Gruppen mit Ausnahme der an der Reaktion teilnehmenden funktionellen Gruppe in geschützter Form vorliegen, oder ein reaktionsfähiges Carbonsäurederivat davon in einem Schritt oder stufenweise mit einer Verbindung der Formel V,

$$H\left[X^5-Y^2-X^7\right]_r A^3 \qquad (V)$$

worin $X^5$ und $Y^2$ die obengenannten Bedeutungen haben, $A^3$ die obengenannte Bedeutung von $A^1$ oder $A^2$ hat, $X^7$ die obengenannte Bedeutung von $X^6$ hat, wenn $A^3$ für $A^1$ steht, oder $X^7$ eine Carbonylgruppe bedeutet, wenn $A^3$ für $A^2$ steht, und r für 1 steht, wenn $A^3$ für $A^1$ steht, oder r die obengenannte Bedeutung von q hat, wenn $A^3$ für $A^2$ steht, oder mit einem reaktionsfähigen Derivat einer Verbindung der Formel V umsetzt, vorhandene Schutzgruppen abspaltet und, wenn erwünscht, eine erhaltene Verbindung der Formel I mit mindestens einer salzbildenden Gruppe in ein Salz überführt.

Die Reaktion kann, wie oben geschildert ist, auch stufenweise durchgeführt werden. Dabei kann man die Verbindung der Formel I, worin der Rest $R^9$ durch Carboxy substituiertes Niederalkyl bedeutet, wobei in einer Verbindung der Formel I vorhandene Carboxygruppen

sowie, wenn erwünscht, andere funktionelle Gruppen mit Ausnahme der an der Reaktion teilnehmenden funktionellen Gruppe in geschützter Form vorliegen, oder ein reaktionsfähiges Carbonsäurederivat davon, zunächst mit einer Verbindung der Formel Va,

$$H-X^5-Y^2-X^6-H \qquad (Va)$$

worin die Substituenten die obengenannten Bedeutungen haben, oder mit einem Derivat dieser Verbindung, worin die Gruppe $H-X^5-$ in reaktionsfähiger Form vorliegt, wobei die Gruppe $X^6-H$ erforderlichenfalls durch eine Schutzgruppe geschützt ist, umsetzt, eine vorhandene Schutzgruppe für die Gruppe $X^6-H$ abspaltet und dann das erhaltene Zwischenprodukt der Formel IXc,

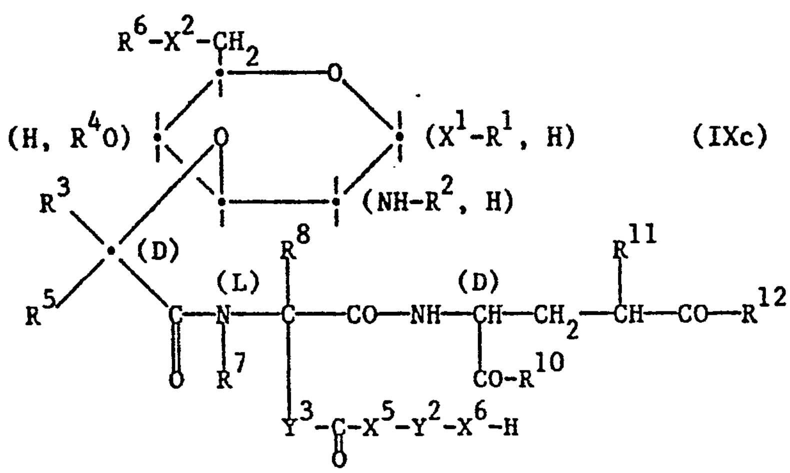
(IXc)

worin $Y^3$ Niederalkylen bedeutet und die übrigen Substituenten die obengenannten Bedeutungen haben, wobei in einer Verbindung der Formel IXc vorhandene funktionelle Gruppen mit Ausnahme der an der Reaktion teilnehmenden Gruppe erforderlichenfalls in geschützter Form vorliegen, oder ein reaktionsfähiges Derivat einer Verbindung der Formel IXc mit einer Verbindung der Formel $H-A^2$, worin $A^2$ die obengenannte Bedeutung hat, wobei darin vorhandene funktionelle Gruppen mit Ausnahme der an der Reaktion teilnehmenden Gruppe erforderlichenfalls in geschützter Form vorliegen, oder mit einem

reaktionsfähigen Derivat davon umsetzen, vorhandene Schutzgruppen abspalten und, wenn erwünscht, eine erhaltene Verbindung der Formel I mit mindestens einer salzbildenden Gruppe in ein Salz überführen. Die Erfindung betrifft auch diejenigen Ausführungsformen des Verfahrens, wobei man von einer auf beliebige Weise erhaltenen Verbindung der Formel IXb oder IXc ausgeht. Die obengenannten Reaktionen werden in analoger Weise, wie bei Verfahrensvariante a) beschrieben, durchgeführt.

Verfahrensvariante e):

Eine weitere Verfahrensvariante zur Herstellung einer Verbindung der Formel I ist dadurch gekennzeichnet, dass man in einer Verbindung der Formel I, worin die Substituenten die obengenannten Bedeutungen haben, wobei aber mindestens eine funktionelle Gruppe durch eine leicht abspaltbare Schutzgruppe geschützt ist, diese Schutzgruppe(n) abspaltet.

Die Schutzgruppen, ihre Einführung und Abspaltung sind bei Verfahrensvariante a) beschrieben. Funktionelle Gruppen, die geschützt sein können, sind insbesondere Hydroxy-, Carboxy- und Aminogruppen, ferner Mercaptogruppen sowie gegebenenfalls Sulfogruppen. Geschützt sein können beliebige dieser funktionellen Gruppen in einem Molekül der Formel I, insbesondere die Gruppen $-X^1-H$ in 1-Stellung des Zuckerteils, -OH in 4-Stellung des Zuckerteils, $-X^2-H$ in 6-Stellung des Zuckerteils sowie freie Carboxylgruppen repräsentiert durch oder enthalten in den Resten $-COR^{10}$, $R^{11}$ und/oder $-COR^{12}$.

Verfahrensvariante f):

Eine weitere Verfahrensvariante zur Herstellung einer Verbindung der Formel I ist dadurch gekennzeichnet, dass man eine Verbindung der Formel X

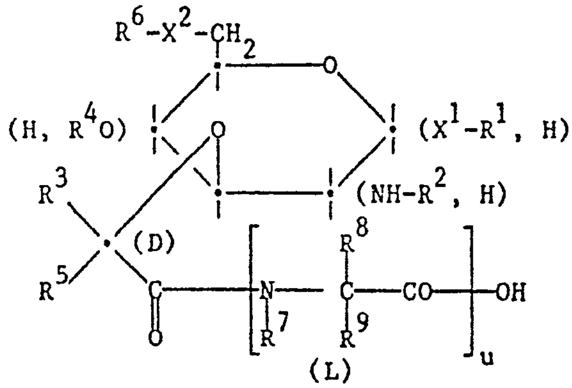

(X)

worin der Index u die untengenannte Bedeutung hat und die Substituenten die obengenannten Bedeutungen haben, wobei in einer Verbindung der Formel X vorhandene Amino- und weitere Carboxygruppen sowie erforderlichenfalls weitere funktionelle Gruppen mit Ausnahme der an der Reaktion teilnehmenden Gruppe in geschützter Form vorliegen, oder ein reaktionsfähiges Säurederivat einer Verbindung der Formel X mit einer Verbindung der Formel XI,

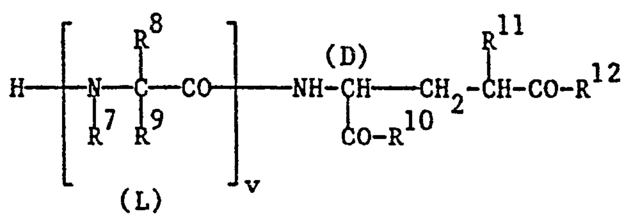

(XI)

worin der Index v für 1 steht, wenn in der Verbindung der Formel X u für 0 steht, oder worin v für 0 steht, wenn u für 1 steht, und die Substituenten die obengenannten Bedeutungen haben, wobei in einer Verbindung der Formel XI vorhandene Carboxyl- und weitere Aminogruppen sowie erforderlichenfalls weitere funktionelle Gruppen mit Ausnahme der an der Rektion teilnehmenden Gruppe in geschützter Form vorliegen, oder mit einem reaktionsfähigen Derivat einer Verbindung der Formel XI umsetzt, vorhandene Schutzgruppen abspaltet und, wenn erwünscht, eine erhaltene Verbindung der Formel I mit mindestens einer salzbildenden Gruppe in ein Salz überführt.

Die Reaktion wird analog Verfahrensvariante a) durchgeführt.

Verfahrensvariante g):

Eine weitere Verfahrensvariante zur Herstellung einer Verbindung der Formel I, worin sich der Zuckerteil von D-Glucose ableitet, $X^1$ und $X^2$ Sauerstoff, $R^1$, $R^4$ und $R^6$ Wasserstoff und $R^2$ unsubstituiertes oder substituiertes Benzoyl bedeuten, ist dadurch gekennzeichnet, dass man in einer Furanoseverbindung der Formel XII,

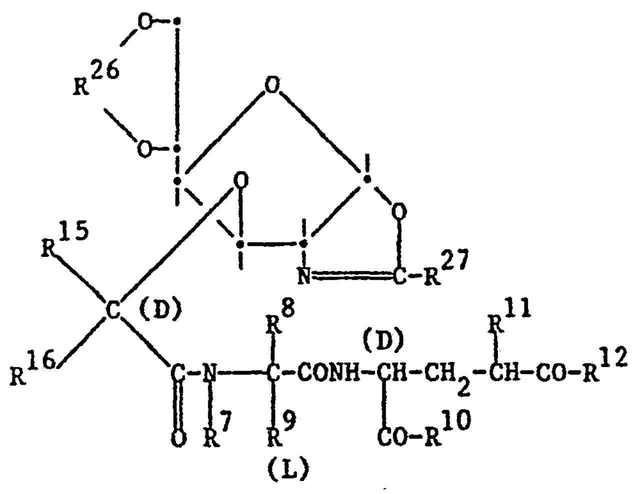

(XII)

worin $R^{26}$ eine bivalente Hydroxyschutzgruppe und $R^{27}$ unsubstituiertes oder substituiertes Phenyl bedeuten und die übrigen Substituenten die obengenannten Bedeutungen haben, den Oxazolin- und den Dioxolanring aufspaltet.

Bivalente Hydroxyschutzgruppen $R^{26}$ sind insbesondere gegebenenfalls substituierte Alkyliden- oder Cycloalkylidengruppen. Alkyliden ist darin insbesondere Niederalkyliden, wie Isopropyliden, und Cycloalkyliden in erster Linie Cyclopentyliden oder Cyclohexyliden. Als Substituenten der Alkylidenreste seine insbesondere aromatische Reste, z.B. Phenylreste, genannt.

Die Substituenten von substituiertem Phenyl $R^{27}$ entsprechen denen von substituiertem Benzoyl $R^2$.

Die Aufspaltung erfolgt vorzugsweise mit verdünnter Säure, am besten bei einem pH-Wert von 2-4, z.B. 3, als Eintopfreaktion in an sich bekannter Weise, z.B. mit 50%iger Essigsäure, einem sauren Ionenaustauscher, insbesondere einem solchen mit Sulfonsäuregruppen, wie Amberlite IR-120 (ein Styrolharz mit stark sauren Sulfogruppen) oder Dowex 50 (Polystyrolsulfonsäuren) oder einer starken anorganischen oder organischen Säure, wie Salzsäure, Bromwasserstoffsäure, Schwefelsäure oder einer Sulfonsäure, z.B. Methansulfonsäure, oder einer gegebenenfalls im aromatischen Ring substituierten Phenylsulfonsäure, wie p-Toluolsulfonsäure, oder Trifluoressigsäure. Arbeitet man dabei in Gegenwart von Wasser, erhält man in 1-Stellung der Glucopyranose eine freie Hydroxygruppe.

Die Ausgangsstoffe der Formel XII erhält man, wenn man in eine Verbindung der Formel XIII,

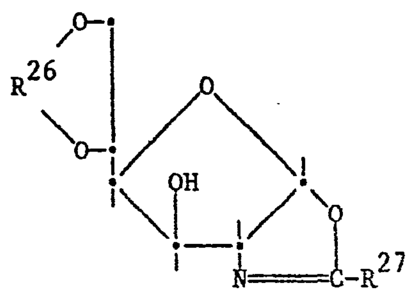

(XIII)

worin die Substituenten die obengenannten Bedeutungen haben, die Seitenkette in einer oder mehreren Stufen, z.B. via eine Verbindung der Formel XIV,

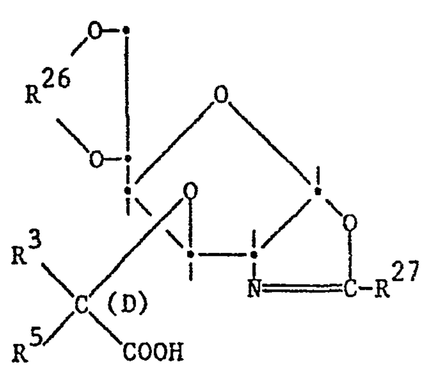

(XIV)

 

worin die Substituenten die obengenannten Bedeutungen haben, einführt.

Verbindungen der Formel XIV, worin $R^3$ für Cycloalkyl und $R^5$ für Wasserstoff stehen, oder worin $R^3$ und $R^5$ zusammen Niederalkyliden, Cycloalkyliden oder unsubstituiertes oder substituiertes Benzyliden bedeuten, sowie deren Salze und reaktionsfähige Säurederivate sind neu und gehören als Zwischenprodukte zur Herstellung der Verbindungen der Formel I via die Verbindungen der Formel XII ebenfalls zum Gegenstand der vorliegenden Erfindung. Die reaktionsfähigen Säurederivate sind analog den bei Verfahrensvariante a) genannten.

Die Zwischenprodukte der Formel XIV, worin $R^3$ und $R^5$ zusammen Niederalkyliden, insbesondere gegebenenfalls durch Niederalkyl substituiertes Methyliden, oder gegebenenfalls substituiertes Benzyliden bedeuten, werden z.B. hergestellt, indem man eine Verbindung der Formel XIII mit Diazo-phosphonessigsäuretriethylester in Gegenwart von Rhodiumdiacetat umsetzt, und die erhaltene Verbindung der Formel XV,

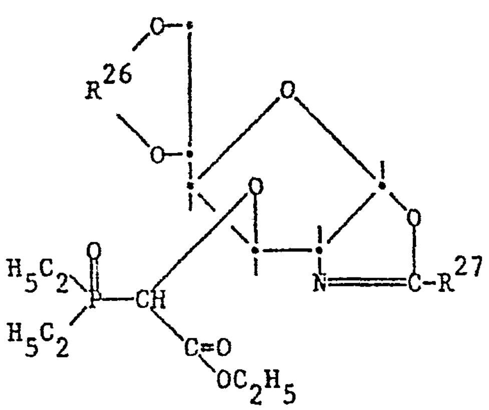

(XV)

worin die Substituenten die obengenannten Bedeutungen haben, mit dem gewünschten Aldehyd, z.B. Paraformaldehyd zur Herstellung der Methyliden-Verbindung, im Sinne einer Wittig-Horner-Reaktion in Gegenwart einer geeigneten Base, z.B. Diazabicycloundecen, umsetzt und anschliessend den erhaltenen Carbonsäureethylester verseift.

Die Verbindungen der Formel XIV, worin $R^3$ für Cycloalkyl und $R^5$ für Wasserstoff stehen, erhält man z.B. in an sich bekannter Weise durch Umsetzung einer Verbindung der Formel XIII mit einem α-Cycloalkyl-α-brom-essigsäure-derivat und einer starken Base, z.B. Natriumhydrid.

Die oben beschriebenen Verfahren, inklusive die Verfahren zur Abspaltung von Schutzgruppen und die zusätzlichen Verfahrensmassnahmen, werden in an sich bekannter Weise, z.B. in An-oder Abwesenheit von Lösungs- und Verdünnungsmitteln, wenn notwendig, in Anwesenheit von Kondensationsmitteln oder Katalysatoren, bei erniedrigter oder erhöhter Temperatur, z.B. in einem Temperaturbereich von etwa -20°C bis etwa 150°C, in einem geschlossenen Gefäss und/oder in einer Inertgas-, z.B. Stickstoffatmosphäre, durchgeführt.

Dabei sind unter Berücksichtigung aller im Molekül befindlichen Substituenten, wenn erforderlich, z.B. bei Anwesenheit leicht hydrolysierbarer Reste, besonders schonende Reaktionsbedingungen, wie kurze Reaktionszeiten, Verwendung von milden sauren oder basischen Mitteln in niedriger Konzentration, stöchiometrische Mengenverhältnisse, Wahl geeigneter Katalysatoren, Lösungsmittel, Temperatur- und/oder Druckbedingungen, anzuwenden.

Die Erfindung betrifft auch diejenigen Ausführungsformen des Verfahrens, bei denen man von einer auf irgendeiner Stufe des Verfahrens als Zwischenprodukt erhältlichen Verbindung ausgeht und die fehlenden Verfahrensschritte durchführt oder das Verfahren auf irgendeiner Stufe abbricht oder einen Ausgangsstoff unter den Reaktionsbedingungen bildet oder in Form eines reaktionsfähigen Derivats oder Salzes verwendet. Dabei geht man vorzugsweise von solchen Ausgangsstoffen aus, die verfahrensgemäss zu den oben als besonders wertvoll beschriebenen Verbindungen führen.

Neue Ausgangsstoffe und/oder Zwischenprodukte sowie Verfahren zu ihrer Herstellung sind ebenfalls Gegenstand der vorliegenden Erfindung. Vorzugsweise werden solche Ausgangsstoffe verwendet und die Reaktionsbedingungen so gewählt, dass man zu den in dieser Anmeldung als besonders bevorzugt aufgeführten Verbindungen gelangt.

Die pharmazeutisch verwendbaren Verbindungen der vorliegenden Erfindung können z.B. zur Herstellung von pharmazeutischen Präparaten verwendet werden, welche eine pharmazeutisch wirkame Menge, z.B. eine zur Immunstimulation ausreichende Menge, der Aktivsubstanz zusammen oder im Gemisch mit einer signifikanten Menge anorganischer oder organischer, fester oder flüssiger, pharmazeutisch verwendbarer Trägerstoffe enthalten.

Bei den erfindungsgemässen pharmazeutischen Präparaten handelt es sich um solche zur enteralen, wie oralen oder rektalen sowie parenteralen, wie intraperitonealen, intramuskulären oder intravenösen Verabreichung an Warmblüter, welche den pharmakologischen Wirkstoff allein oder zusammen mit einem pharmazeutisch anwendbaren Trägermaterial enthalten.

Die Trägerstoffe können anorganisch oder organisch und fest oder flüssig sein. So verwendet man Tabletten oder Gelatinekapseln, welche den Wirkstoff zusammen mit Verdünnungsmitteln, z.B. Lactose, Dextrose, Sukrose, Mannitol, Sorbitol, Cellulose und/oder Glycerin, und/oder Schmiermitteln, z.B. Kieselerde, Talk, Stearinsäure oder Salze davon, wie Magnesium- oder Calciumstearat, und/oder Polyethylenglykol, enthalten. Tabletten können ebenfalls Bindemittel, z.B. Magnesiumaluminiumsilikat, Stärken, wie Mais-, Weizen- oder Reisstärke, Gelatine, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel enthalten. Ferner kann man die pharmakologisch wirksamen Verbindungen der vorliegenden Erfindung in Form von parenteral verabreichbaren Präparaten oder von

Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die genannten Lösungen oder Suspensionen können viskositätserhöhende Stoffe, wie Natrium-Carboxymethylcellulose, Carboxymethylcellulose, Dextran, Polyvinylpyrrolidon oder Gelatine, enthalten. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Drucks und/oder Puffer enthalten. Die vorliegenden pharmazeutischen Präparate, die, wenn erwünscht, weitere pharmakologisch wirksame Stoffe, wie Antibiotika enthalten können, werden in an sich bekannter Weise, z.B. mittels konventioneller Misch-, Granulier-, Dragier-, Lösungs- oder Lyophilisierungsverfahren, hergestellt und enthalten von etwa 0,001 % bis 99 %, insbesondere von etwa 0,01 % bis etwa 10 %, in erster Linie zwischen 0,1 % und 5 %, des bzw. der Aktivstoffe, wobei eine Wirkstoffkonzentration unterhalb von 1 %, insbesondere für topisch zu applizierende Präparate geeignet ist.

Erfindungsgemässe pharmazeutische Präparate können z.B. in Dosiseinheitsform, wie Dragées, Tabletten, Kapseln, Suppositorien oder Ampullen, vorliegen.

Pharmazeutische Präparate zur oralen Anwendung können erhalten werden, indem man den Wirkstoff mit festen Trägerstoffen kombiniert, ein erhaltenes Gemisch gegebenenfalls granuliert und das Gemisch bzw. Granulat, wenn erwünscht oder notwendig nach Zugabe von geeigneten Hilfsstoffen, zu Tabletten oder Dragée-Kernen verarbeitet. Dabei kann man sie auch in Kunststoffträger einbauen, die die Wirkstoffe dosiert abgeben oder diffundieren lassen.

Die Herstellung der Injektionspräparate erfolgt in üblicher Weise unter antimikrobiellen Bedingungen, ebenso das Abfüllen in Ampullen oder Vials sowie das Verschliessen der Behälter.

Die Dosierung des Wirkstoffes hängt von verschiedenen Faktoren, wie Applikationsweise, Spezies, der Abwehrlage des Organismus sowie in entscheidendem Masse von der Art der zu behandelnden Krankheit ab. So liegen die täglich zu verabreichenden Dosen bei oraler Applikation an Warmblüter von etwa 70 kg zwischen etwa 0,0001 und 0,1 g, wobei eine Dosierung von weniger als 0,001 g in erster Linie zur Vermeidung der Metastasenbildung nach Entfernung des Primärtumors verwendet wird.

Die folgenden Beispiele dienen zur Illustration der Erfindung. Temperaturen werden in Celsiusgraden angegeben. $R_f$-Werte werden, wenn nicht anders angegeben, auf Kieselgeldünnschichtplatten (Merck, Darmstadt, Deutschland) ermittelt.

Die Zusammensetzung von Lösungsmittelgemischen ist, wenn nicht anders vermerkt, in Volumenteilen (v/v) angegeben. Die Konzentration, c, der Substanz im Lösungsmittel(gemisch) ist im Falle der optischen Drehung in Prozent (Gewicht/Volumen) angegeben.

Abkürzungen

Boc = tert.Butyloxycarbonyl
i.Vak. = im Vakuum
MeOH = Methanol
PTFE = Polytetrafluorethylen
RT = Raumtemperatur
Smp. = Schmelzpunkt
THF = Tetrahydrofuran
Zers. = Zersetzung

Beispiel 1: 1,51 g (3,75 mMol) 2-{2-[(2,6-Dichlor-phenyl)-amino]-phenyl}-essigsäure-3-amino-2-hydroxy-propylamid-hydrochlorid werden in 50 ml Chloroform-Methanol-Wasser (70:30:5) gelöst. Man tropft 0,784 ml (5,63 mMol) Triethylamin, gelöst in 10 ml obiger Mischung,

zu und trägt bei Raumtemperatur und gutem Rühren 3,80 g (ca. 4,5 mMol) N-Hydroxy-succinimidester von N-Acetyl-muramyl-L-alanyl-D-isoglutamin (ca. 70%ig; enthält zusätzlich noch N-Hydroxy-succinimid und Dicyclohexyl-harnstoff) in fester Form (drei Portionen) zu. Nach 1 1/2 Stunden wird die Suspension filtriert und das Filtrat bei 30° zur Trockene eingedampft. Das als gelbliches Oel anfallende Rohprodukt wird duch zweimalige Chromatographie an Kieselgel 60 [Korngrösse 0,063-0,200 mm (70-230 mesh ASTM)] in Chloroform-Methanol-Wasser (70:30:5) erst bei einem Produkt-Kieselgel-Verhältnis von 1:50 (15 ml Fraktionen), dann 1:90 (8 ml Fraktionen) gereinigt. Die reinen Fraktionen werden gesammelt. Der nach dem Eindampfen des Lösungsmittels verbleibende Rückstand wird in 100 ml eines 1:1-Gemisches von doppelt destilliertem Wasser und tert.-Butanol aufgenommen, die Lösung duch ein Millipore-Filter (PTFE, 0,2 µm) gelassen und lyophilisiert. Man erhält N-Acetyl-muramyl-L-alanyl-D-isoglutamin-3-{2-[2-(2,6-dichlor-phenyl-amino)-phenyl]-acetylamino}-2-hydroxy-propylamid • 1,94 $H_2O$ in Form eines farblosen Pulvers; $[\alpha]_D^{20}$ = +24° (c = 0,908; Methanol), $R_f$ = 0,29 (Chloroform:Methanol:Wasser = 70:30:5), $R_f$ = 0,49 (Acetonitril:Wasser = 3:1), $R_f$ = 0,30 (n-Butanol:Essigsäure:Wasser = 75:7,5:21).

Beispiel 2: 0,200 g (0,187 mMol) N-Acetyl-muramyl-L-alanyl-D-isoglutaminyl-$N^{\varepsilon}$-Boc-L-lysin-3-{2-[2-(2,6-dichlor-phenyl-amino)-phenyl]-acetylamino}-2-hydroxy-propylamid werden in 2 ml auf 0° abgekühlter 99%iger Trifluoressigsäure gelöst. Nach 5minütigem Stehen bei der gleichen Temperatur wird mit 20 ml Methylenchlorid verdünnt und das Ganze bei Raumtemperatur zur Trockene eingedampft (3mal), der ölige Rückstand schliesslich in 15 ml abs. Dioxan aufgenommen und lyophilisiert. Das Rohprodukt (0,185 g) wird durch Flash-Chromatographie [W. Clark Still et al., J.Org.Chem. 43, 2923-25 (1978)] an 50 g Kieselgel 60 [Korngrösse 0,040-0,063 mm (230-400 mesh ASTM)] in Essigsäureethylester-Essigsäure-Wasser-Methanol (67:10:23:12, 5 ml Fraktionen) gereinigt. Die das Produkt enthaltenden Fraktionen werden vereinigt und das Lösungsmittel eingedampft. Der Rückstand wird in 10 ml doppelt destilliertem

Wasser aufgenommen und zur Entfernung von Trifluoressigsäure über 30 ml eines schwach basischen Ionenaustauschers in der Acetat-Form (DOWEX 3, 20/50 mesh) filtriert. Man wäscht mit 70 ml doppelt destilliertem Wasser nach, filtriert die vereinigten Eluate durch ein Millipore-Filter (0,2 µm), fügt 20 ml abs. Dioxan zu und lyophilisiert. Man erhält N-Acetyl-muramyl-L-alanyl-D-isoglutaminyl-L-lysin-3-{2-[2-(2,6-dichlor-phenyl-amino)-phenyl]-acetylamino}-2-hydroxy-propylamid als farbloses, extrem hygroskopisches Pulver; $[\alpha]_D^{20} = +4°$ (c = 0,362; Methanol), $R_f$ = 0,40 (Essigsäure ethylester:Essigsäure:Wasser:Methanol = 67:10:23:12), $R_f$ = 0,20 (Chloroform:Methanol:Wasser:Essigsäure = 70:40:10:5).

Das Ausgangsmaterial erhält man folgendermasen:

Stufe 2.1: Zu einer Lösung von 3,75 g (7,48 mMol) $N^{\alpha}$-Benzyloxycarbonyl-$N^{\varepsilon}$-tert.-butyloxycarbonyl-L-lysin-p-nitrophenylester und 1,80 g (4,44 mMol) 2-[2-(2,6-Dichlor-phenyl-amino)-phenyl]-essigsäure-3-amino-2-hydroxy-propylamid in 20 ml Dimethylformamid-Chloroform (9:1) tropft man 0,62 ml (4,44 mMol) Triethylamin ein und lässt das Ganze unter Rühren während 3 Stunden bei 30° reagieren. Die entstandene Suspension wird eingedampft und der Rückstand 4mal mit je 50 ml Essigsäureethylester, der mit Wasser gesättigt ist, verrieben. Das Unlösliche wird abfiltriert und die vereinigten Essigesterphasen werden nach mehrfachem Waschen mit Wasser getrocknet. Der nach dem Eindampfen verbleibende Rückstand wird in 5 ml Dimethylformamid aufgenommen, 50 ml Essigsäureethylester werden zugefügt und das Produkt wird durch portionenweise Zugabe von 250 ml Petrolether ausgefällt und derselbe Vorgang wird wiederholt. Es verbleibt $N^{\alpha}$-Benzyloxycarbonyl-$N^{\varepsilon}$-tert.butyloxycarbonyl-L-lysin-3-{2-[2-(2,6-dichlor-phenyl-amino)-phenyl]-acetylamino}-2-hydroxy-propylamid als farbloses, amorphes Pulver; $[\alpha]_{546nm}^{20} = -2°$ (c = 0,478; Dimethylformamid), $R_f$ = 0,86 (Acetonitril:Wasser = 3:1), $R_f$ = 0,95 (Chloroform:Methanol:Wasser = 70:30:5), $R_f$ = 0,44 (Chloroform:Isopropanol:Essigsäure = 70:8:2).

Stufe 2.2: 1,80 g (2,46 mMol) $N^{\alpha}$-Benzyloxy-carbonyl-$N^{\varepsilon}$-tert.-butyloxycarbonyl-L-lysin-3-{2-[2-(2,6-dichlor-phenyl-amino)-phenyl]-acetylamino}-2-hydroxy-propylamid werden in 100 ml 1,2-Dimethoxyethan-Dimethylformamid (9:1) gelöst und nach Zugabe von 0,40 g Palladiumauf-Kohle (10%ig) während 1 1/4 Stunden mit Wasserstoff behandelt. Der Katalysator wird abfiltriert, das Filtrat bei 30° am Rotationsverdampfer eingedampft und der Rückstand nach Aufnahme in 100 ml Dioxan-Wasser (7:3) lyophilisiert. Man erhält $N^{\varepsilon}$-tert.-Butyloxycarbonyl-L-lysin-3-{2-[2-(2,6-dichlor-phenyl-amino)-phenyl]-acetylamino}-2-hydroxy-propylamid als farbloses Pulver, das direkt weiter verarbeitet wird; $R_f$ = 0,52 (Acetonitril:Wasser = 3:1), $R_f$ = 0,66 (Essigsäureethylester:n-Butanol:Pyridin:Essigsäure:Wasser = 42:21:21:6:10).

Stufe 2.3: 1,195 g (2 mMol) $N^{\varepsilon}$-Boc-L-lysin-3-{2-[2-(2,6-dichlor-phenyl-amino)-phenyl]-acetylamino}-2-hydroxy-propylamid werden in 50 ml Dimethylformamid-Chloroform (4:1) gelöst und dazu werden bei Raumtemperatur unter Rühren 2,10 g (ca. 2,4 mMol) N-Hydroxy-succinimidester von N-Acetyl-muramyl-L-alanyl-D-isoglutamin (ca. 70%ig) in fester Form in mehreren Portionen eingetragen. Der pH-Wert der Reaktionslösung wird durch tropfenweise Zugabe einer 5%igen Lösung von Triethylamin in Chloroform auf ca. 7 gehalten. Nach 16stündigem Rühren wird die erhaltene Suspension bei 30° zur Trockene eingedampft und der Rückstand zweimal mit je 100 ml Essigsäureethylester-Wasser (1:1) gut gerührt. Das unlösliche Material wird abgesaugt, getrocknet (0,91 g) und an Kieselgel 60 (Korngrösse 0,04-0,063 mm) erst mit Chloroform, dann mit Chloroform-Methanol-Gemischen (95:5 bis 60:40) einer Flash-Chromatographie (1:100, 5 ml Fraktionen; 0,4 bar) unterworfen. Die das Produkt enthaltenden Fraktionen werden gesammelt. Der nach dem Eindampfen des Lösungsmittelgemisches verbleibende Rückstand wird in 50 ml abs. Dioxan aufgenommen und lyophilisiert. Man erhält N-Acetyl-muramyl-L-alanyl-D-isoglutaminyl-$N^{\varepsilon}$-Boc-L-lysin-3-{2-[2-(2,6-dichlor-phenyl)-amino-phenyl]-acetylamino}-2-hydroxy-propylamid als farbloses Pulver; $[\alpha]_D^{20}$ = +15°

(c = 0,196; Methanol), $R_f$ = 0,39 (Chloroform:Methanol:Wasser = 70:30:5), $R_f$ = 0,66 (Essigsäureethylester:n-Butanol:Pyridin:Essigsäure:Wasser = 42:21:21:6:10).

Beispiel 3: 0,250 g (0,40 mMol) N-Acetyl-muramyl-L-alanyl-D-isoglutaminyl-L-lysin werden in 10 ml abs. Dimethylformamid gelöst und unter Rühren erst 0,056 ml (0,40 mMol) Triethylamin, dann 0,237 g (0,60 mMol) N-Hydroxy-succinimidester von 2-[2-(2,6-Dichlor-phenyl-amino)-phenyl]-essigsäure eingetragen. Nach 8 Stunden Stehen bei Raumtemperatur wird die Lösung am Rotationsverdampfer im Hochvakuum eingedampft. Der nach mehrfachem Verreiben mit Essigsäureethylester verbleibende Rückstand (0,28 g) wird durch Chromatographie an 20 g Kieselgel 60 (Korngrösse 0,04-0,063 mm) in Chloroform-Methanol-Wasser (70:30:5) (0,6 ml Fraktionen) wie üblich gereinigt. Die reinen Fraktionen werden gesammelt, in 12 ml doppelt destilliertem Wasser gelöst und die Lösung nach Filtration durch ein Millipore-Filter (0,2 µm) lyophilisiert. Man erhält N-Acetyl-muramyl-L-alanyl-D-isoglutaminyl-$N^{\varepsilon}$-{2-[2-(2,6-dichlor-phenyl-amino)-phenyl]-acetyl}-L-lysin • 3,3 $H_2O$ als farbloses Pulver; $[a]_D^{20}$ = +26° (c = 0,364; Methanol), $R_f$ = 0,17 (Chloroform:Methanol:Wasser = 70:30:5), $R_f$ = 0,22 (n-Butanol:Essigsäure:Wasser = 75:7,5:21), $R_f$ = 0,28 (Essigsäureethylester:n-Butanol:Pyridin:Essigsäure:Wasser = 42:21:21:6:10).

Beispiel 4: Analog Beispiel 3 erhält man aus N-Acetyl-desmethylmuramyl-L-alanyl-D-isoglutaminyl-L-lysin mit überschüssigem N-Hydroxy-succinimidester von 2-[2-(2,6-Dichlor-phenyl-amino)-phenyl]-essigsäure N-Acetyl-desmethylmuramyl-L-alanyl-D-isoglutaminyl-$N^{\varepsilon}$-{2-[2-(2,6-dichlor-phenyl-amino)-phenyl]-acetyl}-L-lysin • 2,33 $H_2O$ als farbloses Pulver; $[a]_D^{20}$ = +4° (c = 0,569; Methanol), $R_f$ = 0,17 (Chloroform:Methanol:Wasser = 70:30:5), $R_f$ = 0,24 (n-Butanol:Essigsäure:Wasser = 75:7,5:21), $R_f$ = 0,29 (Essigsäureethylester:n-Butanol:Pyridin:Essigsäure:Wasser = 42:21:21:6:10).

 

Beispiel 5: In 8 ml absolutem Dimethylacetamid löst man 300 mg (0,745 mMol) 2-{2-[(2,6-Dichlor-phenyl)-amino]-phenyl}-essigsäure-4-amino-butylamid-hydrochlorid, 385 mg (0,78 mMol) N-Acetyl-desmethyl-muramyl-L-alanyl-D-isoglutamin, 193 mg (1,67 mMol) N-Hydroxy-succinimid, 308 mg (1,49 mMol) Dicyclohexylcarbodiimid und 0,113 ml (0,82 mMol) Triethylamin. Man lässt die klare Lösung 15 Stunden bei Raumtemperatur stehen und dampft dann am Hochvakuum zur Trockene. Der Rückstand wird zweimal zwischen Tetrahydrofuran und gesättigter wässriger NaCl-Lösung verteilt, die organische Phase mit $Na_2SO_4$ getrocknet und im Vakuum eingedampft. Der Rückstand wird dreimal mit je 30 ml Chloroform heiss extrahiert und einmal mit heissem Dimethoxyethan-Methanol (9:1). Man erhält so N-Acetyl-desmethyl-muramyl-L-alanyl-D-isoglutamin-4-{2-[2-(2,6-dichlor-phenyl-amino)-phenyl]-acetylamino}-butylamid; Smp. 176-178°; $R_f$ = 0,375 (Chloroform:Methanol = 7:3), $[\alpha]_D^{20}$ = +17,2° (c = 1,166; Dimethylformamid).

Die Ausgangsverbindung erhält man folgendermassen:
Stufe 5.1: 1,4-Diamino-butan und 2-{2-[(2,6-Dichlor-phenyl)-amino]-phenyl}-essigsäure-p-nitro-phenyl-ester lässt man 8 Stunden in Methylenchlorid-Methanol (1:1) bei Raumtemperatur reagieren. Die Reaktionslösung wird anschliessend im Vakuum eingedampft, der Rückstand in Tetrahydrofuran aufgenommen und mit 2N Salzsäure ausgeschüttelt. Die wässrige Phase extrahiert man dreimal mit Diethylether zur Entfernung von Nitrophenol, sättigt sie dann mit NaCl und extrahiert mit Tetrahydrofuran. Die organische Phase wird mit $Na_2SO_4$ getrocknet und eingedampft. Der Rückstand wird mit Diethylether verrieben und liefert ein kristallines Pulver, das aus Essigsäureethylester umkristallisiert wird. Man erhält 2-{2-[(2,6-Dichlor-phenyl)-amino]-phenyl}-essigsäure-4-amino-butylamid-hydrochlorid als Essigester-Solvat; Smp. 137-140°; $R_f$ = 0,177 ($CHCl_3$:Methanol:konz. wässrige Ammoniaklösung = 10:10:0,1).

Beispiel 6: 1,9 g (4,69 mMol) 2-{2-[(2,6-Dichlor-phenyl)-amino]-phenyl}-essigsäure-3-amino-2-hydroxy-propylamid-hydrochlorid, 2,65 g (4,47 mMol) N-Propionyl-desmethylmuramyl-L-alanyl-D-isoglutaminylalanin-hydrat, 0,772 g (6,7 mMol) N-Hydroxy-succinimid, 2,31 g (11,2 mMol) Dicyclohexylcarbodiimid und 0,68 ml Triethylamin löst man in 40 ml absolutem Dimethylacetamid und lässt 15 Stunden bei Raumtemperatur reagieren. Dann dampft man die Lösung im Vakuum zur Trockene, nimmt den Rückstand mit Tetrahydrofuran auf, schüttelt die Lösung viermal mit gesättigter, wässriger NaCl-Lösung aus, trocknet die organische Phase mit $Na_2SO_4$ und dampft zur Trockene. Dieser Rückstand wird viermal mit je 50 ml heissem Chloroform und dreimal mit je 30 ml einer heissen Mischung aus 9 Teilen Tetrahydrofuran und 1 Teil Methanol extrahiert. Die heissen Extrakte lässt man jeweils auf Raumtemperatur abkühlen, dabei kristallisiert die teilweise in Lösung gegangene Substanz wieder aus und wird abgesaugt. Man erhält so farblose Kristalle von N-Propionyl-desmethylmuramyl-L-alanyl-D-isoglutaminyl-L-alanin-3-{2-[2-(2,6-dichlor-phenyl-amino)-phenyl]-acetylamino}-2-hydroxy-propylamid; Smp. 192-198°, $[\alpha]_D^{20}$ = +9°(c= 1,12; Dimethylformamid), $R_f$ = 0,63 ($CHCl_3$:MeOH = 7:3).

Die Ausgangsmaterialien erhält man wie folgt:
Stufe 6.1: 2,6 g (28,8 mMol) 1,3-Diamino-propan-2-ol in 10 ml MeOH werden tropfenweise mit einer Lösung von 3,5 g (8,4 mMol) 2-{2-[(2,6-Dichlor-phenyl)-amino]-phenyl}-essigsäure-p-nitro-phenyl-ester versetzt. Nach 5 Stunden bei Raumtemperatur dampft man im Vakuum zur Trockene. Den Rückstand nimmt man mit Tetrahydrofuran auf, versetzt mit 2N Salzsäure bis pH = 2 und extrahiert die wässrige Phase dreimal mit Diethylether zur Entfernung von Nitrophenol. Die wässrige Phase sättigt man mit NaCl und extrahiert das Produkt mit Tetrahydrofuran. Nach Trocknen mit $Na_2SO_4$, Eindampfen und Verreiben mit Diethylether kristallisiert 2-{2-[(2,6-Dichlor-phenyl)-amino]-phenyl}-essigsäure-3-amino-2-hydroxy-propylamid-hydrochlorid. Die Substanz nimmt an der Luft Wasser auf und kristallisiert mit etwa 1,5 Mol Wasser. Zersetzungspunkt 92°, $R_f$ = 0,3 (Acetonitril:Wasser = 8:2 auf Dünnschichtplatten Opti-$UPC_{12}$, reversed phase, Kieselgel, Antec AG, CH-4431 Bennwil).

Stufe 6.2: Aus α-Benzyl-N-propionyl-desmethylmuramyl-L-alanyl-D-iso-glutaminyl-L-alanin-benzylester erhält man durch katalytische Hydrierung mit 10%iger Pd/Kohle in Tetrahydrofuran-Wasser (4:1) und Lyophilisieren der vom Katalyator und THF befreiten wässrigen Lösung N-Propionyl-desmethylmuramyl-L-alanyl-D-isoglutaminyl-L-alaninhydrat als farbloses Pulver; Smp. 141-150°; $[\alpha]_D^{20}$ = -10° (c = 0,943; $H_2O$), $R_f$ = 0,36 ($CH_3CN:H_2O$ = 3:1).

Beispiel 7: 1 g (2,43 mMol) N-{[2-(2,6-Dichlor-phenyl-amino)-phenyl]-acetyl}-L-asparaginsäure und 0,6 g (3,16 mMol) N-Ethyl-N'-(dimethylaminopropyl)-carbodiimid-hydrochlorid lässt man in 25 ml absolutem Dimethylformamid 3 Stunden bei Raumtemperatur reagieren. Dabei bildet sich das Anhydrid. Zu dieser Lösung gibt man dann 1,58 g (3,16 mMol) N-Acetyl-desmethylmuramyl-L-alanyl-D-isoglutaminnatriumsalz und 0,37 ml Triethylamin. Nach weiteren 6 Stunden bei Raumtemperatur dampft man im Vakuum zur Trockene, nimmt den Rückstand mit Wasser auf, stellt den pH-Wert mit 1N Salzsäure auf 3 und extrahiert die Lösung dreimal mit Essigsäureethylester. Nach Trocknen der organischen Phase mit $Na_2SO_4$ dampft man im Vakuum ein; der Rückstand wird aus Methanol-Dimethoxyethan umgefällt. Man erhält farbloses N-Acetyl-6-O-{N-<[2-(2,6-dichlor-phenyl-amino)-phenyl]-acetyl>-asparagin-ß-yl}-desmethylmuramyl-L-alanyl-D-isoglutamin; Smp. 184° (Zersetzung), $[\alpha]_D^{20}$ = +21° (c = 1,02; Dimethylformamid), $R_f$ = 0,266 ($CHCl_3$: $MeOH:H_2O$ = 55:45:3).

Das Ausgangsmaterial gewinnt man folgendermassen:
Stufe 7.1: Zu 2,00 g L-Asparaginsäure-dinatriumsalz in 10 ml Methanol-Wasser (1:1) tropft man eine Lösung von 3,9 g des N-Hydroxy-succinimidesters von 2-[2-(2,6-Dichlor-phenyl-amino)-phenyl]-essigsäure in Dimethylformamid. Die Lösung wird nach 1 Stunde bei Raumtemperatur im Vakuum eingedampft, der Rückstand mit Essigsäureethylester und verdünnter Salzsäure aufgenommen, verteilt, die Essigester-Phase mit Wasser gewaschen, mit $Na_2SO_4$ getrocknet und eingedampft. Durch Verreiben des Rückstandes mit Diethylether erhält

man Kristalle von N-{[2-(2,6-dichlor-phenyl-amino)-phenyl]-acetyl}-asparaginsäure; Smp. 157-160° (Zers.), $R_f$ = 0,17 ($CHCl_3$: MeOH = 1:1).

Beispiel 8: 3,41 g (7,21 mMol) L-Alanin-4-{2-[2-(2,6-dichlor-phenyl-amino)-phenyl]-acetylamino}-butylamid-hydrochlorid werden in 60 ml abs. Dimethylformamid gelöst, 0,83 ml (7,57 mMol) N-Methyl-morpholin zugetropft und analog Beispiel 1 werden dazu 7,22 g (8,65 mMol) N-Acetyl-desmethylmuramyl-L-alanin-D-isoglutamin-N-hydroxy-succinimidester (ca. 70%ig) in drei Portionen eingetragen. Nach Rühren über Nacht bei Raumtemperatur wird zur Trockne verdampft. Der Rückstand wird erst analog Beispiel 1 durch zweimalige Chromatographie an Kieselgel 60 (1:55; 11 ml Fraktionen) in Chloroform-Methanol-Wasser [70:30:5 (v/v)], dann durch Reverse phase-Chromatographie (Opti $UPC_{12}$; 40-63 µm; 300:1) gereinigt. Als Fliessmittel dient ein Gradient von Acetonitril:Wasser = 1:4 bis 3:1 (v/v). Die reinen Fraktionen werden gesammelt und das Lösungsmittel verdampft. Der Rückstand wird in 100 ml doppeltdestilliertem Wasser aufgenommen und durch Zugabe von 20 ml tert.Butanol in Lösung gebracht. Nach Filtration durch ein Milliporefilter (0,45 µ) und Lyophilisation erhält man N-Acetyl-desmethylmuramyl-L-alanyl-D-iso-glutaminyl-L-alanin-4-{2-[2-(2,6-dichlor-phenyl-amino)-phenyl]-acetylamino}-butylamid•2,3 $H_2$ als farbloses Pulver; $[\alpha]_D^{20}$ = + 13,9° (c = 0,505; Dimethylformamid), $R_f$ = 0,32 (Chloroform:Methanol:Wasser = 70:30:5), $R_f$ = 0,49 (Essigsäureethylester:n-Butanol:Pyridin:Essigsäure:Wasser = 42:21:21:6:10).

Das Ausgangsmaterial erhält man folgendermassen:
Stufe 8.1: 8,48 g (30 mMol) N-Benzyloxycarbonyl-L-alanin, 5,68 g (49,4 mMol) N-Hydroxy-succinimid und 15,30 g (38 mMol) 2-{2-[(2,6-Dichlor-phenyl)-amino]-phenyl}-essigsäure-4-amino-butylamid (Herstellung vgl. Stufe 5.1) werden in 200 ml abs. Dimethylformamid gelöst, 4,19 ml (38 mMol) N-Methyl-morpholin zugetropft und schliesslich 10,19 g (49,4 mMol) Dicyclohexylcarbodiimid zugefügt. Man rührt während 16 Stunden bei Raumtemperatur. Die gelbe Suspension wird bei 30° im Vakuum zur Trockne verdampft und der Rückstand in 300 ml Essigsäureethylester aufgenommen. Der unlösliche Dicyclohexylharnstoff wird abfiltriert, die Essigesterphase wie üblich erst mit gesättigter Natriumhydrogencarbonatlösung (5 x 100 ml), dann mit 2N Zitronensäurelösung (4 x 100 ml) und dann mit Wasser bzw. gesättigter Kaliumchlorid-Lösung gewaschen. Nach Trocknen über Natriumsulfat wird die Lösung auf etwa 150 ml eingeengt und unter Rühren portionenweise mit total einem Liter Diethylether versetzt. Die ausgeschiedenen Kristalle werden nach Stehen über Nacht bei -10°C abgesaugt. Aus der Mutterlauge erhält man eine zweite Fraktion. Beide werden gemeinsam aus 35 ml Chloroform-Methanol-Wasser (70:30:5) und 600 ml Diethylether-Petrolether (1:1) umkristallisiert.

Man erhält N-Benzyloxycarbonyl-L-alanin-4-{2-[2-(2,6-dichlor-phenylamino)-phenyl]-acetylamino}-butylamid, als schwach gelbliches Pulver; Smp. 146-148°, $[\alpha]_D^{20}$ = -7,5 (c = 0,533; Methanol), $R_f$ = 0,50 (Chloroform:Isopropanol:Essigsäure = 70:8:2), $R_f$ = 0,78 (Chloroform:Methanol:Wasser = 70:30:5).

Stufe 8.2: 11,20 g (19,6 mMol) N-Benzyloxycarbonyl-L-alanin-4-{2-[2-(2,6-dichlor-phenyl-amino)-phenyl]-acetylamino}-butylamid werden in 200 ml Methanol gelöst und nach Zugabe von 1 g Pd auf Kohle (10%ig) hydriert, wobei der pH-Wert der Lösung durch Zugabe von einnormaler Salzsäure in Methanol konstant auf 4,5 gehalten wird. Der Katalysator wird abfiltriert, die Lösung stark eingeengt und nach Zugabe von 100 ml abs. Dioxan lyophilisiert. Man erhält L-Alanin-4-{2-[2-(2,6-dichlor-phenyl-amino)-phenyl]-acetylamino}-butylamid-hydrochlorid·0,9 Dioxan als schwach gelbliches Pulver; $R_f$ = 0,43 (Chloroform:Methanol:Wasser = 70:30:5), $R_f$ = 0,30 (Essigsäureethylester:n-Butanol:Pyridin:Essigsäure:Wasser = 42:21:21:6:10).

Beispiel 9: Nach den beschriebenen Methoden erhält man N-Benzoyl-desmethylmuramyl-L-alanyl-D-isoglutaminyl-L-alanin-3-{2-[2-(2,6-dichlor-phenyl-amino)-phenyl]-acetylamino}-2-hydroxy-propylamid,

N-{2-[2-(2,6-dichlor-phenyl-amino)-phenyl]-acetoxy}-acetyl-muramyl-L-alanyl-D-isoglutamin, N-Acetyl-1-desoxy-1ß-{2-[2-(2,6-dichlor-phenyl-amino)-phenyl]-acetyl}-thiodesmethylmuramyl-L-alanyl-D-isoglutamin-methylester, N-Acetyl-1-desoxy-1ß-{2-[2-(2,6-dichlor-phenyl-amino)-phenyl]-acetyl}-thiodesmethylmuramyl-L-alanyl-D-glutamin-methylester, N-Acetyl-1-desoxy-1ß-{2-[2-(2,6-dichlor-phenyl-amino)-phenyl]-acetyl}-amino-desmethylmuramyl-L-alanyl-D-glutamin-methylester, S-(N-Acetyl-desmethylmuramyl-L-alanyl-D-isoglutaminyl)-N-{2-[2-(2,6-dichlor-phenyl-amino)-phenyl]-acetyl}-L-cystein-methylester, N-Acetyl-muramyl-L-alanyl-D-isoglutaminyl-L-alanin-3-{2-[2-(2,6-dichlor-phenyl-amino)-phenyl]-acetylamino}-2-hydroxy-propylamid, N-Acetyl-muramyl-L-alanyl-D-isoglutaminyl-L-alanin-2-{2-[2-(2,6-dichlor-phenyl-amino)-phenyl]-acetoxy}-ethyl-amid, $N^{\alpha}$-(N-Acetyl-muramyl-L-alanyl-D-isoglutaminyl)-$N^{\beta}$-{2-[2-(2,6-dichlor-phenyl-amino)-phenyl]-acetyl}-α,ß-diamino-propionsäure, $N^{\alpha}$-(N-Acetyl-muramyl-L-alanyl-D-isoglutaminyl)-$N^{\delta}$-{2-[2-(2,6-dichlor-phenyl-amino)-phenyl]-acetyl}-L-ornithin, N-(N-Acetyl-des-methylmuramyl-L-alanyl-D-isoglutaminyl)-S-2-{2-[2-(2,6-dichlor-phenyl-amino)-phenyl-acetylamino}-ethyl-L-cystein, N-(N-Acetyl-muramyl-L-alanyl-D-isoglutaminyl)-O-{2-[2-(2,6-dichlor-phenyl-amino)-phenyl]-acetylamino}-ethyl-L-serin, N-(N-Acetyl-muramyl-L-alanyl-D-isoglutaminyl)-O-{2-[2-(2,6-dichlor-phenyl-amino)-phenyl]-acetoxy}-L-serin, N-Acetyl-muramyl-L-alanyl-D-(γ-methoxycarbonyl)-isoglutaminyl-L-alanin-3-{2-[2-(2,6-dichlor-phenyl-amino)-phenyl]-acetylamino}-2-hydroxy-propylamid, N-Acetyl-muramyl-L-alanyl-D-isoglutaminyl-L-alanin-3-{2-[1-(4-chlor-benzoyl)-5-methoxy-2-methyl-indol-3-yl]-acetylamino}-2-hydroxy-propylamid, N-Propionyl-desmethylmuramyl-L-alanyl-D-glutamyl-($C_{\alpha}$)-methylester-($C_{\gamma}$)-L-alanin-3-{2-[1-(4-chlor-benzoyl)-5-methoxy-2-methyl-indol-3-yl]-acetyl-amino}-2-hydroxy-propylamid, N-Propionyl-desmethylmuramyl-L-alanyl-D-isoglutaminyl-L-alanin-3-[2-(3-benzoyl-phenyl)-propionylamino]-2-hydroxy-propylamid, N-Propionyl-desmethylmuramyl-L-alanyl-D-iso-glutaminyl-L-alanin-3-[2-(6-methoxy-naphth-2-yl)-propionylamino]-2-hydroxy-propylamid, N-Propionyl-desmethylmuramyl-L-alanyl-D-iso-glutaminyl-L-alanin-3-[2-(4-isobutyl-phenyl)-propionylamino]-2-hydroxy-propylamid, N-Propionyl-desmethylmuramyl-L-alanyl-D-iso-

glutaminyl-L-alanin-3-{2-[3-chlor-4-(2,5-dihydro-1H-pyrrol-1-yl)-phenyl]-propionylamino}-2-hydroxy-propylamid und N-Acetyl-muramyl-L-alanyl-D-isoglutaminyl-L-alanin-3-{2-[3-chlor-4-(2,5-dihydro-1H-pyrrol-1-yl)-phenyl]-propionylamino}-2-hydroxy-propylamid.

Beispiel 10: Analog Beispiel 1 werden 0,70 g (1,49 mMol) L-Alanin-2-{2-[2-(2,6-dichlor-phenylamino)-phenyl]-acetoxy}-ethylamid-acetat, gelöst in 25 ml Dimethylformamid, mit 1,68 g (1,78 mMol) des N-Hydroxysuccinimidesters von N-Acetyl-muramyl-L-alanyl-D-isoglutamin (ca. 60%ig) versetzt und während fünf Stunden bei Raumtemperatur gerührt. Die Suspension wird bei 30° im Hochvakuum stark eingeengt, mit abs. Dioxan versetzt und lyophilisiert. Der rötliche Rückstand wird durch Chromatographie an Kieselgel (1:50; 7 ml Fraktionen) erst in Chloroform, dann Chloroform-Methanol-Gemischen (9:1, 85:15, 8:2; jeweils 500 ml) gereinigt. Die das Produkt enthaltenden Fraktionen werden gesammelt und auf einer zweiten Säule wie oben, jedoch im System Chloroform-Methanol-Wasser (70:30:5), gereinigt. Das in den Fraktionen 20-55 enthaltene Material wird gesammelt, in 5 ml Chloroform aufgenommen und durch portionenweise Zugabe von 100 ml Diethylether gefällt, dann eine Stunde bei 0° gerührt. Der Niederschlag wird abfiltriert, im doppelt destilliertem Wasser plus Zusätze von tert. Butanol und Dioxan gelöst, durch ein Millipore-Filter (0,2 µm) filtriert und lyophilisiert.

Man erhält die in Beispiel 9 genannte Verbindung N-Acetyl-muramyl-L-alanyl-D-isoglutaminyl-L-alanin-2-{2-[2-(2,6-dichlor-phenylamino)-phenyl]-acetoxy}-ethylamid (α,β-Gemisch) als farbloses Pulver, 1,6 Mol Wasser und 2,3 Mol tert.Butanol enthaltend;
$[\alpha]_D^{20} = -22,7 \pm 4,4°$ (c = 0,229; Dioxan), $R_f$ = 0,33 (Chloroform:Methanol:Wasser = 70:30:5), $R_f$ = 0,43 (Acetonitril:Wasser = 3:1), $R_f$ = 0,57 (Chloroform:Methanol:Wasser:Essigsäure = 75:27:5:0,5).

Das Ausgangsmaterial erhält man wie folgt:

Stufe 10.1: 8,00 g (25 mMol) N-Benzyloxycarbonyl-L-alanin-N-hydroxysuccinimidester werden in 40 ml abs. Tetrahydrofuran gelöst und unter Rühren 1,53 g (25 mMol) 2-Amino-ethanol, gelöst in 10 ml Tetrahydrofuran, eingetropft. Die entstandene weisse Suspension wird nach vierstündigem Rühren filtriert, der Niederschlag mit Diethylether gewaschen und das Filtrat zur Trockene verdampft. Der Rückstand wird mehrfach zwischen Essigsäureethylester Diethylether-Petrolether und Wasser verteilt. Die wässrige Phase wird stark eingeengt, in 80 ml doppelt destilliertem Wasser aufgenommen und lyophilisiert. Man erhält N-Benzyloxycarbonyl-L-alanin-ethanolamid als farbloses Pulver; $[\alpha]_D^{20} = -14 \pm 1°$ (c = 0,484; Wasser), $R_f$ = 0,60 (Chloroform:Methanol:Wasser = 70:30:5), $R_f$ = 0,77 (Essigsäureethylester:n-Butanol:Pyridin:Essigsäure:Wasser = 42:21:21:6:10).

10.2: 1,94 g (7,3 mMol) N-Benzyloxycarbonyl-L-alanin-ethanolamid und 2,81 g (7,3 mMol) 2-[(2,6-Dichlor-phenyl)-N-benzylamino]-phenyl-essigsäure, gelöst in 50 ml abs. Dimethylformamid, werden unter Rühren mit 1,97 g (14,6 mMol) 1-Hydroxy-benztriazol, 0,89 g (7,3 mMol) Dimethylaminopyridin und schliesslich mit 1,66 g (8,03 mMol) Dicyclohexylcarbodiimid versetzt. Nach 16stündigem Rühren bei Raumtemperatur wird die Suspension mit 500 ml Essigsäureethylester versetzt und der Niederschlag nach kurzem Rühren abfiltriert. Das Filtrat wird nacheinander dreimal mit je 100 ml Wasser, gesättigter Natriumhydrogencarbonat-Lösung, 2N Zitronensäurelösung und Wasser extrahiert. Der nach dem Verdampfen des Lösungsmittels verbleibende Rückstand wird durch Flashchromatographie (0,65 bar; vergl. Beispiel 2) an 500 g Kieselgel gereinigt, und zwar erst mit Chloroform (500 ml), dann mit Chloroform-Dimethoxyethan (95:5 und 9:1; 1 Liter bzw. 3 Liter). Das in den Fraktionen 71-150 enthaltene Material wird gesammelt. Zur weiteren Reinigung wird ein Teil des erhaltenen Materials durch Umkehrphasen-Chromatographie (Opti $UPC_{12}$; 40-63 µm, 1:300) mit Essigsäureethylester als Fliessmittel gereinigt.

Die reinen Fraktionen werden gesammelt, in abs. Dioxan aufgenommen, und lyophilisiert. Man erhält N-Benzyloxycarbonyl-L-alanin-2-{2-[2-(2,6-dichlorphenyl-N-benzylamino)-phenyl]-acetoxy}-ethylamid als farbloses Pulver, 0,36 Mol Dioxan enthaltend;

$[\alpha]^{20}_{546\ nm}$ = +3,6 ± 1,1° (c = 0,926; Dioxan), $R_f$ = 0,77 (Acetonitril:Wasser = 3:1), $R_f$ = 0,93 (Chloroform:Methanol:Wasser = 70:30:5).

Stufe 10.3: 1,00 g (1,58 mMol) N-Benzyloxycarbonyl-L-alanin-2-{2-[2-(2,6-dichlor-phenyl-N-benzylamino)-phenyl]-acetoxy}-ethylamid, gelöst in 20 ml Essigsäure, werden nach Zusatz von 0,1 g Pd/Kohle (10%ig) wie üblich mit Wasserstoff behandelt. Der Katalysator wird abfiltriert und die Lösung lyophilisiert. Man erhält L-Alanin-2-{2-[2-(2,6-dichlor-phenylamino)-phenyl-acetoxy}-ethylamid als Acetat, $R_f$ = 0,38 (Chloroform:Methanol:Wasser = 70:30:5), $R_f$ = 0,48 (Essigsäureethylester:Essigsäure:Wasser: Methanol = 67:10:23:12).

Beispiel 11: 3,30 g (4,94 mMol) N-Acetyl-muramyl-L-alanyl-D-isoglutaminyl-L-ornithin und 2,73 g (6,93 mMol) N-Hydroxysuccinimidester von 2-[2-(2,6-Dichlor-phenylamino)-phenyl]-essigsäure werden analog Beispiel 3 umgesetzt und das rohe Material durch Chromatographie an Kieselgel (1:28, 5 ml Fraktionen) im System Chloroform: Methanol:Wasser (70:30:5) gereinigt. Die reinen Fraktionen werden in 60 ml doppelt destilliertem Wasser aufgenommen, durch ein Millipore-Filter (0,2 µm) filtriert und lyophilisiert. Man erhält $N^{\alpha}$-(N-Acetyl-muramyl-L-alanyl-D-isoglutaminyl)-$N^{\delta}$-{2-[2-(2,6-dichlor-phenyl-amino)-phenyl]-acetyl}-L-ornithin (α,β-Gemisch), als farbloses Pulver, 3 Mol Wasser enthaltend; $[\alpha]^{20}_{D}$ = +20 ± 2,2° (c = 0,480; Methanol), $R_f$ = 0,07 (Chloroform:Methanol:Wasser = 70:30:5), $R_f$ = 0,20 (n-Butanol:Essigsäure:Wasser = 75:7,5:21), $R_f$ = 0,27 (Essigsäureethylester:n-Butanol:Pyridin:Essigsäure:Wasser = 42:21:21:6:10).

Das Ausgangsmaterial erhält man wie folgt.

Stufe 11.1: 4,40 g (11,2 mMol) $N^{\delta}$-Benzyloxycarbonyl-L-ornithin-benzylester-hydrochlorid, gelöst in 50 ml Dimethylformamid, werden analog Beispiel 1 mit 14,73 g (14,55 mMol) N-Acetyl-muramyl-L-alanyl-D-isoglutamin-N-hydroxysuccinimidester (etwa 60%ig) umgesetzt. Nach 4 Stunden werden 300 ml Essigsäureethylester eingetropft und die Suspension wird während einer Stunde bei 0° gerührt. Der Niederschlag wird abgesaugt, gewaschen, dann wieder in 50 ml Dimethylformamid aufgenommen und wie oben gefällt (zweimal). Das Unlösliche wird abfiltriert, in 35 ml Dimethylformamid aufgenommen und durch Zutropfen von 500 ml Wasser ausgefällt. Nach zweistündigem Rühren bei 0° wird der Niederschlag abgesaugt, gewaschen und getrocknet; $R_f$ = 0,37 (n-Butanol:Essigsäure:Wasser = 75:7,5:21), $R_f$ = 0,75 (Essigsäureethylester:Essigsäure:Wasser:Methanol = 67:10:23:12).

Das Rohprodukt, das noch Dicyclohexylharnstoff enthält, wird in 100 ml Eisessig gelöst und nach Zugabe von 0,70 g Pd/Kohle (10%ig) hydriert. Der Katalysator wird abgesaugt, das Filtrat eingedampft und nach Zugabe von 40 ml abs. Dioxan lyophilisiert. Der Rückstand wird in 200 ml doppelt destilliertem Wasser suspendiert, der Niederschlag abgesaugt, die wässrige Phase viermal mit je 50 ml Essigsäureethylester extrahiert und mit wenig Wasser rückextrahiert.

Die vereinigten wässrigen Phasen werden sterilfiltriert (0,45 µm) und lyophilisiert. Man erhält N-Acetyl-muramyl-L-alanyl-D-isoglutaminyl-L-ornithin (α,β-Gemisch) als farbloses Pulver; $[\alpha]_D^{20}$ = +13,9 ± 1,6° (c = 0,624; Methanol), $R_f$ = 0,04 (Chloroform:Methanol:Wasser = 70:30:5), $R_f$ = 0,08 (Essigsäureethylester:Essigsäure:Wasser:Methanol = 67:10:23:12).

Beispiel 12: 4,50 g (8,71 mMol) $N^{\alpha}$-Benzyloxycarbonyl-$N^{\beta}$-{2-[2-(2,6-dichlor-phenyl-amino)-phenyl]-acetyl}-L-α,β-diamino-propionsäure werden in 75 ml Eisessig gelöst und wie üblich während 2½ Stunden hydriert. Der Katalysator wird entfernt, die Lösung am Hochvakuum bei 30° eingeengt und dann lyophilisiert; $R_f$ = 0,39 (n-Butanol:Essigsäure:Wasser = 75:7,5:21). 3,00 g (6,78 mMol) des Rohproduktes, gelöst in 100 ml abs. Dimethylformamid, werden analog Stufe 2.3 mit 8,20 g (8,14 mMol) N-Acetyl-muramyl-L-alanyl-D-isoglutamin-N-hydroxysuccinimidester (ca. 60%ig) umgesetzt. Nach 24stündigem Rühren bei Raumtemperatur wird die Suspension am Hochvaakum bei 30° zur Trockene verdampft und der Rückstand zwischen je 250 ml n-Butanol und Wasser verteilt. Die Oberphase wird noch zweimal mit je 75 ml Wasser ausgezogen und rückextrahiert. Die vereinigten Oberphasen werden stark eingeengt und das Material durch portionenweise Zugabe von 800 ml Diethylether-Petrolether (1:1) in der Kälte ausgefällt. Das Rohprodukt wird an Kieselgel (1:100; 15 ml Fraktionen) im System Chloroform:Methanol:Wasser (70:30:5) gereinigt. Das nach einem Vorlauf von einem Liter in den Fraktionen 391-450 enthaltene Material wird gesammelt, im obigen Lösungsmittelgemisch gelöst und durch vorsichtige Zugabe von verdünnter Natriumhydroxid-Lösung auf pH=7 gestellt. Die Lösung wird durch ein Millipore-Filter (PTFE; 0,2 µm) filtriert, stark eingeengt und nach Zugabe von abs. Dioxan lyophilisiert. Man erhält $N^{\alpha}$-(N-Acetyl-muramyl-L-alanyl-D-isoglutaminyl)-$N^{\beta}$-{2-[2-(2,6-dichlor-phenyl-amino)-phenyl]-acetyl}-L-α,β-diamino-propionsäure-natriumsalz (α,β-Gemisch) als farbloses Pulver, 2,7 Mol Wasser enthaltend (vgl. Beispiel 9);

$[\alpha]_D^{20}$ = +38,3 ± 1,3° (c = 0,752; Methanol), $R_f$ = 0,15 (n-Butanol:Essigsäure:Wasser = 75:7,5:21), $R_f$ = 0,17 (Chloroform:Methanol:Wasser = 70:30:5), $R_f$ = 0,27 (Essigsäureethylester:n-Butanol:Pyridin:Essigsäure:Wasser = 42:21:21:6:10).

Das Ausgangsmaterial erhält man wie folgt:

Stufe 12.1: 4,00 g (11,8 mMol) $N^{\alpha}$-Benzyloxycarbonyl-L-α,β-diamino-propionsäure, 1,19 g (11,8 mMol) Triethylamin und 6,02 g (15,3 mMol) N-Hydroxysuccinimidester von 2-[2-(2,6-Dichlor-phenyl-amino)-phenyl]-essigsäure werden in 100 ml eines Gemisches aus Dimethoxyethan-Acetonitril-Wasser (1:1:1) suspendiert und während 24 Stunden bei Raumtemperatur gerührt. Der nach dem Verdampfen des Lösungsmittels verbleibende Rückstand wird in 100 ml Essigsäureethylester aufgenommen, erst 2mal mit je 50 ml verdünnter Salzsäure, dann 3mal mit je 50 ml kaltgesättigter Natriumhydrogencarbonatlösung extrahiert. Die weitere Reinigung erfolgt durch alternierendes Schütteln: Die Essigesterlösung wird 8mal abwechselnd mit je 50 ml gesättigter Natriumhydrogencarbonatlösung und Wasser extrahiert, wobei die entsprechenden Extrakte getrennt gesammelt werden. Die das Produkt enthaltende wässrige Phase wird angesäuert und das ausgefallene Material mit Essigsäureethylester extrahiert. Die organische Phase wird getrocknet, auf 50 ml eingeengt und durch portionenweise Zugabe von Diethylether zur Kristallisation gebracht. Man erhält $N^{\alpha}$-Benzyloxycarbonyl-$N^{\beta}$-{2-[2-(2,6-dichlor-phenyl-amino)-phenyl]-acetyl}-L-α,β-diamino-propionsäure in Form farbloser Nadeln; Smp. 188,5-190°, $[\alpha]_D^{20}$ = +6,2 ± 1,3° (c = 0,764; Methanol), $R_f$ = 0,39 (Chloroform:Methanol:Wasser = 70:30:5), $R_f$ = 0,61 (Acetonitril:Wasser = 3:1).

Beispiel 13: 0,90 g (1,55 mMol) $N^{\varepsilon}$-{[2-(2,6-Dichlor-phenyl-amino)-phenyl]-acetyl}-L-thialysin-benzylester-hydrobromid und 0,217 g (2,1 mMol) Triethylamin, gelöst in 30 ml Dimethylformamid, werden analog Stufe 2.3 bei Raumtemperatur mit 1,70 g (2,01 mMol) N-Acetyl-muramyl-L-alanyl-D-isoglutamin-N-hydroxysuccinimidester (~ 70%ig) umgesetzt. Nach 4stündigem Rühren wird die Suspension zur Trockene verdampft, der Rückstand in 60 ml ausgekochtem, destilliertem Wasser aufgeschlämmt und nach Rühren im Eisbad filtriert. Der Rückstand wird durch Chromatographie an Kieselgel (1:100; 5 ml Fraktionen) im System Chloroform-Methanol-Wasser (70:30:5) ge-

Stufe 13.2: 1,40 g (2,1 mMol) $N^{\alpha}$-Benzyloxycarbonyl-$N^{\varepsilon}$-2-[(2,6-dichlor-phenyl)-amino]-phenylacetyl-L-thialysinbenzylester werden in 30 ml trockenem Essigsäureethylester gelöst und in der Kälte unter Rühren und Ausschluss von Feuchtigkeit mit 60 ml Bromwasserstoffsäure in Eisessig (33%ig) gelöst. Nach 2stündigem Rühren bei Raumtemperatur wird die bräunliche Reaktionslösung im Eisbad abgekühlt und das Produkt durch Eintropfen von 200 ml Diethylether-Petrolether (1:1) ausgefällt. Der Niederschlag wird nach 1stündigem Rühren bei 0° unter Ausschluss von Feuchtigkeit abfiltriert, gut gewaschen und über Natriumhydroxid auf Träger (Merck) scharf getrocknet. Man erhält $N^{\varepsilon}$-{[2-(2,6-Dichlor-phenylamino)-phenylacetyl-L-thialysinbenzylester-hydrobromid als stark hygroskopisches Pulver, das direkt weiterverarbeitet wird; $R_f$ = 0,15 (Chloroform:Isopropanol:Essigsäure = 70:8:2), $R_f$ = 0,84 (Chloroform:Methanol:Wasser = 70:30:5).

Beispiel 14: Die im Beispiel 13 erhaltenen Mischfraktionen 45-80 werden in 4 ml Dimethylsulfoxid-Dimethylformamid (1:1) gelöst und unter Rühren in der Kälte mit 0,14 ml 30%iger Wasserstoffperoxidlösung versetzt. Nach 2tägigem Stehen bei Raumtemperatur wird die Reaktionslösung im Hochvakuum auf ca. 1 ml eingeengt und nach Zugabe von 10 ml abs. Dioxan lyophilisiert. Der Rückstand wird wie üblich durch Chromatographie an Kieselgel (1:240; 4 ml Fraktionen) im System Chloroform-Methanol-Wasser (70:30:5) gereinigt. Das in den Fraktionen 50-130 enthaltene Material wird gesammelt und analog Beispiel 13 sterilfiltriert. Nach Zugabe von abs. Dioxan und Lyophilisation verbleibt N-(N-Acetyl-muramyl-L-alanyl-D-isoglutaminyl)-S-{2-(2,6-dichlor-phenyl-amino)-phenylacetylamino}-ethyl-L-cysteinbenzylester-sulfoxid (α,β-Gemisch) als farbloses Pulver, 2,73 ml Wasser enthaltend; $[\alpha]_D^{20}$ = +11,5 ± 2,3° (c = 0,436; Dimethylsulfoxid).

Beispiel 15: Zu einer Lösung von 2,79 g (3,2 mMol) ca. 70%iger N-Acetyl-muramyl-L-alanyl-D-isoglutamin-N-hydroxysuccinimidester in 65 ml Dimethylacetamid gibt man bei Raumtemperatur unter Rühren

reinigt. Die Fraktionen 24-44 enthalten reines Material, die Fraktionen 45-80 hingegen zusätzlich noch oxidiertes Material (= Sulfoxid; vgl. Beispiel 14). Die erste Fraktion wird in 80 ml des oben verwendeten Lösungsmittelgemisches durch kurzes Erwärmen gelöst und durch ein Millipore-Filter (PTFE; 0,2 µm) filtriert. Das Filtrat wird am Rotationsverdampfer auf ca. 15 ml eingeengt, 40 ml abs. Dioxan hinzugefügt und lyophilisiert. Man erhält N-(N-Acetyl-muramyl-L-alanyl-D-isoglutaminyl)-S-{2-(2,6-dichlor-phenyl-amino)-phenyl-acetylamino}-ethyl-L-cystein-benzylester (α,β-Gemisch) als farbloses Pulver, 1,85 Mol Wasser enthaltend;
$[\alpha]_D^{20}$ = +16 ± 2° (c = 0,497; Dimethylsulfoxid), $R_f$ = 0,59; Sulfoxid: 0,50 (Chloroform:Methanol:Wasser = 70:30:5), $R_f$ = 0,73; Sulfoxid: 0,63 (Essigsäureethylester:n-Butanol:Pyridin:Essigsäure:Wasser = 42:21:21:6:10).

Das Ausgangsmaterial erhält man folgendermassen:

Stufe 13.1: 1,27 g (3,00 mMol) $N^{\alpha}$-Benzyloxycarbonyl-L-thialysinbenzylester-hydrochlorid und 0,34 g (3,3 mMol) Triethylamin, gelöst in 18 ml abs. Dimethylformamid, werden analog Beispiel 3 mit 1,53 g (3,9 mMol) N-Hydroxysuccinimidester von 2-[2-(2,6-Dichlor-phenyl-amino)-phenyl]-essigsäure umgesetzt. Nach 2stündigem Rühren bei Raumtemperatur wird zur Trockene verdampft, der Rückstand in 200 ml Essigsäureethylester aufgenommen und die Lösung 8mal mit je 40 ml Wasser extrahiert. Der nach dem Trocknen und Verdampfen des Lösungsmittels verbleibende Rückstand kristallisiert beim Stehen in der Kälte (-10°). Die Kristallmasse wird mit 50 ml Methanol aufgeschlämmt, 30 Minuten in der Kälte gerührt, dann abfiltriert und getrocknet. Man erhält $N^{\alpha}$-Benzyloxycarbonyl-$N^{\varepsilon}$-2-[(2,6-dichlor-phenyl)-amino]-phenylacetyl-L-thialysinbenzylester in Form farbloser Kristalle; Smp. 128-129°, $[\alpha]_D^{20}$ = -10 ± 1,4° (c = 0,714; Methanol), $R_f$ = 0,80 (n-Butanol:Essigsäure:Wasser = 75:7,5:21).

1,56 g (3,0 mMol) L-Alanin-3-{2-[1-benzoyl-5-methoxy-2-methyl-indol-3-yl]-acetylamino}-2-hydroxy-propylamid-hydrochlorid, das 0,49 Mol Wasser enthält, und 0,416 ml (3,0 mMol) Triethylamin.

Die so erhaltene gelbe Lösung wird 18 Stunden bei Raumtemperatur gerührt und danach im Hochvakuum bei 30° zur Trockene eingedampft. Den Rückstand nimmt man in 50 ml Methylenchlorid auf, filtriert und versetzt das Filtrat mit 50 ml Diethylether. Die gebildeten Kristalle werden abgesaugt und mit Diethylether nachgewaschen. Das Rohprodukt reinigt man durch Säulenchromatographie an 370 g Kieselgel (Typ 60, Merck; 0,063-0,2 mm) im System Chloroform-Methanol-Wasser (70:30:5, 15 ml Fraktionen).

Die Fraktionen 66-180 werden vereinigt und im Hochvakuum bei 30° eingedampft. Der Rückstand wird zweimal aus Chloroform-Methanol (1:1 40 bzw. 50 ml) mit Diethylether (150 bzw. 100 ml) kristallisiert und die so erhaltenen Kristalle mit Diethylether nachgewaschen. Man erhält N-Acetyl-muramyl-L-alanyl-D-isoglutaminyl-L-alanin-3-{2-[1-benzoyl-5-methoxy-2-methyl-indol-3-yl]-acetylamino}-2-hydroxy-propylamid (als Anomeren- und Diastereomerengemisch) in Form leicht gelb gefärbter Kristalle vom Smp. 204-206° (Zers.). Dieses Produkt wird in 200 ml tert.Butanol-doppeltdestilliertem Wasser (1:1) gelöst und die so erhaltene Lösung durch ein Milliporefilter (Fluoropore, PTFE, 0,2 μm) filtriert und im Hochvakuum lyophilisiert. Das noch tert. Butanol enthaltende Lyophilisat wird nochmals in 100 ml doppelt destilliertem Wasser gelöst, zweimal durch ein Milliporefilter (Nalgene S; 0,45 und 0,2 μm) filtriert und erneut lyophilisiert.

Man erhält N-Acetyl-muramyl-L-alanyl-D-isoglutaminyl-L-alanin-3-{2-[1-benzoyl-5-methoxy-2-methyl-indol-3-yl]-acetylamino}-2-hydroxy-propylamid als farbloses Pulver, das 3,05 Mol Wasser enthält; $[\alpha]_D^{20} = +23,5 \pm 2,0°$ (c = 0,477; Dimethylformamid), $R_f$ = 0,60 (Chloroform:Methanol = 9:1), $R_f$ = 0,58 (Chloroform:Methanol:Wasser = 70:30:5).

0163286

Das Ausgangsmaterial enthält man wie folgt:

Stufe 15.1: Zu einer Lösung von 7,156 g (20 mMol) 1-(p-Chlor-benzoyl)-5-methoxy-2-methyl-3-indolyl-essigsäure in 200 ml absolutem Tetrahydrofuran gibt man bei Raumtemperatur unter Rühren 4,54 g (22 mMol) N,N-Dicyclohexylcarbodiimid und 2,53 g (22 mMol) N-Hydroxy-succinimid. Nach 27 Stunden wird der ausgefallene N,N-Dicyclohexyl-harnstoff abfiltriert und das Filtrat im Hochvakuum bei 30° eingedampft. Der Rückstand wird aus 350 ml Essigsäureethylester-Isopropanol (1:1) umkristallisiert.

Man erhält 1-(p-Chlor-benzoyl)-5-methoxy-2-methyl-3-indolyl-essigsäure-N-hydroxysuccinimidester als farblose Kristalle vom Smp. 199-200°; $R_f$ = 0,85 (Chloroform:Methanol = 9:1).

Stufe 15.2: Zu einer Lösung von 7,6 g (60 mMol) 1,3-Diamino-2-propanol-hydrochlorid (Eastman, pract.) in 200 ml Wasser tropft man bei Raumtemperatur unter Rühren während 45 Minuten eine Lösung von 6,4 g (20 mMol) N-Benzyloxycarbonyl-L-alanin-N-hydroxysuccinimidester in 200 ml Tetrahydrofuran und rührt das Ganze 17 Stunden bei Raumtemperatur. Danach engt man die Lösung auf 50 ml ein, filtriert und dampft das Filtrat im Hochvakuum bei 30° zur Trockne. Den so erhaltenen Rückstand renigt man durch Säulenchromatographie an 2000 g Kieselgel (Typ 60, Merck; 0,063-0,2 mm) im System Chloroform-Methanol (7:3, 20 ml Fraktionen). Die Fraktionen 76-314 werden vereinigt und im Hochvakuum bei 30° eingedampft. Der Rückstand (weisser Schaum) kristallisiert aus Isopropanol-Diethylether (1:7). Man erhält N-Benzyloxycarbonyl-L-alanin-3-amino-2-hydroxy-propylamid-hydrochlorid als farblose Kristalle, die bei 96° sintern und zwischen 114-116° schmelzen; $[\alpha]_D^{20}$ = -18,9 ± 1,0° (c = 0,975; Wasser), $R_f$ = 0,3 (Chloroform:Methanol:Wasser = 70:30:5).

Stufe 15.3.: Zu einer Lösung von 3,32 g (10,0 mMol) N-Benzyloxycarbonyl-L-alanin-3-amino-2-hydroxy-propylamid-hydrochlorid in 165 ml Tetrahydrofuran-Wasser (3:2) gibt man bei Raumtemperatur unter Rühren 1,39 ml (10,0 mMol) Triethylamin und anschliessend während 45 Minuten eine Lösung von 4,40 g (9,67 mMol) 1-(p-Chlorbenzoyl)-5-methoxy-2-methyl-3-indolyl-essigsäure-N-hydroxysuccinimidester in 165 ml Tetrahydrofuran. Die so erhaltene gelbliche Lösung wird 21 Stunden bei Raumtemperatur gerührt und danach im Hochvakuum bei 30° zur Trockne eingedampft. Der feste Rückstand wird in 300 ml Essigsäurethylester aufgenommen und die so erhaltene Lösung zweimal mit je 100 ml 1 N Salzsäure extrahiert. Die Essigesterphase wird abgetrennt, dreimal mit je 100 ml 10%iger Natriumhydrogencarbonatlösung und viermal mit je 100 ml Wasser gewaschen, über Natriumsulfat getrocknet und im Hochvakuum bei 30° eingedampft. Nach Umkristallisieren des Rückstandes aus 100 ml Essigsäureethylester erhält man reines N-Benzyloxycarbonyl-L-alanin-3-{2-[1-p-chlorbenzoyl)-5-methoxy-2-methyl-indol-3-yl]-acetylamino}-2-hydroxypropylamid als leicht gelbliche Kristalle vom Smp. 142-143°, die 0,31 Mol Wasser enthalten;

$[\alpha]_D^{20}$ = +2,4 ± 0,9° (c = 1,058; Dimethylformamid), $R_f$ = 0,29 (Chloroform:Methanol = 9:1), $R_f$ = 0,83 (Chloroform:Methanol:Wasser = 70:30:5), $R_f$ = 0,92 (Chloroform:Methanol = 7:3).

Stufe 15.4.: Eine Lösung von 4,65 g (7,26 mMol) N-Benzyloxycarbonyl-L-alanin-3-{2-[1-(p-chlor-benzoyl)-5-methoxy-2-methyl-indol-3-yl]-acetylamino}-2-hydroxy-propylamid, das 0,31 Mol Wasser enthält, in 150 ml Methanol wird bei Raumtemperatur und Normaldruck 50 Minuten mit 1,0 g 10%iger Palladiumkohle als Katalysator hydriert, wobei der pH-Wert der Lösung von anfangs 7,3 auf 3,5 fällt. Dann wird vom Katalysator abfiltriert und das Filtrat im Hochvakuum bei 30° eingedampft. Der Rückstand wird in 20 ml Wasser gelöst, mit 6,84 ml 1 N Salzsäure versetzt und erneut eingedampft. Der so erhaltene Schaum wird mit 50 ml Diethylether 1 Stunde bei Raumtemperatur verrührt, die gebildeten Kristalle abgesaugt und mit Diethylether nachgewaschen. Man erhält nach Chromatogramm noch schwach verunreinigtes

L-Alanin-3-{2-[1-benzoyl-5-methoxy-2-methyl-indol-3-yl]-acetyl-amino}-2-hydroxy-propylamid-hydrochlorid als farblose Kristalle, die sich bei 160° zersetzen und 0,49 Mol Wasser enthalten. Sie werden ohne weitere Reinigung verarbeitet;
$[\alpha]_D^{20}$ = +11,1 ± 1,0° (c = 1,029; Dimethylformamid), $R_f$ = 0,57 (Chloroform:Methanol:Wasser = 70:30:5), $R_f$ = 0,46 (Chloroform:Methanol = 7:3).

Beispiel 16: Analog Beispiel 15 enthält man aus 2,43 g (2,8 mMol) ca. 70%igem N-Propionyl-desmethylmuramyl-L-alanyl-D-glutaminsäure-($C_\alpha$)-methylester-($C_\gamma$)-N-hydroxysuccinimidester, 1,34 g (2,57 mMol) L-Alanin-3-{2-[1-benzoyl-5-methoxy-2-methyl-indol-3-yl]-acetyl-amino}-2-hydroxy-propylamid-hydrochlorid, das 0,49 Mol Wasser enthält, und 0,357 ml (2,57 mMol) Triethylamin N-Propionyl-desmethyl-muramyl-L-alanyl-D-glutaminsäure-($C_\alpha$)-methylester-($C_\gamma$)-L-alanin-3-{2-[1-benzoyl-5-methoxy-2-methyl-indol-3-yl]-acetylamino}-2-hydroxy-propylamid (Anomeren- und Diastereomerengemisch) als farbloses Pulver, das 2,67 Mol Wasser enthält. Die Substanz kann auch aus Chloroform-Methanol-Diethylether (1:1:10) kristallisiert werden;
Smp. 149-151° (Zers.),
$[\alpha]_D^{20}$ = -1,9 ± 2,3° (c = 0,427; Wasser), $R_f$ = 0,70 (Chloroform:Methanol = 7:3), $R_f$ = 0,69 (Chloroform:Methanol:Wasser = 70:30:5).

Beispiel 17: 2,55 g (3,0 mMol; 1,175 mMol/g) N-Propionyl-desmethyl-muramyl-L-alanyl-D-isoglutamin-N-hydroxysuccinimidester werden in 60 ml Dimethylacetamid suspendiert. Zu dieser Suspension gibt man bei Raumtemperatur unter Rühren 1,127 g (2,71 mMol) L-Alanin-3-{d-2-[6-methoxy-2-naphthyl]-propionylamino}-2-hydroxy-propylamid-hydrochlorid und 0,376 ml (2,71 mMol) Triethylamin. Nach 22 Stunden wird die leicht gelbe Suspension am Hochvakuum bei 30° zur Trockne eingedampft. Der Rückstand wird in 40 ml Methylenchlorid aufgenommen, filtriert und die so erhaltene klare Lösung mit 40 ml Diethylether versetzt. Die gebildeten farblosen Kristalle werden abgesaugt und mit Diethylether nachgewaschen. Das Rohprodukt reinigt man durch

Säulenchromatographie an 350 g Kieselgel (Typ 60, Merck; 0,063-0,2 mm) im System Chloroform-Methanol-Wasser (70:30:5); 10 ml Fraktionen).

Die Fraktionen 64-90 werden vereinigt und im Hochvakuum bei 30° eingedampft. Der Rückstand kristallisiert aus 40 ml Chloroform-Methanol (1:1) nach Zugabe von 200 ml Diethylether. Die Kristalle werden abgenutscht und mit Diethylether nachgewaschen, dann in 40 ml Chloroform-Methanol (1:1) gelöst und die so erhaltene Lösung durch ein Milliporefilter (Fluoropore, PTFE; 0,2 µm) filtriert. Das klare Filtrat wird mit 200 ml durch ein Milliporefilter (Fluoropore, PTFE; 0,2 µm) filtriertem Diethylether versetzt. Die ausgefallenen Kristalle werden abgenutscht und mit durch ein Milliporefilter filtriertem Diethylether gewaschen. Man erhält reines N-Propionyl-desmethylmuramyl-L-alanyl-D-isoglutaminyl-L-alanin-3-{d-2-[6-methoxy-2-naphthyl]-propionylamino}-2-hydroxy-propylamid (Anomeren- und Diastereomerengemisch) als farblose Kristalle vom Smp. 182-184° (Zers.), die 0,38 Mol Wasser enthalten;
$[\alpha]_D^{20}$ = +8,1 ± 0,9° (c = 1,105; Dimethylformamid), $R_f$ = 0,27 (n-Butanol:Essigsäure:Wasser = 75:7,5:21), $R_f$ = 0,49 (Chloroform:Methanol = 7:3), $R_f$ = 0,53 (Chloroform:Methanol:Wasser = 70:30:5).

Das Ausgangsmaterial erhält man folgendermassen:

<u>Stufe 17.1:</u> Analog Stufe 15.1 erhält man aus 5,75 g (25,0 mMol) d-2-(6-Methoxy-2-naphthyl)-propionsäure, 5,67 g (27,5 mMol) N,N-Dicyclohexylcarbodiimid und 3,16 g (27,5 mMol) N-Hydroxysuccinimid in 250 ml Tetrahydrofuran d-2-(6-Methoxy-2-naphthyl)-propionsäure-N-hydroxysuccinimidester als farblose Kristalle vom Smp. 128-129° (aus Isopropanol); $[\alpha]_D^{20}$= +70,0 ± 1,0° (c = 1,017; Chloroform), $R_f$ = 0,54 (Chloroform:Methanol = 95:5), $R_f$ = 0,88 (Chloroform:Methanol = 9:1), $R_f$ = 0,92 (Chloroform:Methanol = 7:3).

Stufe 17.2: Analog Stufe 15.3 erhält man aus 3,32 g (10,0 mMol) N-Benzyloxycarbonyl-L-alanin-3-amino-2-hydroxy-propylamid-hydrochlorid und 3,27 g (10,0 mMol) d-2-(6-Methoxy-2-naphthyl)-propionsäure-N-hydroxysuccinimidester N-Benzyloxycarbonyl-L-alanin-3-{d-2-[6-methoxy-2-naphthyl]-propionylamino}-2-hydroxy-propylamid als farblose Kristalle vom Smp. 183-184° (aus Essigsäureethylester:Isopropanol = 1:1); $[\alpha]_D^{20}$ = +8,3 ± 1,0° (c = 0,963; Methanol), $R_f$ = 0,63 (Chloroform:Ethanol = 9:1), $R_f$ = 0,73 (Chloroform:Methanol = 9:1), $R_f$ = 0,90 (Chloroform:Methanol = 4:1).

Stufe 17.3: Analog Stufe 15.4 erhält man aus 3,2 g (6,3 mMol) N-Benzyloxycarbonyl-L-alanin-3-{d-2-[6-methoxy-2-naphthyl]-propionylamino}-2-hydroxy-propylamid durch katalytische Hydrierung [1 g Palladiumkohle (10 % Palladium), 10 Minuten, Raumtemperatur, Normaldruck] in Methanol L-Alanin-3-{d-2-[6-methoxy-2-naphthyl]-propionylamino}-2-hydroxy-propylamid-hydrochlorid als farblose Kristalle, die sich ab 102° zersetzen und 0,29 Mol Wasser enthalten; $[\alpha]_D^{20}$ = +32,3 ± 0,9° (c = 1,098; Methanol), $R_f$ = 0,31 (Chloroform:Methanol = 4:1), $R_f$ = 0,33 (Chloroform:Methanol = 7:3), $R_f$ = 0,58 (Chloroform:Methanol:Wasser = 70:30:5).

Beispiel 18: Analog Beispiel 17 erhält man aus 2,34 g (2,75 mMol; 1,175 mMol/g) N-Propionyl-desmethylmuramyl-L-alanyl-D-isoglutamin-N-hydroxysuccinimidester, 0,965 g (2,45 mMol) L-Alanin-3-[2-(4-isobutyl-phenyl)-propionylamino]-2-hydroxy-propylamid-hydrochlorid, das 0,43 Mol Wasser enthält, und 0,340 ml (2,45 mMol) Triethylamin in 55 ml Dimethylacetamid N-Propionyl-desmethylmuramyl-L-alanyl-D-isoglutaminyl-L-alanin-3-[2-(4-isobutyl-phenyl)-propionylamino]-2-hydroxy-propylamid (Anomeren- und Diastereomerengemisch) als farblose Kristalle vom Smp. 181-183° (Zers.), die 0,43 Mol Wasser enthalten; $[\alpha]_D^{20}$ = +13,0 ± 1,0° (c = 1,013; Dimethylformamid), $R_f$ = 0,31 (n-Butanol:Essigsäure:Wasser = 75:7,5:21, $R_f$ = 0,51 (Chloroform:Methanol:Wasser = 70:30:5), $R_f$ = 0,53 (Chloroform:Methanol = 7:3).

Das Ausgangsmaterial erhält man wie folgt:

Stufe 18.1: Analog Stufe 15.1 erhält man aus 5,15 g (25,0 mMol) 2-(4-Isobutyl-phenyl)-propionsäure, 5,67 g (27,5 mMol) N,N-Dicyclohexylcarbodiimid und 3,16 g (27,5 mMol) N-Hydroxysuccinimid in 250 ml Tetrahydrofuran 2-(4-Isobutyl-phenyl)-propionsäure-N-hydroxysuccinimidester als farblose Kristalle vom Smp. 96-97° (aus Isopropanol); $R_f$ = 0,45 (Chloroform:Essigsäureethylester = 99:1), $R_f$ = 0,85 (Chloroform:Methanol = 9:1).

Stufe 18.2: Analog Stufe 15.3 erhält man aus 3,32 g (10,0 mMol) N-Benzyloxycarbonyl-L-alanin-3-amino-2-hydroxy-propylamid-hydrochlorid und 3,04 g (10,0 mMol) 2-(4-Isobutyl-phenyl)-propionsäure-N-hydroxysuccinimidester N-Benzyloxycarbonyl-L-alanin-3-[2-(4-isobutyl-phenyl)-propionylamino]-2-hydroxy-propylamid als farblose Kristalle vom Smp. 127-128°; $[\alpha]_D^{20}$ = -9,7 ± 0,8° (c = 1,218; Methanol), $R_f$ = 0,50 (Chloroform:Ethanol = 9:1), $R_f$ = 0,58 (Chloroform:Methanol = 9:1), $R_f$ = 0,87 (Chloroform:Methanol = 4:1), $R_f$ = 0,92 (Chloroform:Methanol:Wasser = 70:30:5).

Stufe 18.3: Analog Stufe 15.4 erhält man aus 3,24 g (6,7 mMol) N-Benzyloxycarbonyl-L-alanin-3-[2-(4-isobutyl-phenyl)-propionylamino]-2-hydroxy-propylamid durch katalytische Hydrierung [1 g Palladiumkohle (10 % Palladium), 15 Minuten, Raumtemperatur, Normaldruck] in Methanol bei pH 7 (Titration mit 1 N Salzsäure) L-Alanin-3-[2-(4-isobutyl-phenyl)-propionylamino]-2-hydroxy-propylamid-hydrochlorid als farblose Kristalle, die sich ab 88° zersetzen (mit Diethylether verrieben) und 0,43 Mol Wasser enthalten; $[\alpha]_D^{20}$ = +8,3 ± 0,9° (c = 1,065; Methanol), $R_f$ = 0,30 (Chloroform:Methanol = 4:1), $R_f$ = 0,58 (Chloroform:Methanol:Wasser = 70:30:5).

Beispiel 19: 500 mg, 0,53 mMol, N-Propionyl-desmethylmuramyl-L-alanyl-D-isoglutaminyl-L-alanin-3-{2-[2-(2,6-dichlor-phenyl-amino)-phenyl]-acetylamino}-2-hydroxy-propylamid (Beispiel 6) löst man in 10 ml Pyridin, versetzt mit 0,5 ml Acetanhydrid und 5 mg 4-Dimethylaminopyridin. Nach 18 Stunden bei Raumtemperatur versetzt man mit 5 ml MeOH und dampft im Vakuum zur Trockne. Den Rückstand extrahiert man zweimal mit je 15 ml Essigsäureethylester, löst den Rückstand in 20 ml Ethanol bei 40°, kühlt auf Raumtemperatur und gibt 20 ml Diethylether zu. Man erhält so farblose Kristalle von 1,4,6-Tri-O-acetyl-N-propionyl-desmethylmuramyl-L-alanyl-D-isoglutaminyl-L-alanin-3-{2-[2-(2,6-dichlor-phenyl-amino)-phenyl]-acetylamino}-2-hydroxy-propylamid vom Smp. 189-190°; $[\alpha]_D^{20}$ = +1,8° ± 1° (c = 0,987; $CHCl_3$:MeOH = 1:1), $R_f$ = 0,71 ($CHCl_3$:MeOH = 8:2).

Beispiel 20: Analog Beispiel 3 erhält man aus 1,75 g (2,56 mMol) N-Acetyl-muramyl-L-alanyl-D-isoglutaminyl-D,L-oxalysin, 1,01 g (3,07 mMol) d-2-(6-Methoxy-2-naphthyl)-propionsäure-N-hydroxy-succinimidester und 0,26 g (2,56 mMol) Triethylamin, gelöst in 30 ml Dimethylformamid-Wasser (95:5), und anschliessender Chromatographie an Kieselgel (1:60; 10 ml Fraktionen) im System Chloroform-Methanol-Wasser (70:30:5) N-(N-Acetyl-muramyl-L-alanyl-D-isoglutaminyl-O-[2-(6-methoxy-naphth-2-yl)-propionyl]-DL-serin (α,β-Gemisch) als farbloses Pulver, 2,7 Mol Wasser enthaltend; $[\alpha]_D^{20}$ = +5,3 ± 1,8° (c = 0,562; Wasser), $R_f$ = 0,07 (Chloro-form:Methanol:Wasser = 70:30:5), $R_f$ = 0,19 (Essigsäureethylester:n-Butanol:Pyridin:Essigsäure:Wasser = 42:21:21:6:10), $R_f$ = 0,45 (Acetonitril:Wasser = 3:1).

Das Ausgangsmaterial erhält man wie folgt:

Stufe 20.1: 1,05 g (3,71 mMol) $N^{\varepsilon}$-Benzyloxycarbonyl-DL-oxalysin, 1,50 ml (14,43 mMol) Benzylalkohol und 1,27 g (6,68 mMol) p-Toluolsulfonsäure werden in 50 ml Benzol gelöst und während 4 Stunden bei 85° durch azeotrope Entfernung des Wassers verestert. Die Reaktionslösung wird auf ca. 5 ml eingeengt, mit 100 ml n-Butanol-Essigsäureethylester (1:9) verdünnt, und die Lösung 6mal mit je 20 ml Wasser extrahiert. Die wässrige Phase wird rückextrahiert. Die vereinigten organischen Phasen werden auf etwa 5 ml eingeengt und in der Kälte durch Zugabe von 120 ml Essigsäureethylester zur Kristallisation gebracht. Der Niederschlag wird abfiltriert. Nach dem Trocknen verbleibt $N^{\varepsilon}$-Benzyloxycarbonyl-DL-oxalysinbenzylester-p-toluolsulfonat in Form farbloser Kristalle; Smp. 126-127°, $R_f$ = 0,49 (n-Butanol:Essigsäure:Wasser = 75:7,5:21), $R_f$ = 0,70 (Essigsäureethylester:n-Butanol:Pyridin:Essigsäure:Wasser = 42:21:21:6:10).

Stufe 20.2: 1,05 g (2,92 mMol) $N^{\varepsilon}$-Benzyloxycarbonyl-DL-oxalysin-benzylester-p-toluolsulfonat, 3,27 g (3,76 mMol) N-Acetyl-muramyl-L-alanyl-D-isoglutamin-N-hydroxysuccinimidester (etwa 70%ig) und 2,93 g (2,92 mMol) Triethylamin, gelöst in 30 ml Dimethylformamid, werden analog Stufe 2.3 während 6 Stunden bei Raumtemperatur umgesetzt. Die Suspension wird mit 300 ml Essigsäureethylester verdünnt und nach 1stündigem Rühren bei 0° das Ungelöste abfiltriert. Der Niederschlag wird in der Kälte 6mal mit je 40 ml kaltem Wasser aufgeschlämmt, das Ungelöste abfiltriert und die wässrige Lösung 6mal mit je 20 ml mit Wasser gesättigtem n-Butanol extrahiert; $R_f$ = 0,55 (Chloroform:Isopropanol:Essigsäure = 70:8:2). Rückstand und Butanol-Extrakt werden in 80 ml Eisessig gelöst und nach Zugabe von 0,45 g Pd/Kohle (10%ig) hydriert. Der Katalysator wird abfiltriert, das Filtrat im Vakuum bei 30° eingeengt und lyophilisiert. Das erhaltene Material wird in 60 ml destilliertem Wasser in der Kälte aufgeschlämmt und das Ungelöste abfiltriert. Das Filtrat wird mehrfach und jeweils 10 ml Essigsäureethylester ausgezogen, auf das halbe Volumen eingeengt, durch ein Millipore-Filter (0,45 µm) filtriert und lyophilisiert. Man erhält N-Acetyl-muramyl-L-alanyl-D-iso-glutaminyl-DL-oxalysin (α,β-Gemisch) als farbloses Pulver, 1 Mol Wasser enthaltend; $[\alpha]_D^{20}$ = +20,5 ± 2,6° (c = 0,385; Wasser), $R_f$ = 0,08 (Acetonitril:Wasser = 3:1).

0163286

Beispiel 21: Analog Beispiel 19 erhält man durch Acetylierung von 0,52 g (0,608 mMol) N-Propionyl-desmethylmuramyl-L-alanyl-D-iso-glutaminyl-L-alanin-3-{d-2-[6-methoxy-2-naphthyl]-propionylamino}-2-hydroxy-propylamid (siehe Beispiel 17), das 0,38 Mol Wasser enthält, mit 345 µl (3,65 mMol) Essigsäureanhydrid in 6 ml absolutem Pyridin unter Zusatz katalytischer Mengen von 4-Dimethylamino-pyridin (20 Stunden, Raumtemperatur) (1α,β),4,6-Tri-O-acetyl-N-propionyl-desmethylmuramyl-L-alanyl-D-isoglutaminyl-L-alanin-3-[d-2-(6-methoxy-2-naphthyl]-propionylamino]-2-acetyloxy-propylamid (Anomeren- und Diastereomerengemisch) als farbloses Pulver (Lyophilisat), das 2,38 Mol Wasser enthält. Aus Chloroform-Methanol-Diethylether erhält man farblose Kristalle; Smp. 207-208° (Zers.), $[\alpha]_D^{20}$ = +22,6 ± 2,1° (c = 0,469; Dimethylformamid), $R_f$ = 0,15 (Chloroform:Methanol = 9:1), $R_f$ = 0,58 (Chloroform:Methanol = 4:1), $R_f$ = 0,74 (Chloroform:Methanol:Wasser = 70:30:5).

Beispiel 22: Analog Beispiel 17 erhält man aus 3,0 g (3,52 mMol; 1,175 mMol/g) N-Propionyl-desmethylmuramyl-L-alanyl-D-isoglutamin-N-hydroxysuccinimidester und 1,40 g (3,42 mMol) L-Alanin-3-[2-(3-hydroxybenzyl-phenyl)-propionylamino]-2-hydroxy-propylamid, das 0,45 Mol Wasser enthält, in 70 ml Dimethylacetamid N-Propionyl-des-methylmuramyl-L-alanyl-D-isoglutaminyl-L-alanin-3-[2-(3-hydroxybenzyl-phenyl)-propionylamino]-2-hydroxy-propylamid (Anomeren- und Diastereomerengemisch) als farbloses Pulver (Lyophilisat), das 2,71 Mol Wasser enthält. Die Substanz kristallisiert aus Chloroform-Methanol-Diethylether (10:1:20) in Form farbloser Kristalle, die sich ab 147° zersetzen; $[\alpha]_D^{20}$ = -9,6 ± 0,9° (c = 1,121; Wasser), $R_f$ = 0,37 (Chloroform:Methanol = 7:3), $R_f$ = 0,48 (Chloroform:Methanol:Wasser = 70:30:5).

Das Ausgangsmaterial erhält man wie folgt:

Stufe 22.1: Analog Stufe 15.1 erhält man aus 5,06 (20,0 mMol) 2-(3-Benzoyl-phenyl)-propionsäure, 4,54 g (22,0 mMol) N,N-Dicyclohexylcarbodiimid und 2,53 g (22,0 mMol) N-Hydroxysuccinimid in

250 ml Tetrahydrofuran 2-(3-Benzoyl-phenyl)-propionsäure-N-hydroxy-succinimidester als farblose Kristalle vom Smp. 100-101° (aus Ethanol); $R_f$ = 0,55 (Chloroform:Essigsäureethylester = 95:5).

Stufe 22.2: Analog Stufe 15.3 erhält man aus 3,32 g (10,0 mMol) N-Benzyloxycarbonyl-L-alanin-3-amino-2-hydroxy-propylamid-hydrochlorid und 3,51 g (10,0 mMol) 2-(3-Benzoyl-phenyl)-propionsäure-N-hydroxysuccinimidester N-Benzyloxycarbonyl-L-alanin-3-[2-(3-benzoyl-phenyl)-propionylamino]-2-hydroxy-propylamid als farblosen Schaum, der 0,22 Mol Wasser enthält; $[\alpha]_D^{20}$ = -7,9 ± 0,8° (c = 1,195; Methanol), $R_f$ = 0,45 (Chloro-form:Methanol = 9:1), $R_f$ = 0,94 (Chloroform:Methanol = 4:1).

Stufe 22.3: Analog Stufe 15.4 erhält man aus 4,0 g (7,47 mMol) N-Benzyloxycarbonyl-L-alanin-3-[2-(3-benzoyl-phenyl)-propylamino]-2-hydroxy-propylamid, das 0,22 Mol Wasser enthält, durch katalytische Hydrierung [1 g Palladiumkohle (10 % Palladium), 40 Minuten, Raumtemperatur, Normaldruck] in Methanol nach Säulenchromatographie der freien Base (freigesetzt mit 1 N Natronlauge) an 400 g Kieselgel (Typ 60, Merck; 0,063-0,2 mm; System Chloroform-Methanol-Wasser [70:30:5]) L-Alanin-3-[2-(3-hydroxybenzyl-phenyl)-propionylamino]-2-hydroxy-propylamid als farblose Kristalle vom Smp. 135-136° (Zers., aus Isopropanol:Diethylether = 15:70), die 0,45 Mol Wasser enthalten; $[\alpha]_D^{20}$= +5,9 ± 1,0° (c = 1,018; Methanol), $R_f$ = 0,31 (Chloroform:Methanol:Wasser = 70:30:5), $R_f$ = 0,39 (n-Butanol:Essigsäure:Wasser = 75:7,5:21).

Beispiel 23: 0,5 g (0,5 mMol) 2-Phenyl-4,5-[3-O-{vinyl-1-carbonyl-L-alanyl-D-isoglutaminyl-L-alanyl-4-(2-[2-(2,6-dichlor-phenyl-amino)-phenyl]-acetylamino)-butylamido}-5,6-O-isopropyliden-D-glucofurano]-$\Delta^2$-oxazolin löst man in 15 ml 50%iger Essigsäure und hydrolysiert 6 Stunden bei 50°. Man dampft im Vakuum zu einem Harz ein. Dieses extrahiert man dreimal mit je 20 ml Diethylether, löst den Rückstand in 15 ml Chloroform-Methanol (1:9) bei 30° und gibt

dazu 40 ml Diethylether. Man erhält N-2-Benzoylamino-2-desoxy-D-glucos-3-O-yl-vinyl-1-carbonyl-L-alanyl-D-isoglutaminyl-L-alanin-4-{2-[2-(2,6-dichlor-phenylamino)-phenyl]-acetylamino}-butylamid als farblose, mikrokristalline Verbindung, die bei 155-160° schmilzt;
$[\alpha]_D^{20}$ = +11,5° ± 1 ° (c = 0,97; DMSO),
$R_f$ = 0,30 (Chloroform:Methanol:Wasser = 85:15:0,5).

Das Ausgangsmaterial erhält man wie folgt:
Man löst 0,64 g (1,568 mMol) 2-Phenyl-4,5-[3,0-(1-ethoxycarbonyl-vinyl)-5,6-O-isopropyliden-D-glucofurano]-$\Delta^2$-oxazolin in 10 ml Ethanol, 10 ml Dimethoxyethan und 0,872 ml 2 N Natronlauge. Nach 5 Stunden bei Raumtemperatur ist der Ester verseift, man dampft im Vakuum ein, was man viermal mit je 20 ml Toluol wiederholt. Der Rückstand wird 1 Stunde bei 2,7 Pa (0,02 Torr) und 45° getrocknet, in 15 ml absolutem N,N-Dimethylacetamid gelöst und nacheinander mit 0,46 g (2,22 mMol) N,N'-Dicyclohexylcarbodiimid, 0,3 g (2,22 mMol) N-Hydroxy-benztriazol und bei 0° mit 0,133 ml (1,745 mMol) Trifluoressigsäure versetzt. Nach 30 Minuten bei Raumtemperatur gibt man 1,29 g (1,745 mMol) Trifluoracetat von L-Alanyl-D-isoglutaminyl-L-alanin-4-{2-[2-(2,6-dichlor-phenyl-amino)-phenyl]-acetylamino}-butylamid und 0,243 ml Triethylamin zu. Diese Mischung wird nach 48 Stunden bei Raumtemperatur im Vakuum zur Trockne gedampft. Man nimmt den Rückstand in 60 ml Essigsäureethylester auf, extrahiert die organische Phase dreimal mit je 50 ml 5%iger $NaHCO_3$-Lösung sowie zweimal mit je 50 ml Wasser und extrahiert die wässrigen Phasen zweimal mit je 50 ml Essigsäureethylester. Nach Trocknen der vereinigten organischen Phasen mit $Na_2SO_4$ und Eindampfen im Vakuum erhält man ein Rohprodukt. Dieses extrahiert man dreimal mit je 30 ml Hexan bei 30°. Das zurückbleibende Harz chromatographiert man an 100 g Kieselgel (Merck, 0,04-0,63 mm) mit Methylenchlorid-Methanol (95:5). Die die gesuchte Verbindung mit $R_f$ = 0,62 ($CHCl_3$:MeOH = 85:15) enthaltenden Fraktionen dampft man ein und kristallisiert um durch Versetzen einer 10%igen Lösung in Acetonitril-Methanol (1:1) mit dem gleichen Volumenteil Diethylether. Man erhält farblose Kristalle von 2-Phenyl-4,5-[3-O-{vinyl-1-carbonyl-L-alanyl-D-iso-

glutaminyl-L-alanyl-4-(2-[2-(2,6-dichlor-phenyl-amino)-phenyl]-acetylamino)-butylamido}-5,6-O-isopropyliden-D-glucofurano]-$\Delta^2$-oxazolin vom Smp. 165-170° (Zers.),
$[\alpha]_D^{20} = -15,1° \pm 1°$ (c = 0,962, $CHCl_3$:MeOH = 1:1).

Das peptidische Ausgangsmaterial erhält man in bekannter Weise durch Spaltung des tert. Butoxycarbonyl-Derivats und Trifluoressigsäure. Das tert.-Butoxycarbonyl-L-alanyl-D-isoglutaminyl-L-alanin-4-{2-[2-(2,6-dichlor-phenylamino)-phenyl]-acetylamino}-butylamid, erhalten auf konventionelle Art, ist eine farblose Verbindung mit dem Smp. 208-212° (Zers.); $R_f$ = 0,45 ($CHCl_3$:Methanol = 9:1).

Das 2-Phenyl-4,5-[3,O-(1-ethoxycarbonyl-vinyl)-5,6-O-isopropyliden-D-glucofurano]-$\Delta^2$-oxazolin erhält man durch Wittig-Horner-Reaktion von Paraformaldehyd mit 2-Phenyl-4,5-[3,O-(carbethoxy-diethyl-phosphonyl-methyl)-5,6-O-isopropyliden-D-glucofurano]-$\Delta^2$-oxazolin und Diazabicycloundecen in Dimethoxyethan.

500 mg des obengenannten Phosphonesters 2-Phenyl-4,5-[3,O-(carbethoxy-diethylphosphonyl-methyl)-5,6-O-isopropyliden-D-glucofurano]-$\Delta^2$-oxazolin löst man in 5 ml absol. Dimethoxyethan und gibt dazu 85 mg Paraformaldehyd und 170 µl Diazabicycloundecen (DBU). Man erhält eine rote Suspension, der man nach 3 Stunden bei Raumtemperatur weitere 40 mg Paraformaldehyd und 40 µl DBU zufügt. Dann dampft man im Vakuum zur Trockne und filtriert den Rückstand über 20 g Kieselgel (Merck, 0,04-0,63 mm) mit Chloroform. Die das Produkt mit $R_f$ = 0,37 ($CHCl_3$: Essigsäureethylester = 95:5) enthaltenden Fraktionen dampft man ein und kristallisiert den Rückstand aus Ethanol um. Man erhält farblose Kristalle von 2-Phenyl-4,5-[3,O-(1-ethoxycarbonyl-vinyl)-5,6-O-isopropyliden-D-glucofurano]-$\Delta^2$-oxazolin vom Smp. 119-120°; $[\alpha]_D^{20} = -17,5° \pm 1°$ (c = 0,892; $CHCl_3$).

Den als Ausgangsmaterial dienenden Phosphonester erhält man wie folgt:

Zu 20 g 2-Phenyl-4,5-[5,6-O-isopropyliden-D-glucofurano]-$\Delta^2$-oxazolin in 150 ml Toluol gibt man 34,5 g Diazo-phosphonessigsäure-triethylester und 0,36 g Rhodiumdiacetat. Man erhitzt die Mischung 18 Stunden unter Stickstoff auf 75-80°, gibt nochmals 11,5 g Diazoester zu und erhitzt weitere 24 Stunden. Dann ist der Zucker vollständig umgesetzt. Man dampft die Lösung im Vakuum ein, nimmt den Rückstand in 250 ml Diethylether auf und schüttelt dreimal mit je 200 ml Wasser aus. Die wässerigen Extrakte werden anschliessend zweimal mit je 200 ml Diethylether ausgeschüttelt. Nach Trocknen der vereinigten Etherlösungen über $MgSO_4$ dampft man die organische Phase zu einem gelbbraunen Oel ein. Dieses Rohprodukt chromatographiert man an 800 g Kieselgel (Merck, 0,04-0,63 mm) mit Methylenchlorid:Aceton = 95:5. Man erhält 2-Phenyl-4,5-[3,O-(carbethoxy-diethylphosphonyl-methyl)-5,6-O-isopropyliden-D-glucofurano]-$\Delta^2$-oxazolin als Diastereomerenemisch in Form eines gelben Oels mit $R_f$ = 0,39 und 0,50 (Tertiärbutyl-methylether), $[\alpha]_D^{20}$ = +21° ± 2,4° (c = 0,424; $CHCl_3$).

Beispiel 24: Analog Beispiel 17 erhält man aus 1,19 g (1,4 mMol; 1,175 mMol/g) N-Propionyl-desmethylmuramyl-L-alanyl-D-isoglutamin-N-hydroxysuccinimidester, 0,72 g (1,4 mMol) L-Alanin-3-[2-(3-benzoyl-phenyl)propionylmino]-2-hydroxy-propylamid-trifluoracetat, das 0,25 Mol Wasser enthält, und 195 µl (1,4 mMol) Triethylamin in 28 ml Dimethylacetamid N-Propionyl-desmethylmuramyl-L-alanyl-D-iso-glutaminyl-L-alanin-3-[2-(3-benzoyl-phenyl)-propionylmino]-2-hydroxy-propylmid als farbloses Pulver, das 2,98 Mol Wasser enthält; $[\alpha]_D^{20}$ = -11,5 ± 2,0° (c = 0,494; Wasser), $R_f$ = 0,32 (Chloroform:Methanol = 7:3), $R_f$ = 0,39 (Chloroform:Methanol:Wasser = 70:30:5).

Das Ausgangsmaterial erhält man wie folgt:

Stufe 24.1: Analog Stufe 15.2 erhält man aus 26,53 g (209 mMol) 1,3-Diamino-2-propanol-hydrochlorid (Eastman, pract.) und 20,0 g (69,8 mMol) N-tert.-Butyloxycarbonyl-L-alanin-N-hydroxysuccinimidester nach Säulenchromatographie an 1000 g Kieselgel (Typ 60, Merck; 0,063-0,2 mm), im System Chloroform-Methanol-Wasser (70:30:5) N-tert.-Butyloxycarbonyl-L-alanin-3-amino-2-hydroxy-propylamid-hydrochlorid, das 0,56 Mol Wasser enthält;
$[\alpha]_D^{20}$ = -13,2 ± 1,1° (c = 0,945; Methanol), $R_f$ = 0,26 (Chloroform:Methanol:Wasser = 70:30:5).

Stufe 24.2: Zu einer Lösung von 1,5 g (4,87 mMol) N-tert.-Butyloxycarbonyl-L-alanin-3-amino-2-hydroxy-propylamid-hydrochlorid, das 0,56 Mol Wasser enthält, in 90 ml Tetrahydrofuran-Wasser (3:2) gibt man bei Raumtemperatur unter Rühren 0,68 ml (4,87 mMol) Triethylamin (Dichte = 0,728) und anschliessend innerhalb von 45 Minuten eine Lösung von 1,72 g (4,87 mMol) 2-(3-Benzoyl-phenyl)-propionsäure-N-hydroxysuccinimidester in 90 ml Tetrahydrofuran. Nach 24 Stunden Rühren bei Raumtemperatur wird die Lösung auf 30 ml eingeengt und die wässrige Phase zweimal mit je 200 ml Essigsäureethylester extrahiert. Die Essigesterphasen werden je einmal mit 50 ml 0,1 N Salzsäure und 100 ml 10%iger Natriumhydrogencarbonatlösung, sowie zweimal mit je 100 ml Wasser gewaschen, über Natriumsulfat getrocknet und im Hochvakuum bei 30° zur Trockne eingedampft.

Man erhält 2,34 g eines farblosen Schaumes, der in 50 ml heissem Cyclohexan gelöst wird. Nach Abkühlen der Lösung scheidet sich das Rohprodukt als farbloses, dickflüssiges Oel ab. Das Lösungsmittel wird abdekantiert. Nach erneutem Abdekantieren mit 100 ml Pentan wird der Rückstand im Hochvakuum bei 30° getrocknet.

Man erhält 2,1 g Rohprodukt als farblosen Schaum, der durch Säulenchromatographie an 200 g Kieselgel (Typ 60, Merck; 0,063-0,2 mm) im System Chloroform-Methanol (9:1) gereinigt wird (10 ml Fraktionen).

Die Fraktionen 33-50 werden vereinigt und im Hochvakuum bei 30° eingedampft. Man erhält 2,05 g eines farblosen Schaumes, der nochmals durch Säulenchromatographie gereinigt wird (200 g Kieselgel, Typ 60, Merck, 0,063-0,2 mm, Chloroform-Methanol [9:1], 10 ml Fraktionen).

Die Fraktionen 36-70 werden wiederum vereinigt und im Hochvakuum bei 30° eingedampft.

Man erhält 2,0 g eines farblosen Schaumes, der in 50 ml heissem Diethylether aufgeschlämmt wird. Die so erhaltene Suspension wird nach Abkühlung mit 100 ml Pentan versetzt und 2 Stunden bei Raumtemperatur gerührt. Die so erhaltenen farblosen Kristalle werden abgesaugt und mit Pentan gewaschen. Man erhält N-tert.-Butyloxycarbonyl-L-alanin-3-[2-(3-benzoyl-phenyl)-propionylamino]-2-hydroxy-propylamid als farblose Kristalle vom Smp. 76-78° (Zers.), die 0,21 Mol Wasser enthalten; $[\alpha]_D^{20} = -6,6 \pm 1,1°$ (c = 0,870; Methylenchlorid), $R_f = 0,31$ (Chloroform:Methanol = 9:1), $R_f = 0,60$ (Chloroform:Methanol = 4:1), $R_f = 0,70$ (Chloroform:Methanol = 7:3).

Stufe 24.3:

Eine Lösung von 1,59 g (3,17 mMol) N-tert.-Butyloxycarbonyl-L-alanin-3-[2-(3-benzoyl-phenyl)-propionylamino]-2-hydroxy-propylamid, das 0,21 Mol Wasser enthält, in 25 ml absolutem Methylenchlorid wird bei 0° unter Rühren mit 6,4 ml (83,6 mMol) Trifluoressigsäure (Dichte = 1,49) versetzt und die so erhaltene Lösung 3 Stunden bei 0° gerührt. Danach wird die klare, farblose Lösung mit 60 ml Diethylether versetzt und die so erhaltene Suspension 15 Minuten bei 0° gerührt. Die gebildeten, farblosen Kristalle werden abgenutscht und mit Diethylether gewaschen. Man erhält 1,45 g Rohprodukt, das nochmals in 50 ml heissem Diethylether aufgeschlemmt wird. Nach Abkühlung rührt man die Suspension noch 1 Stunde bei Raumtemperatur, saugt die erhaltenen Kristalle ab und wäscht sie mit

Diethylether. Man erhält L-Alanin-3-[2-(3-benzoyl-phenyl)-propionylamino]-2-hydroxy-propylamid-trifluoracetat als farblose Kristalle, die sich ab 92° zersetzen und 0,25 Mol Wasser enthalten;
$[\alpha]_D^{20}$ = +10,9 ± 0,9° (c = 1,054; Methylenchlorid),
$R_f$ = 0,12 (Chloroform:Methanol = 4:1), $R_f$ = 0,17 (Chloroform:Methanol = 7:3), $R_f$ = 0,23 (Chloroform:Methanol:Wasser = 70:30:5).

Beispiel 25: Analog Beispiel 17 erhält man aus 1,7 g (2,0 mMol; 1,175 mMol/g) N-Propionyl-desmethylmuramyl-L-alanyl-D-isoglutamin-N-hydroxysuccinimidester, 0,70 g (1,61 mMol) L-Alanin-3-[2-(3-chlor-4-{pyrrol-1-yl}-phenyl)-propionylamino]-2-hydroxy-propylamid-hydrochlorid, das 0,35 Mol Wasser enthält, und 223,7 µl (1,61 mMol) Triethylamin in 45 ml Dimethylacetamid unter Argon N-Propionyl-desmethylmuramyl-L-alanyl-D-isoglutaminyl-L-alanin-3-[2-(3-chlor-4-{pyrrol-1-yl}-phenyl)-propionylamino]-2-hydroxy-propylamid (Anomeren- und Diastereomerengemisch) als farbloses Pulver, das 2,67 Mol Wasser enthält; $[\alpha]_D^{20}$ = -10,4 ± 1,3° (c = 0,779; Wasser), $R_f$ = 0,34 (Chloroform:Methanol = 7:3), $R_f$ = 0,39 (Chloroform:Methanol:Wasser = 70:30:5).

Das Ausgangsmaterial erhält man wie folgt:

Stufe 25.1: Analog Stufe 15.1 erhält man aus 5,0 g (20,0 mMol) 2-(3-Chlor-4-{pyrrol-1-yl}-phenyl)-propionsäure, 4,54 g (22,0 mMol) N,N-Dicyclohexylcarbodiimid und 2,53 g (22,0 mMol) N-Hydroxysuccinimid in 200 ml Tetrahydrofuran unter Argon 2-(3-Chlor-4-{pyrrol-1-yl}-phenyl)-propionsäure-N-hydroxysuccinimidester; Smp. 116-117° (aus Isopropanol:Essigsäureethylester = 8:1), $R_f$ = 0,51 (Chloroform:Essigsäureethylester = 9:1), $R_f$ = 0,82 (Chloroform:Ethanol = 9:1).

Stufe 25.2: Analog Stufe 24.2 erhält man aus 1,5 g (4,87 mMol) N-tert.-Butyloxycarbonyl-L-alanin-3-amino-2-hydroxy-propylamid-hydrochlorid, das 0,56 Mol Wasser enthält, 0,68 ml (4,87 mMol) Triethylamin (Dichte 0,728) und 1,68 g (4,87 mMol) 2-(3-Chlor-4-{pyrrol-1-yl}-phenyl)-propionsäure-N-hydroxysuccinimidester in insgesamt 180 ml Tetrahydrofuran-Wasser (5:1) unter Argon N-tert.-Butyloxycarbonyl-L-alanin-3-[2-(3-chlor-4-{pyrrol-1-yl}-phenyl)-propionylamino]-2-hydroxy-propylamid als farblose Kristalle vom Smp. 75-77° (Zers.; aus Cyclohexan:Diethylether:Petrolether = 5:4:10), die 0,42 Mol Wasser enthalten; $[\alpha]_D^{20} = -7,1 \pm 1,1°$ (c = 0,928; Methylenchlorid), $R_f$ = 0,63 (Chloroform:Methanol = 4:1), $R_f$ = 0,72 (Chloroform:Methanol:Wasser = 70:30:5).

Stufe 25.3: Eine Lösung von 1,078 g (2,15 mMol) N-tert.-Butyloxycarbonyl-L-alanin-3-[2-(3-chlor-4-{pyrrol-1-yl}-phenyl)-propionylamino]-2-hydroxy-propylamid, das 0,42 Mol Wasser enthält, in 25 ml Essigsäureethylester wird bei 0° mit 25 ml einer Lösung von ca. 5 N Salzsäure in Essigsäureethylester versetzt und das Ganze anschliessend 2 Stunden bei 0° unter Argon gerührt.

Danach gibt man zu der gelblichen Lösung 50 ml Diethylether und rührt die so erhaltene Suspension 15 Minuten bei 0° bis zur Kristallisation. Die Kristalle werden abgesaugt und mit Diethylether nachgewaschen. Man erhält 0,83 g Rohprodukt, das anschliessend in 60 ml Essigsäureethylester-Diethylether (1:2) suspendiert wird. Die Suspension wird 2 Stunden bei Raumtemperatur bis zur vollständigen Kristallisation gerührt, die Kristalle abgenutscht und mit Diethylether nachgewaschen.

Man erhält L-Alanin-3-[2-(3-chlor-4-{pyrrol-1-yl}-phenyl)-propionylamino]-2-hydroxy-propylamid-hydrochlorid als farblose Kristalle, die sich ab 179° zersetzen und 0,35 Mol Wasser enthalten; $[\alpha]_D^{20} = +6,9 \pm 3,0°$ (c = 0,334; Methanol), $R_f$ = 0,32 (Chloroform:Methanol:Wasser = 70:30:5).

Beispiel 26: Zu einer Lösung von 1,148 g (2,0 mMol) N-Propionyl-desmethylmuramyl-L-alanyl-D-isoglutaminyl-L-alanin, das 0,59 Mol Wasser enthält, in 25 ml Dimethylformamid gibt man nacheinander unter Rühren bei Raumtemperatur 0,638 g (4,0 mMol) 1-Hydroxybenzotriazol (enthält ca. 10 % Wasser), 0,244 g (2,0 mMol) 4-Dimethylamino-pyridin, 1,493 g (4,0 mMol) 2-[4,5-Di-(4-methoxy-phenyl)-thiazol-2-yl-thio]-ethanol und 0,825 g (4,0 mMol) N,N-Dicyclohexylcarbodiimid.

Die anfangs klare, farblose Lösung wird 22 Stunden bei Raumtemperatur gerührt. Danach wird die so erhaltene Suspension filtriert und das Filtrat im Hochvakuum bei 30° zur Trockne eingedampft.

Man erhält das Rohprodukt als gelb gefärbten Schaum, der durch Säulenchromatographie an 400 g Kieselgel (Typ 60, Merck; 0,063-0,2 mm) im System Chloroform-Methanol-Wasser (70:30:5) gereinigt wird (10 ml Fraktionen).

Die Fraktionen 47-74 werden vereinigt und im Hochvakuum bei 30° eingedampft. Der Rückstand (0,9 g) wird in Chloroform-Methanol (15:2) gelöst und die so erhaltene Lösung mit 50 ml Diethylether versetzt. Die Suspension wird 30 Minuten bei Raumtemperatur bis zur vollständigen Kristallisation gerührt, die Kristalle abgenutscht und mit Diethylether nachgewaschen. Anschliessend werden die Kristalle in 20 ml heissem Tetrahydrofuran aufgeschlemmt, die so erhaltene Suspension auf Raumtemperatur abgekühlt und, nach Zugabe von 10 ml Diethylether, 15 Minuten bis zur vollständigen Kristallisation gerührt. Die Kristalle werden abgenutscht und mit Diethylether nachgewaschen. Man erhält 0,62 g farblose Kristalle vom Smp. 164-166° (Zers.). Diese werden unter leichtem Erwärmen in 100 ml bidestilliertem Wasser-tert.-Butanol (1:1) gelöst. Die farblose Lösung wird durch ein Milliporefilter (Fluoropore, PTFE, 0,2 µm) filtriert und im Hochvakuum lyophilisiert.

Zur Entfernung des restlichen tert.-Butanols wird das Lyophilisat nochmals in 80 ml bidestilliertem Wasser aufgenommen und erneut lyophilisiert.

Man erhält N-Propionyl-desmethylmuramyl-L-alanyl-D-isoglutaminyl-L-alanin-2-[4,5-di-(4-methoxy-phenyl)-thiazol-2-yl-thio]-ethylester (Anomerengemisch) als farbloses Pulver, das 1,99 Mol Wasser enthält; $[\alpha]_D^{20}$ = -3,0 ± 1,9° (c = 0,537; Methanol), $R_f$ = 0,47 (Chloroform:Methanol = 7:3), $R_f$ = 0,57 (Chloroform:Methanol:Wasser = 70:30:5). Die Verbindung zersetzt sich in wässeriger Lösung (Raumtemperatur, 16 Stunden: teilweise Zersetzung).

Beispiel 27: Analog Beispiel 23 hydrolysiert man 2-Phenyl-4,5-[3-O-{2-n-propyl-(E,Z)-vinyl-(1)-carbonyl-L-alanyl-D-isoglutaminyl-L-lanyl-4-(2-<2-(2,6-dichlor-phenylamino)-phenyl>-acetylmino)-butylamido}-5,6-O-isopropyliden-D-glucofurano]-$\Delta^2$-oxazolin mit 50%iger Essigsäure und erhält nach analoger Aufarbeitung N-Benzoyl-(α,β)-glucosaminyl-3-O-(2-n-propyl-(E,Z)-vinyl(1)-carbonyl-L-alanyl-D-isoglutaminyl-L-alanin-4-{2-[2-(2,6-dichlor-phenylamino)-phenyl]-acetylamino}-butylamid mit 1 Mol Kristallwasser; Smp. 152-157°, $[\alpha]_D^{20}$ = +9,7° ± 1,1° (c = 0,879; DMSO), $R_f$ = 0,39 (Chloroform:Methanol:Wasser = 85:15:1).

Das Ausgangsmaterial erhält man analog Beispiel 23 als E,Z-Gemisch; Smp. 176-179°, $[\alpha]_D^{20}$ = -8,5° ± 1,1° (c = 0,944; $CHCl_3$:MeOH = 1:1), $R_f$ (Isomer A) = 0,35 ($CHCl_3$:MeOH = 9:1), $R_f$ (Isomer B) = 0,32 ($CHCl_3$:MeOH = 9:1).

Das 2-Phenyl-4,5-[3-O-(1-ethoxycarbonyl-2-n-propyl-(E,Z)-vin-1-yl-5,6-O-isopropyliden-D-glucofurano]-$\Delta^2$-oxazolin erhält man analog Beispiel 23 durch Wittig-Horner-Reaktion aus dem entsprechenden Phosphonester mit n-Butyraldehyd als farbloses Oel;
$R_f$ = 0,38 ($CHCl_3$), $[\alpha]_D^{20}$ = -4,1 ± 0,5° (c = 2,15; $CHCl_3$).

Beispiel 28: Analog Beispiel 23 hydrolysiert man 0,5 g 2-Phenyl-4,5-[3-O-{2-p-chlor-phenyl-(E,Z)-vinyl-(1)-carbonyl-L-alanyl-D-isoglutaminyl-L-alanyl-4-(2-<2-(2,6-dichlor-phenylamino)-phenyl>-acetylamino)-butylamido}-5,6-O-isopropyliden-D-glucofurano]-$\Delta^2$-oxazolin in 20 ml 50%iger Essigsäure bei 40° 6 Stunden. Dann dampft man im Vakuum zur Trockne, verreibt den Rückstand mit 30 ml Diethylether und chromatographiert ihn an 25 g Kieselgel (Merck; 0,04-0,63 mm) mit dem Elutionsgemisch Methylenchlorid-Methanol-Wasser (90:10:0,1). Die Fraktionen mit $R_f$ = 0,39 ($CHCl_3$:MeOH:$H_2O$ = 85:15:1) werden eingedampft. Der Rückstand wird in Acetonitril-Methanol (7:3) gelöst und bis zur Trübung Diethylether zugegeben. Man erhält so Kristalle von N-Benzoyl-glucosaminyl-3-O-(2-p-chlor-phenyl-(E,Z)-vinyl-(1)-carbonyl-L-alanyl-D-isoglutaminyl-L-alanin-4-{2-[2-(2,6-dichlor-phenylamino)-phenyl]-acetylamino}-butylamid mit 1,2 Mol Kristallwasser; Smp. 157-161°, $[\alpha]_D^{20}$ = +18,8 ± 1° (c = 0,825; DMSO).
Das Ausgangsmaterial erhält man analog Beispiel 23 wie folgt:
1 g (1,946 mMol) 2-Phenyl-4,5-[3-O-(1-ethoxycarbonyl-2-p-chlor-phenyl-vinyl-(1))-5,6-O-isopropyliden-D-glucofurano]-$\Delta^2$-oxazolin verseift man mit Natronlauge in Ethanol. Das trockene $Na^+$-Salz kuppelt man analog mit 1,53 g des Trifluoracetats von L-Alanyl-D-isoglutaminyl-L-alanin-4-{2-[2-(2,6-dichlor-phenylamino)-phenyl]-acetylamino}-butylamid und 300 µl Triethylamin. Man erhält nach analoger Aufarbeitung und Chromatographie an 300 g Kieselgel mit dem Elutionsgemisch Methylenchlorid-Ethanol (95:5) aus den Fraktionen mit $R_f$ = 0,35 ($CHCl_3$:MeOH = 9:1) die E- und Z-Isomeren der Ausgangsverbindung von Beispiel 23 als Gemisch.

Die beiden Isomeren lassen sich durch nochmalige Chromatographie an einer Niederdrucksäule an Kieselgel (0,04-0,63 mm) mit Methylenchlorid-Methanol-Wasser (98:2:0,05) trennen.

Man erhält Isomer A mit Smp. 259-261°, $[\alpha]_D^{20}$ = +16,2° ± 2,9° (c = 0,34; DMSO) und $R_f$ = 0,39 ($CHCl_3$:MeOH = 9:1) sowie Isomer B mit Smp. 140-145°, $[\alpha]_D^{20}$ = +7,4° ± 1,1° (c = 0,91, DMSO) und $R_f$ = 0,36 ($CHCl_3$:MeOH = 9:1).

Das 2-Phenyl-4,5-[3-O-(1-ethoxycarbonyl-2-p-chlor-phenyl-vin-1-yl)-5,6-O-isopropyliden-D-glucofurano]-$\Delta^2$-oxazolin erhält man analog Beispiel 23 durch Wittig-Horner-Reaktion des entspechenden Phosphonesters mit p-Chlor-benzaldehyd als E,Z-Gemisch vom Smp. 54-56° und $[\alpha]_D^{20}$ = -1° ± 1° (c = 1,028; $CHCl_3$), das sich durch nochmalige Chromatographie an Kieselgel (0,04-0,63 mm) mit Methylenchlorid teilweise trennen lässt.

Man erhält die gemäss NMR-Spektrum reine E-Form mit $[\alpha]_D^{20}$ = +63,5° ± 4,1° (c = 0,241; $CHCl_3$) und $R_f$ = 0,51 ($CH_2Cl_2$: Essigsäureethylester = 98:2) sowie die Z-Form, die gemäss NMR-Spektrum noch etwa 10 % der E-Form enthält, mit $[\alpha]_D^{20}$ = -32,9° ± 0,9° (c = 1,125; $CHCl_3$). Für die reine Z-Form mit $R_f$ = 0,43 ($CH_2Cl_2$: Essigsäureethylester = 98:2) errechnet sich danach $[\alpha]_D^{20}$ = -44,8° ($CHCl_3$).

Beispiel 29: Analog Beispiel 17 erhält man aus 1,1 g (1,29 mMol; 1,175 mMol/g) N-Propionyl-desmethylmuramyl-L-alanyl-D-isoglutamin-N-hydroxysuccinimidester, 0,537 g (0,99 mMol) L-Alanin-3-{2-[1-(4-chlor-benzoyl)-5-methoxy-2-methyl-indol-3-yl]-acetylamino}-2-hydroxy-propylamid-hydrochlorid, das 0,17 Mol Wasser enthält, und 137,6 µl (0,99 mMol) Triethylamin in 30 ml Dimethylacetamid N-Propionyl-desmethylmuramyl-L-alanyl-D-isoglutaminyl-L-alanin-3-{2-[1-(4-chlor-benzoyl)-5-methoxy-2-methyl-indol-3-yl]-

acetylamino}-2-hydroxy-propylamid (Anomeren- und Diastereomerengemisch) als farbloses Pulver (Lyophilisat), das 2,96 Mol Wasser und 0,4 Mol tert.-Butanol enthält. Die Verbindung kann aus Methanol-Chloroform-Diethylether (5:3:15) kristallisiert werden;
Smp. 182-184°, $[\alpha]_D^{20}$ = -8,6 ± 2,3° (c = 0,429; Wasser), $R_f$ = 0,31 (Chloroform:Methanol = 7:3), $R_f$ = 0,70 (Chloroform:Methanol:Wasser = 70:30:5).

Das Ausgangsmaterial erhält man wie folgt:

Stufe 29.1: Analog Stufe 24.2 erhält man aus 0,893 g (2,9 mMol) N-tert.-Butyloxycarbonyl-L-alanin-3-amino-2-hydroxy-propylamid-hydrochlorid, das 0,56 Mol Wasser enthält, 403 µl (2,9 mMol) Triethylamin und 1,32 g (2,9 mMol) 1-(4-Chlor-benzoyl)-5-methoxy-2-methyl-3-indolyl-essigsäure-N-hydroxysuccinimidester in insgesamt 100 ml Tetrahydrofuran-Wasser (4:1) N-tert.-Butyloxycarbonyl-L-alanin-3-{2-[1-(4-chlor-benzoyl)-5-methoxy-2-methyl-indol-3-yl]-acetylamino}-2-hydroxy-propylamid als schwach gelb gefärbte Kristalle vom Smp. 106-108° (Zers., aus Methylenchlorid-Diethylether-Petrolether [3:4:30]), die 0,21 Mol Wasser enthalten;
$[\alpha]_D^{20}$ = -7,5 ± 1,7° (c = 0,574; Methylenchlorid), $R_f$ = 0,38 (Chloroform:Ethanol = 9:1), $R_f$ = 0,52 (Chloroform:Methanol = 9:1), $R_f$ = 0,69 (Chloroform:Methanol = 4:1).

Stufe 29.2: Analog Stufe 25.3 erhält man aus 1,44 g (2,38 mMol) N-tert.-Butyloxycarbonyl-L-alanin-3-{2-[1-(4-chlor-benzoyl)-5-methoxy-2-methyl-indol-3-yl]-acetylamino}-2-hydroxy-propylamid, das 0,21 Mol Wasser enthält, in 40 ml Essigsäureethylester mit 40 ml ca. 5 N Salzsäure in Essigsäureethylester (2,5 Stunden, 0°) L-Alanin-3-{2-[1-(4-chlor-benzoyl)-5-methoxy-2-methyl-indol-3-yl]-acetylamino}-2-hydroxy-propylamid-hydrochlorid als gelbliche Kristalle, die sich ab 153° zersetzen (aus heissem Essigsäureethylester aufgeschlämmt) und 0,17 Mol Wasser enthalten;
$[\alpha]_D^{20}$ = +5,8 ± 1,0° (c = 1,043; Methanol), $R_f$ = 0,22 (Chloroform:Methanol = 7:3).

Beispiel 30.: 1 g (2,03 mMol) L-Alanin-3-{2-[2-(3-trifluormethylphenyl-N-methyl-amino)-phenyl]-acetylamino}-2-hydroxy-propylamid-hydrochlorid und 2,185 g (3,7 mMol) N-Propionyl-desmethylmuramyl-L-alanyl-D-isoglutamin-N-hydroxy-succinimidester löst man in einer Mischung aus 25 ml N,N-Dimethylformamid und 25 ml Dimethoxyethan, gibt 0,3 ml Triethylamin zu und lässt 17 Stunden bei Raumtemperatur rühren. Man dampft bei 1,3 Pa (0,01 Torr) und 40°C zur Trockene und chromatographiert den Rückstand an 140 g Kieselgel (Merck; 0,04-0,63 mm) mit Chloroform-Methanol-Wasser (90:10:0,5) als Elutionsmittel. Man erhält nach Eindampfen der Fraktionen mit $R_f$ = 0,24 ($CHCl_3$:MeOH:$H_2O$ = 80:20:1) einen amorphen Rückstand, der mit Essigsäureethylester verrieben wird. So erhält man farbloses, kristallines α,β-N-Propionyl-desmethylmuramyl-L-alanyl-D-iso-glutaminyl-L-alanin-3-{2-[2-(3-trifluormethyl-phenyl-N-methyl-amino)-phenyl]-acetylamino}-2-hydroxy-propylamid; Smp. 135-138°, $[\alpha]_D^{20}$ = -11° ± 2° (c = 0,515; $H_2O$).

Das Ausgangsmaterial erhält man wie folgt:
980 mg 2-[2-(3-Trifluormethyl-phenyl-N-methyl-amino-phenyl]-essigsäure löst man in einer Mischung aus 10 ml Chloroform und 2 ml N,N-Dimethylformamid und versetzt mit 540 mg N-Hydroxy-benztriazol und 1,5 g N,N'-Dicyclohexylcarbodiimid. Nach einer Stunde bei Raumtemperatur gibt man bei 0° 1,1 g (3,3 mMol) N-Benzyloxy-carbonyl-L-alanin-3-amino-2-hydroxy-propylamid-hydrochlorid (siehe Stufe 15.2) und 0,43 ml Triethylamin in 5 ml Chloroform zu. Man lässt 2 Stunden bei Raumtemperatur reagieren, dampft bei 1,3 Pa (0,1 Torr) zur Trockne, nimmt mit 100 ml Methanol und 5 ml Wasser auf, stellt den pH-Wert mit Essigsäure auf 5, filtriert vom ausgefallenen Dicyclohexylharnstoff ab, dampft das Filtrat im Vakuum ein und nimmt den Rückstand mit Essigsäureethylester auf. Diese Lösung extrahiert man mit gesättigter $NaHCO_3$-Lösung und Wasser, trocknet sie mit $Na_2SO_4$ und dampft ein. Den Rückstand reinigt man durch Chromatographie an Kieselgel (Typ Cr 60, Merck) mit dem Elutionsgemisch Chloroform-Methanol (95:5). Man erhält so farbloses, kristallines N-Benzyloxycarbonyl-L-alanin-3-{2-[2-(3-trifluormethyl-phenyl-N-methyl-amino)-phenyl]-acetylamino}-2-hydroxy-propylamid; Smp. 129-130°, $R_f$ = 0,60 (Chloroform:Methanol = 9:1).

Diese Verbindung wird mit 5%iger Palladiumkohle in Methanol unter Zusatz von 1 N methanolischer Salzsäure hydriert zum Hydrochlorid des L-Alanin-3-{2-[2-(3-trifluormethyl-phenyl-N-methyl-amino)-phenyl]-acetylamino}-2-hydroxy-propylamids; $R_f$ = 0,19 (Chloroform:Methanol = 8:2).

Beispiel 31: Nach den in dieser Anmeldung besschriebenen Verfahren erhält man die folgenden Verbindungen: 2-Benzoylamino-2-desoxy-6-O-{N-<[2-(2,6-dichlor-phenyl-amino)-phenyl]-acetyl>-L-valyl}-D-glucos-3-O-yl-vinyl-1-carbonyl-L-α-aminobutyryl-D-glutaminsäure-dimethylester,
N-Acetyl-muramyl-L-alanyl-D-(γ-methoxycarbonyl)-isoglutaminyl-L-alanin-3-{2-[2-(2,6-dichlor-phenylamino)-phenyl]-acetylamino-2-hydroxy-propylamid,
N-Propionyl-desmethylmuramyl-L-alanyl-D-glutaminyl-L-alanin-2-hydroxy-3-[d-2-(6-methoxy-2-naphthyl)-propionylamino]-propylamid,
1,4,6-Tri-O-acetyl-N-propionyl-desmethylmuramyl-L-alanyl-D-glutaminyl-L-alanin-2-acetoxy-3-[d-2-(6-methoxy-2-naphthyl)-propionylamino]-propylamid oder
N-(N-Acetyl-muramyl)-O-{2-[2-(2,6-dichlor-phenyl-amino)-phenyl]-acetyl}-L-serin-D-isoglutamin.

Beispiel 32: Herstellung von 1000 Kapseln mit je 0,1 mg Wirkstoff pro Kapsel:

Zusammensetzung:

| | | |
|---|---|---|
| N-Propionyl-desmethylmuramyl-L-alanin-D-isoglutaminyl-L-alanin-3-{2-[2-(2,6-dichlor-phenyl-amino)-phenyl]-acetylamino}-2-hydroxy-propylamid | 0,1 | g |
| Talk | 72 | g |
| Weizenstärke | 48 | g |
| Magnesiumstearat | 32 | g |
| Lactose | 8 | g |
| | 160,1 | g |

Zubereitung:

Die Substanzen werden durch ein Sieb mit einer Maschenweite von 0,6 mm geschlagen und gründlich gemischt. Mit je 160,1 mg (pro Kapsel) dieser Mischung werden mit einer Kapselfüllmaschine Gelatine-Kapseln hergestellt.

Patentansprüche für die Vertragsstaaten BE, CH, DE, FR, GB, IT, LI, LU, NL und SE:

1. Zuckerderivate der Formel I,

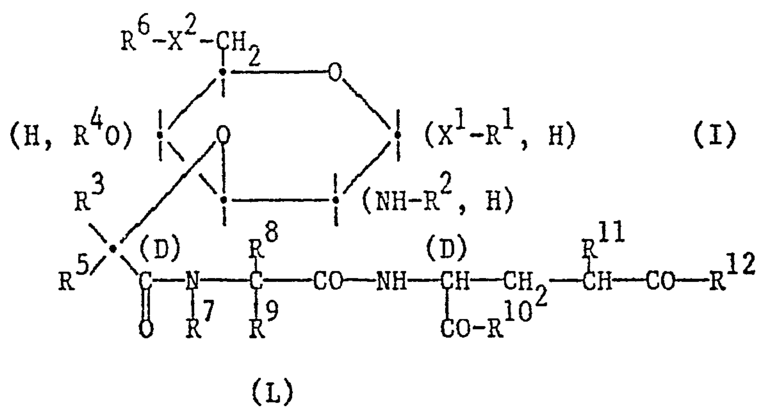

(I)

worin sich der Zuckerteil von D-Glucose, D-Mannose oder D-Galactose ableitet, $X^1$ Sauerstoff, Schwefel oder die Gruppe NH, $X^2$ Sauerstoff oder die Gruppe NH, $R^1$, $R^4$ und $R^6$ unabhängig voneinander Wasserstoff, Niederalkanoyl, einen Rest der Formel Ia,

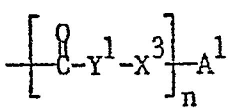

(Ia)

worin n für 0 oder 1, $Y^1$ für unsubstituiertes oder substituiertes Alkylen, das durch Carbonylimino oder Carbonyloxy unterbrochen sein kann, $X^3$ für Sauerstoff oder die Gruppe NH und $A^1$ für durch Aryl, Heteroaryl oder Heteroarylthio substituiertes Niederalkanoyl, das zusätzlich durch einen Ethylenrest, der mit dem obengenannten Aryl- oder Heteroarylsubstituenten einen fünfgliedrigen Ring bildet, substituiert sein kann, oder für ortho- oder ortho/meta-substituiertes Aroyl stehen, oder einen Rest der Formel Ib,

$$-\left[\overset{O}{\overset{\|}{C}}-Y^1\right]_n-\overset{O}{\overset{\|}{C}}-A^2 \qquad \text{(Ib)}$$

worin n und $Y^1$ die obengenannten Bedeutungen haben und $A^2$ für durch Aryl, Heteroaryl oder Heteroarylthio substituiertes Niederalkoxy steht, oder $R^1$ auch unsubstituiertes oder substituiertes Benzyl, $R^2$ unsubstituiertes oder durch Hydroxy substituiertes Niederalkanoyl, unsubstituiertes oder substituiertes Benzoyl oder einen der obengenannten Reste der Formeln Ia oder Ib, $R^3$ Wasserstoff, Niederalkyl oder Cycloalkyl und $R^5$ Wasserstoff oder $R^3$ und $R^5$ zusammen Niederalkyliden, Cycloalkyliden oder unsubstituiertes oder substituiertes Benzyliden, $R^7$ Wasserstoff oder Niederalkyl oder $R^7$ und $R^9$ zusammen Trimethylen, $R^8$ Wasserstoff oder Niederalkyl, $R^9$ Wasserstoff oder Niederalkyl, das unsubstituiert oder durch Hydroxy, Mercapto, Niederalkylthio, Carboxy, Niederalkoxycarbonyl, Carbamoyl oder einen Rest der Formel Ic, Id, Ie oder If,

$$-X^4-\left[\overset{O}{\overset{\|}{C}}-Y^1-X^3\right]_q-A^1 \qquad \text{(Ic)}$$

$$-X^4-\left[\overset{O}{\overset{\|}{C}}-Y^1\right]_q-\overset{O}{\overset{\|}{C}}-A^2 \qquad \text{(Id)}$$

$$-\overset{O}{\overset{\|}{C}}-X^5-Y^2-X^6-A^1 \qquad \text{(Ie)}$$

$$-\overset{O}{\overset{\|}{C}}-\left[X^5-Y^2-\overset{O}{\overset{\|}{C}}\right]_q-A^2 \qquad \text{(If)}$$

worin q für 0 oder 1, $X^4$ für Sauerstoff oder Schwefel und $X^5$ und $X^6$ unabhängig voneinander für Sauerstoff oder die Gruppe NH stehen und die übrigen Substituenten die obengenannten Bedeutungen haben, substituiert ist, $R^{10}$ und $R^{12}$ unabhängig voneinander Niederalkoxy, Hydroxy, Amino, Niederalkylamino, das durch Carboxy, Carbamoyl oder Niederalkoxycarbonyl substituiert ist und das zusätzlich durch

Amino, Hydroxy, Carboxy, 2-Amino-ethylthio, 2-Amino-ethoxy und/oder die Sulfogruppe $-SO_3H$ substituiert sein kann, einen Rest der Formel Ig,

$$-X^5-Y^2-X^6-A^1 \qquad (Ig)$$

worin $Y^2$ für unsubstituiertes oder substituiertes Alkylen, worin eine Methylengruppe durch Sauerstoff, Schwefel oder Sulfinyl ersetzt sein kann und das durch Carbonylimino oder Carbonyloxy unterbrochen sein kann, steht, und worin $X^5$, $X^6$ und $A^1$ die obengenannten Bedeutungen haben, oder einen Rest der Formel Ih,

$$\left[-X^5-Y^2-\overset{O}{\overset{\|}{C}}\right]_q-A^2 \qquad (Ih)$$

worin q, $X^5$, $Y^2$ und $A^2$ die obengenannten Bedeutungen haben, und $R^{11}$ Wasserstoff, Carboxy, Niederalkoxycarbonyl oder Carbamoyl bedeuten, wobei die Verbindungen der Formel I mindestens einen und höchstens drei Reste $A^1$ und/oder $A^2$ aufweisen, und Salze von solchen Verbindungen mit mindestens einer salzbildenden Gruppe.

2. Verbindungen nach Anspruch 1, worin $R^1$, $R^4$ und $R^6$ unabhängig voneinander Wasserstoff, Niederalkanoyl, einen Rest der Formel Ia,

$$-\left[\overset{O}{\overset{\|}{C}}-Y^1-X^3\right]_n-A^1 \qquad (Ia)$$

worin n für 0 oder 1, $Y^1$ für unsubstituiertes oder substituiertes Alkylen, das durch Carbonylimino oder Carbonyloxy unterbrochen sein kann, $X^3$ für Sauerstoff oder die Gruppe NH und $A^1$ für durch Aryl, Heteroaryl oder Heteroarylthio substituiertes Niederalkanoyl, das zusätzlich durch einen Ethylenrest, der mit dem obengenannten Aryl- oder Heteroarylsubstituenten einen fünfgliedrigen Ring bildet,

0163286

substituiert sein kann, oder für ortho- oder ortho/meta-substituiertes Aroyl stehen, oder einen Rest der Formel Ib,

$$-\left[\overset{O}{\overset{\|}{C}}-Y^1\right]_n-\overset{O}{\overset{\|}{C}}-A^2 \qquad \text{(Ib)}$$

worin n und $Y^1$ die obengenannten Bedeutungen haben und $A^2$ für durch Aryl, Heteroaryl oder Heteroarylthio substituiertes Niederalkoxy steht, $R^2$ unsubstituiertes oder durch Hydroxy substituiertes Niederalkanoyl oder einen der obengenannten Reste der Formeln Ia oder Ib, $R^3$ Wasserstoff, Niederalkyl oder Cycloalkyl und $R^5$ Wasserstoff oder $R^3$ und $R^5$ zusammen Niederalkyliden oder Cycloalkyliden, $R^{10}$ und $R^{12}$ unabhängig voneinander Niederalkoxy, Hydroxy, Amino, Niederalkylamino, das durch Carboxy, Carbamoyl oder Niederalkoxycarbonyl substituiert ist und das zusätzlich durch Amino, Hydroxy, Carboxy, 2-Amino-ethylthio, 2-Amino-ethoxy und/oder die Sulfogruppe $-SO_3H$ substituiert sein kann, einen Rest der Formel Ig,

$$-X^5-Y^2-X^6-A^1 \qquad \text{(Ig)}$$

worin $Y^2$ für unsubstituiertes oder substituiertes Alkylen, worin eine Methylengruppe durch Sauerstoff oder Schwefel ersetzt sein kann und das durch Carbonylimino oder Carbonyloxy unterbrochen sein kann, steht, und worin $X^5$, $X^6$ und $A^1$ die obengenannten Bedeutungen haben, oder einen Rest der Formel Ih,

$$\left[-X^5-Y^2-\overset{O}{\overset{\|}{C}}\right]_q-A^2 \qquad \text{(Ih)}$$

worin q, $X^5$, $Y^2$ und $A^2$ die obengenannten Bedeutungen haben, und $R^{11}$ Wasserstoff, Carboxy oder Carbamoyl bedeuten, und die übrigen Substituenten die in Anspruch 1 genannten Bedeutungen haben, und Salze von solchen Verbindungen mit mindestens einer salzbildenden Gruppe.

3. Verbindungen der Formel I nach Anspruch 1 oder 2, worin sich der Zuckerteil von D-Glucose ableitet, und Salze von solchen Verbindungen mit mindestens einer salzbildenden Gruppe.

4. Verbindungen der Formel I nach einem der Ansprüche 1-3, worin $A^1$ für den Acylrest einer Carbonsäure, ausgewählt aus der Gruppe, bestehend aus 6-Chlor-5-cyclohexyl-indan-1-carbonsäure (Clindanac), 2-[4,5-bis-(4-Methoxy-phenyl)-oxazol-2-yl]-propionsäure, 2-(5-Chlor-4-cyclohexyl-2-hydroxy-phenyl)-esigsäure, 2-[4,5-bis-(4-Methoxy-phenyl)-oxazol-2-yl]-2-methyl-propionsäure, 2-(3-Fluor-4-phenyl-phenyl)-propionsäure (Flurbiprofen), (±)-5-Benzoyl-3H-1,2-dihydro-pyrrolo[a]-pyrrol-1-carbonsäure, 2-[4-(1,3-Dihydro-1-oxo-2H-isoindol-2-yl)-phenyl]-propionsäure (Indoprofen), 2-{2-[(2,6-Dichlor-phenyl)-amino]-phenyl}-essigsäure (Diclofenac), 2-{2-[(2,6-Dichlor-4-fluor-phenyl)-amino]-5-fluor-phenyl}-essigsäure , 2-(2,3-Dimethyl-phenyl)-amino-benzoesäure, 2-[4,5-bis-(4-Methoxy-phenyl)-imidazol-2-yl]-2-methyl-propionsäure, 2-{2-[(2,6-Dichlor-phenyl)-amino]-5-fluor-phenyl}-essigsäure, 2-(3-Benzoyl-phenyl)-propionsäure (Ketoprofen), 2-S-[4,5-bis-(4-Methoxy-phenyl)-thiazol-2-yl]-mercaptoessigsäure, 3-S-[4,5-bis-(4-Methoxy-phenyl)-thiazol-2-yl]-mercaptopropionsäure, 2-{2-[(2,6-Dichlor-4-fluor-phenyl)-amino]-phenyl}-essigsäure, 5-(2,4-Difluor-phenyl)-2-hydroxy-benzoesäure (Diflunisal), 2-(6-Chlor-9H-carbazol-2-yl)-propionsäure (Carprofen), 2-(4-Isobutyl-phenyl)-propionsäure (Ibuprofen), 1-(4-Chlor-benzoyl)-5-methoxy-2-methyl-indol-3-yl-essigsäure (Indomethacin), 2-(6-Methoxy-naphth-2-yl)-propionsäure (Naproxen), 2-[3-Chlor-4-(3-pyrrolin-1-yl)-phenyl]-propionsäure (Pirprofen), 2-(5H-[1]benzo-pyrano[2,3-b]pyridin-7-yl)-propionsäure (Pranoprofen), 5-(4-Methyl-benzoyl)-1-methyl-pyrrol-2-yl-essigsäure (Tolmetin) und 2-[4,5-bis-(4-Methoxy-phenyl)-oxazol-2-yl]-essigsäure steht, und Salze von solchen Verbindungen mit mindestens einer salzbildenden Gruppe.

5. Verbindungen der Formel I nach einem der Ansprüche 1-4, worin $A^2$ für substituiertes Niederalkoxy, ausgewählt aus der Gruppe, bestehend aus 2-[4,5-bis-(4-Methoxy-phenyl)-thiazol-2-yl-thio]-ethoxy,

2-[4,5-bis-(4-Methoxy-phenyl)-imidazol-2-yl]-2-methyl-propoxy und 3-[4,5-bis-(4-Methoxy-phenyl)-thiazol-2-yl-thio]-propoxy, steht, und Salze von solchen Verbindungen mit mindestens einer salzbildenden Gruppe.

6. Verbindungen der Formel I nach einem der Ansprüche 1-5, worin $X^1$ und $X^2$ Sauerstoff, $R^2$ unsubstituiertes oder durch Hydroxy substituiertes $C_{2-4}$-Alkanoyl oder einen Rest der wie oben definierten Formeln Ia oder Ib, $R^3$ Wasserstoff oder Niederalkyl, $R^5$, $R^7$ und $R^8$ Wasserstoff, $R^9$ Niederalkyl, das unsubstituiert oder durch Hydroxy, Mercapto, Methylthio oder einen Rest der wie oben definierten Formeln Ic, Id, Ie oder If substituiert ist, $R^{10}$ Hydroxy oder Amino, $R^{11}$ Wasserstoff, $R^{12}$ Niederalkoxy, Hydroxy, Amino oder einen Rest der wie oben definierten Formeln Ig oder Ih bedeuten, wobei die Verbindungen der Formel I einen Rest $A^1$ oder $A^2$ aufweisen müssen, und Salze von solchen Verbindungen mit mindestens einer salzbildenden Gruppe.

7. Verbindungen der Formel I nach einem der Ansprüche 1-6, die einen Rest der Formel Ia, Ib, Ic, Id, Ie, If, Ig oder Ih aufweisen, worin $Y^1$ beziehungsweise $Y^2$ unsubstituiertes oder durch Hydroxy oder Carboxy substituiertes Alkylen mit bis zu 12 C-Atomen, das durch Carbonylimino oder Carbonyloxy unterbrochen sein kann, bedeuten, und Salze von solchen Verbindungen mit einer salzbildenden Gruppe.

8. Verbindungen der Formel I nach einem der Ansprüche 1-7, die einen Rest der Formel Ia, Ic, Ie oder Ig aufweisen, worin $A^1$ für den Acylrest von Ketoprofen, Naproxen oder Ibuprofen steht, und Salze von solchen Verbindungen mit einer salzbildenden Gruppe.

9. Verbindungen der Formel I nach einem der Ansprüche 1-7, die einen Rest der Formel Ia, Ic, Ie oder Ig aufweisen, worin $A^1$ für den Acylrest von Diclofenac steht, und Salze von solchen Verbindungen mit einer salzbildenden Gruppe.

10. Verbindungen nach einem der Ansprüche 1, 3-5 und 7-9, worin $R^3$ Wasserstoff, Niederalkyl oder, zusammen mit $R^5$, gegebenenfalls durch $C_{1-3}$-Alkyl substituiertes Methyliden oder gegebenenfalls substituiertes Benzyliden bedeutet, und Salze von solchen Verbindungen mit mindestens einer salzbildenden Gruppe.

11. Verbindungen nach einem der Ansprüche 1, 3-5 und 7-9, worin $R^2$ Benzoyl und $R^3$ zusammen mit $R^5$ unsubstituiertes oder durch $C_{1-3}$-Alkyl substituiertes Methyliden oder unsubstituiertes oder durch Halogen substituiertes Benzyliden bedeuten, und Salze von solchen Verbindungen mit mindestens einer salzbildenden Gruppe.

12. Verbindungen der Formel I nach Anspruch 1, worin sich der Zuckerteil von D-Glucose ableitet, $X^1$ und $X^2$ Sauerstoff, $R^1$ Wasserstoff oder Niederalkanoyl, $R^2$ Niederalkanoyl oder Benzoyl, $R^3$ Wasserstoff oder Niederalkyl oder zusammen mit $R^5$ einen Niederalkylidenrest, der unsubstituiert ist oder durch gegebenenfalls Halogen-substituiertes Phenyl substituiert ist, $R^4$ Wasserstoff oder Niederalkanoyl, $R^5$ Wasserstoff oder zusammen mit $R^3$ einen Niederalkylidenrest, der unsubstituiert ist oder durch gegebenenfalls Halogen-substituiertes Phenyl substituiert ist, $R^6$ Wasserstoff, Niederalkanoyl oder einen Rest der Formel Ia, worin n für 1, $Y^1$ für unsubstituiertes oder durch Carboxy substituiertes Niederalkyliden, $X^3$ für NH und $A^1$ für 2-{2-[(2,6-Dichlor-phenyl)-amino]-phenyl}-acetyl stehen, $R^7$ und $R^8$ Wasserstoff, $R^9$ Niederalkyl, $R^{10}$ Amino, Niederalkoxy oder einen Rest der Formel Ig, worin $X^5$ für NH, $Y^2$ für einen Niederalkylenrest, der durch Carbonylimino unterbrochen und durch Hydroxy substituiert sein kann, $X^6$ für NH und $A^1$ für 2-(6-Methoxy-naphth-2-yl)-propionyl stehen, $R^{11}$ Wasserstoff oder Niederalkoxycarbonyl und $R^{12}$ Niederalkoxy, Hydroxy, Amino, einen Rest der Formel Ig, worin $X^5$ für NH, $Y^2$ für unsubstituiertes oder durch Hydroxy, Niederalkanoyloxy, Amino, Carboxy und/oder Benzyloxycarbonyl substituiertes $C_{2-10}$-Alkylen, worin eine Methylengruppe durch Sauerstoff, Schwefel, Sulfinyl ($-\overset{O}{S}-$) oder Carbonylimino ($-\overset{O}{C}-NH-$) ersetzt sein kann, $X^6$ für NH oder Sauerstoff und $A^1$ für

15. 1,4,6-Tri-O-acetyl-N-propionyl-desmethylmuramyl-L-alanyl-D-isoglutaminyl-L-alanin-3-{2-[2-(2,6-dichlor-phenyl-amino)-phenyl]-acetylamino}-2-hydroxy-propylamid nach Anspruch 1.

16. N-(N-Acetyl-muramyl-L-alanyl-D-isoglutaminyl)-S-{2-(2,6-dichlor-phenyl-amino)-phenylacetylamino}-ethyl-L-cysteinbenzylester-sulfoxid nach Anspruch 1.

17. Ein pharmazeutisch verwendbares Salz einer Verbindung der Formel I mit mindestens einer salzbildenden Gruppe nach einem der Ansprüche 1-9.

18. Pharmazeutische Präparate, die eine pharmazeutisch wirksame Menge einer Verbindung der Formel I nach einem der Ansprüche 1-17 oder eines pharmazeutisch verwendbaren Salzes einer solchen Verbindung mit mindestens einer salzbildenden Gruppe zusammen mit einer signifikanten Menge eines pharmazeutischen Trägermaterials enthalten.

19. Eine Verbindung der Formel I nach einem der Ansprüche 1-17 oder ein pharmazeutisch verwendbares Salz einer solchen Verbindung mit mindestens einer salzbildenden Gruppe zur Anwendung als Arzneimittel.

20. Verwendung einer Verbindung der Formel I nach einem der Ansprüche 1-17 oder eines pharmazeutisch verwendbaren Salzes einer solchen Verbindung mit mindestens einer salzbildenden Gruppe zur Herstellung eines Arzneimittels zur Anwendung für die Stimulierung des Immunsystems eines Warmblüters.

21. Verfahren zur Herstellung einer Verbindung der Formel I nach einem der Ansprüche 1-17, oder eines Salzes einer solchen Verbindung mit mindestens einer salzbildenden Gruppe, dadurch gekennzeichnet, dass man

a) zur Herstellung einer Verbindung der Formel I, worin mindestens einer der Reste $R^{10}$ und $R^{12}$ für einen Rest der Formel Ig oder Ih steht, oder eines Salzes einer solchen Verbindung mit mindestens einer salzbildenden Gruppe eine Verbindung der Formel IV,

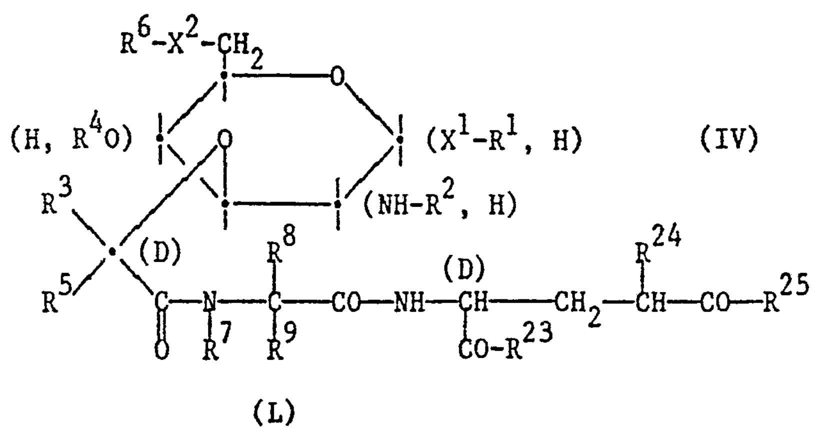

(IV)

worin mindestens einer der Reste $R^{23}$ und $R^{25}$ für Hydroxy steht und der andere der Reste $R^{23}$ und $R^{25}$ durch eine Carboxylschutzgruppe geschütztes Hydroxy, Niederalkoxy, Amino, Niederalkylamino, das durch Niederalkoxycarbonyl oder geschütztes Carboxy substituiert ist und das zusätzlich durch Amino, Hydroxy, 2-Amino-ethylthio, 2-Amino-ethoxy, geschütztes Carboxy oder eine gegebenenfalls in geschützter Form vorliegende Sulfogruppe substituiert sein kann, oder einen wie oben definierten Rest der Formel Ig oder Ih bedeutet, $R^{24}$ Wasserstoff, Carbamoyl oder geschütztes Carboxy bedeutet und die übrigen Substituenten die obengenannten Bedeutungen haben, wobei in einer Verbindung der Formel IV vorhandene Carboxy- und Aminogruppen sowie erforderlichenfalls Hydroxy- und andere funktionelle Gruppen mit Ausnahme der an der Reaktion teilnehmenden Gruppe in geschützter Form vorliegen, oder ein reaktionsfähiges Carbonsäurederivat einer Verbindung der Formel IV in einem Schritt oder stufenweise mit einer Verbindung der Formel V,

 0163286

$$H\left[-X^5-Y^2-X^7-\right]_r-A^3 \qquad (V)$$

worin $X^5$ und $Y^2$ die obengenannten Bedeutungen haben, $A^3$ die obengenannte Bedeutung von $A^1$ oder $A^2$ hat, $X^7$ die obengenannte Bedeutung von $X^6$ hat, wenn $A^3$ für $A^1$ steht, oder $X^7$ eine Carbonylgruppe bedeutet, wenn $A^3$ für $A^2$ steht, und r für 1 steht, wenn $A^3$ für $A^1$ steht, oder r die obengenannte Bedeutung von q hat, wenn $A^3$ für $A^2$ steht, oder mit einem reaktionsfähigen Derivat einer Verbindung der Formel V umsetzt und vorhandene Schutzgruppen abspaltet, oder

b) zur Herstellung einer Verbindung der Formel I, worin mindestens einer der Reste $R^1$, $R^2$, $R^4$ und $R^6$ für einen Rest der Formel Ia oder Ib steht, oder eines Salzes einer solchen Verbindung mit mindestens einer salzbildenden Gruppe eine Verbindung der Formel VI

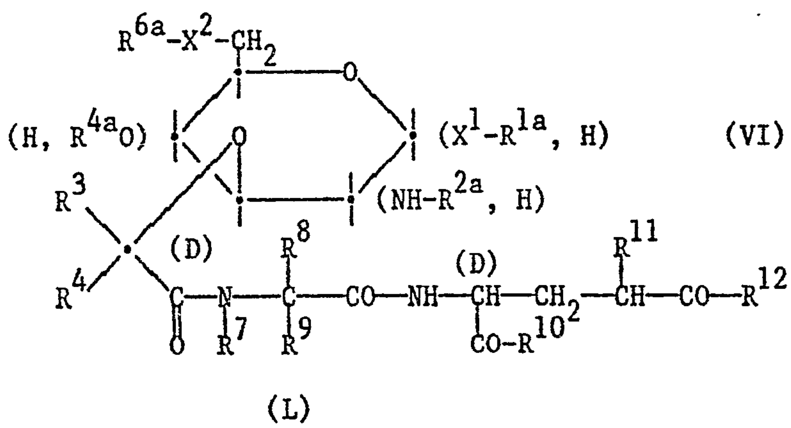

(VI)

worin mindestens einer und höchstens drei der Reste $R^{1a}$, $R^{2a}$, $R^{4a}$ und $R^{6a}$ für Wasserstoff stehen und die anderen der Reste $R^{1a}$, $R^{2a}$, $R^{4a}$ und $R^{6a}$ die obengenannten Bedeutungen der Reste $R^1$, $R^2$, $R^4$ beziehungsweise $R^6$ haben und die übrigen Substituenten die obengenannten Bedeutungen haben, wobei in einer Verbindung der Formel VI vorhandene Hydroxy, Amino oder Mercaptogruppen mit Ausnahme der an der Reaktion teilnehmenden Gruppe(n) sowie erforderlichenfalls auch

andere funktionelle Gruppen in geschützter Form vorliegen, oder ein reaktionsfähiges Derivat einer Verbindung der Formel VI in einem Schritt oder stufenweise mit einer Carbonsäure der Formel VII,

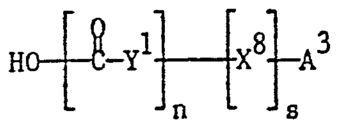

(VII)

worin $Y^1$ und n die obengenannten Bedeutungen haben, $A^3$ die obengenannte Bedeutung von $A^1$ oder $A^2$ hat, $X^8$ die obengenannte Bedeutung von $X^3$ hat, wenn $A^3$ für $A^1$ steht, oder $X^8$ eine Carbonylgruppe bedeutet, wenn $A^3$ für $A^2$ steht, und s die obengenannte Bedeutung von n hat, wenn $A^3$ für $A^1$ steht, oder s für 1 steht, wenn $A^3$ für $A^2$ steht, wobei in einer Verbindung der Formel VII vorhandene funktionelle Gruppen mit Ausnahme der an der Reaktion teilnehmenden Gruppe erforderlichenfalls in geschützter Form vorliegen, oder mit einem reaktionsfähigen Derivat einer Verbindung der Formel VII umsetzt und vorhandene Schutzgruppen abspaltet, oder

c) zur Herstellung einer Verbindung der Formel I, worin der Rest $R^9$ Niederalkyl bedeutet, das durch einen wie oben definierten Rest der Formel Ic oder Id substituiert ist, oder eines Salzes einer solchen Verbindung mit mindestens einer salzbildenden Gruppe eine Verbindung der Formel I, worin der Rest $R^9$ für durch Hydroxy oder Mercapto substituiertes Niederalkyl steht, wobei in einer Verbindung der Formel I vorhandene Hydroxy-, Mercapto- und Aminogruppen sowie, wenn erwünscht, andere funktionelle Gruppen mit Ausnahme der an der Reaktion teilnehmenden funktionellen Gruppe in geschützter Form vorliegen, oder ein reaktionsfähiges Derivat davon, in einem Schritt oder stufenweise mit einer Carbonsäure der Formel VIII,

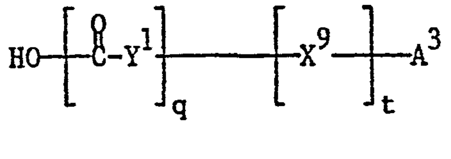

(VIII)

worin $Y^1$ und q die obengenannten Bedeutungen haben, $A^3$ die obengenannte Bedeutung von $A^1$ oder $A^2$ hat, $X^9$ die obengenannte Bedeutung von $X^3$ hat, wenn $A^3$ für $A^1$ steht, oder $X^9$ eine Carbonylgruppe bedeutet, wenn $A^3$ für $A^2$ steht, und t die obengenannte Bedeutung von q hat, wenn $A^3$ für $A^1$ steht, oder q für 1 steht, wenn $A^3$ für $A^2$ steht, wobei in einer Verbindung der Formel VIII vorhandene funktionelle Gruppen mit Ausnahme der an der Reaktion teilnehmenden Gruppe erforderlichenfalls in geschützter Form vorliegen, oder mit einem reaktionsfähigen Derivat einer Verbindung der Formel VIII umsetzt, und vorhandene Schutzgruppen abspaltet, oder

d) zur Herstellung einer Verbindung der Formel I, worin der Rest $R^9$ durch einen wie oben definierten Rest der Formeln Ie oder If substituiertes Niederalkyl bedeutet, oder eines Salzes einer solchen Verbindung mit mindestens einer salzbildenden Gruppe eine Verbindung der Formel I, worin der Rest $R^9$ durch Carboxy substituiertes Niederalkyl bedeutet, wobei in einer Verbindung der Formel I vorhandene Carboxygruppen sowie, wenn erwünscht, andere funktionelle Gruppen mit Ausnahme der an der Reaktion teilnehmenden funktionellen Gruppe in geschützter Form vorliegen, oder ein reaktionsfähiges Carbonsäurederivat davon in einem Schritt oder stufenweise mit einer Verbindung der Formel V,

$$H-\left[X^5-Y^2-X^7\right]_r-A^3 \qquad (V)$$

worin $X^5$ und $Y^2$ die obengenannten Bedeutungen haben, $A^3$ die obengenannte Bedeutung von $A^1$ oder $A^2$ hat, $X^7$ die obengenannte Bedeutung von $X^6$ hat, wenn $A^3$ für $A^1$ steht, oder $X^7$ eine Carbonylgruppe bedeutet, wenn $A^3$ für $A^2$ steht, und r für 1 steht, wenn $A^3$ für $A^1$ steht, oder r die obengenannte Bedeutung von q hat, wenn $A^3$ für $A^2$ steht, oder mit einem reaktionsfähigen Derivat einer Verbindung der Formel V umsetzt und vorhandene Schutzgruppen abspaltet, oder

e) in einer Verbindung der Formel I, worin die Substituenten die obengenannten Bedeutungen haben, wobei aber mindestens eine funktionelle Gruppe durch eine leicht abspaltbare Schutzgruppe geschützt ist, diese Schutzgruppe(n) abspaltet, oder

f) eine Verbindung der Formel X

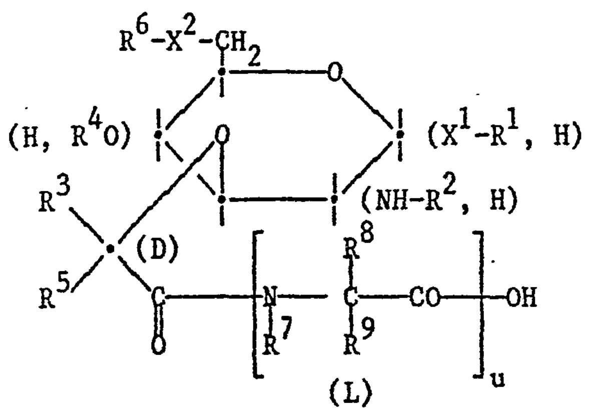

(X)

worin der Index u die untengenannte Bedeutung hat und die Substituenten die obengenannten Bedeutungen haben, wobei in einer Verbindung der Formel X vorhandene Amino- und weitere Carboxygruppen sowie erforderlichenfalls weitere funktionelle Gruppen mit Ausnahme der an der Rektion teilnehmenden Gruppe in geschützter Form vorliegen, oder ein reaktionsfähiges Säurederivat einer Verbindung der Formel X mit einer Verbindung der Formel XI,

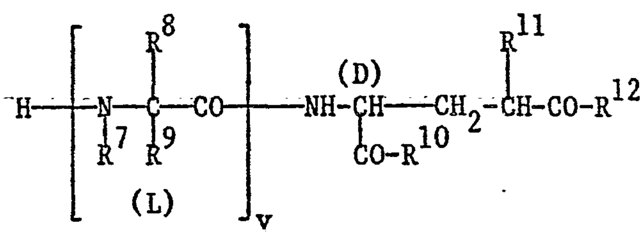

(XI)

worin der Index v für 1 steht, wenn in der Verbindung der Formel X u für 0 steht, oder worin v für 0 steht, wenn u für 1 steht, und die Substituenten die obengenannten Bedeutungen haben, wobei in einer Verbindung der Formel XI vorhandene Carboxyl- und weitere Aminogruppen sowie erforderlichenfalls weitere funktionelle Gruppen mit Ausnahme der an der Rektion teilnehmenden Gruppe in geschützter Form

vorliegen, oder mit einem reaktionsfähigen Derivat einer Verbindung der Formel XI umsetzt, und vorhandene Schutzgruppen abspaltet, oder

g) zur Herstellung einer Verbindung der Formel I, worin sich der Zuckerteil von D-Glucose ableitet, $X^1$ und $X^2$ Sauerstoff, $R^1$, $R^4$ und $R^6$ Wasserstoff und $R^2$ unsubstituiertes oder substituiertes Benzoyl bedeuten, in einer Furanoseverbindung der Formel XII,

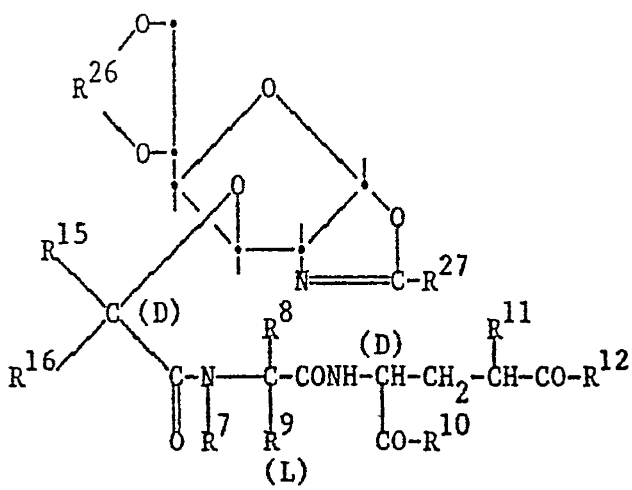

(XII)

worin $R^{26}$ eine bivalente Hydroxyschutzgruppe und $R^{27}$ unsubstituiertes oder substituiertes Phenyl bedeuten und die übrigen Substituenten die obengenannten Bedeutungen haben, den Oxazolin- und den Dioxolanring aufspaltet und nach Ausführung einer der Verfahrensvarianten a-g) zur Herstellung eines Salzes eine erhaltene Verbindung der Formel I mit mindestens einer salzbildenden Gruppe in ein Salz überführt.

22. Die nach den Verfahren des Anspruchs 21 erhältlichen Verbindungen.

23. Eine Verbindung der Formel II,

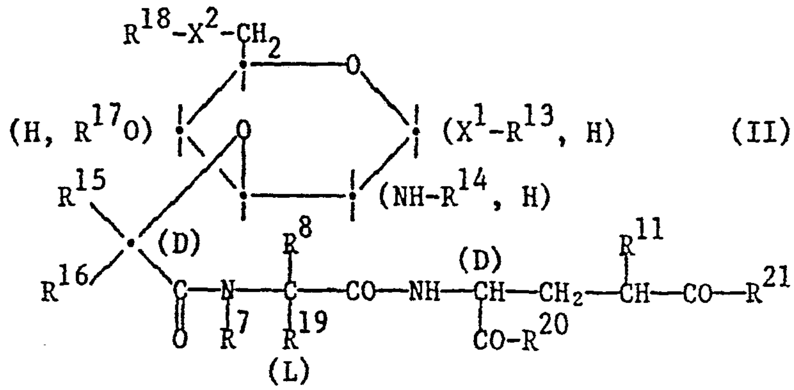

(II)

worin sich der Zuckerteil von D-Glucose, D-Mannose oder D-Galactose ableitet, $X^1$, $X^2$, $R^7$, $R^8$ und $R^{11}$ die obengenannten Bedeutungen haben, $R^{13}$, $R^{17}$ und $R^{18}$ unabhängig voneinander Wasserstoff oder Niederalkanoyl, oder $R^{13}$ auch unsubstituiertes oder substituiertes Benzyl, $R^{14}$ unsubstituiertes oder durch Hydroxy substituiertes Niederalkanoyl oder unsubstituiertes oder substituiertes Benzoyl, $R^{15}$ Cycloalkyl und $R^{16}$ Wasserstoff oder $R^{15}$ und $R^{16}$ zusammen Niederalkyliden, Cycloalkyliden oder unsubstituiertes oder substituiertes Benzyliden, $R^{19}$ Wasserstoff oder Niederalkyl, das unsubstituiert oder durch Hydroxy, Mercapto, Niederalkylthio, Carboxy, Niederalkoxycarbonyl oder Carbamoyl substituiert ist, $R^{20}$ und $R^{21}$ unabhängig voneinander Niederalkoxy, Hydroxy, Amino oder Niederalkylamino, das durch Carboxy, Carbamoyl oder Niederalkoxycarbonyl substituiert ist und das zusätzlich durch Amino, Hydroxy, Carboxy, 2-Amino-ethylthio, 2-Amino-ethoxy und/oder die Sulfogruppe $-SO_3H$ substituiert sein kann, bedeuten, oder ein Salz einer solchen Verbindung mit mindestens einer salzbildenden Gruppe.

24. Eine Verbindung nach Anspruch 23, worin $R^{13}$, $R^{17}$ und $R^{18}$ unabhängig voneinander Wasserstoff oder Niederalkanoyl, $R^{14}$ unsubstituiertes oder durch Hydroxy substituiertes Niederalkanoyl, $R^{15}$ Cycloalkyl und $R^{16}$ Wasserstoff oder $R^{15}$ und $R^{16}$ zusammen Niederalkyliden bedeuten, oder ein Salz einer solchen Verbindung mit mindestens einer salzbildenden Gruppe.

 0163286

Patentansprüche für den Vertragsstaat AT:

1. Verfahren zur Herstellung eines Zuckerderivats der Formel I,

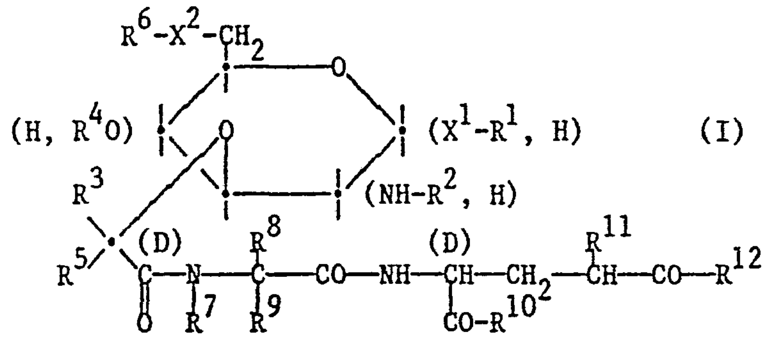

(I)

(L)

worin sich der Zuckerteil von D-Glucose, D-Mannose oder D-Galactose ableitet, $X^1$ Sauerstoff, Schwefel oder die Gruppe NH, $X^2$ Sauerstoff oder die Gruppe NH, $R^1$, $R^4$ und $R^6$ unabhängig voneinander Wasserstoff, Niederalkanoyl, einen Rest der Formel Ia,

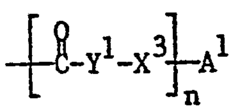

(Ia)

worin n für 0 oder 1, $Y^1$ für unsubstituiertes oder substituiertes Alkylen, das durch Carbonylimino oder Carbonyloxy unterbrochen sein kann, $X^3$ für Sauerstoff oder die Gruppe NH und $A^1$ für durch Aryl, Heteroaryl oder Heteroarylthio substituiertes Niederalkanoyl, das zusätzlich durch einen Ethylenrest, der mit dem obengenannten Aryl- oder Heteroarylsubstituenten einen fünfgliedrigen Ring bildet, substituiert sein kann, oder für ortho- oder ortho/meta-substituiertes Aroyl stehen, oder einen Rest der Formel Ib,

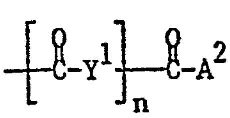

(Ib)

worin n und $Y^1$ die obengenannten Bedeutungen haben und $A^2$ für durch Aryl, Heteroaryl oder Heteroarylthio substituiertes Niederalkoxy steht, oder $R^1$ auch unsubstituiertes oder substituiertes Benzyl, $R^2$ unsubstituiertes oder durch Hydroxy substituiertes Niederalkanoyl, unsubstituiertes oder substituiertes Benzoyl oder einen der obengenannten Reste der Formeln Ia oder Ib, $R^3$ Wasserstoff, Niederalkyl oder Cycloalkyl und $R^5$ Wasserstoff oder $R^3$ und $R^5$ zusammen Niederalkyliden, Cycloalkyliden oder unsubstituiertes oder substituiertes Benzyliden, $R^7$ Wasserstoff oder Niederalkyl oder $R^7$ und $R^9$ zusammen Trimethylen, $R^8$ Wasserstoff oder Niederalkyl, $R^9$ Wasserstoff oder Niederalkyl, das unsubstituiert oder durch Hydroxy, Mercapto, Niederalkylthio, Carboxy, Niederalkoxycarbonyl, Carbamoyl oder einen Rest der Formel Ic, Id, Ie oder If,

$$-X^4-\left[\overset{O}{\overset{\|}{C}}-Y^1-X^3\right]_q-A^1 \qquad (Ic)$$

$$-X^4-\left[\overset{O}{\overset{\|}{C}}-Y^1\right]_q-\overset{O}{\overset{\|}{C}}-A^2 \qquad (Id)$$

$$-\overset{O}{\overset{\|}{C}}-X^5-Y^2-X^6-A^1 \qquad (Ie)$$

$$-\overset{O}{\overset{\|}{C}}-\left[X^5-Y^2-\overset{O}{\overset{\|}{C}}\right]_q-A^2 \qquad (If)$$

worin q für 0 oder 1, $X^4$ für Sauerstoff oder Schwefel und $X^5$ und $X^6$ unabhängig voneinander für Sauerstoff oder die Gruppe NH stehen und die übrigen Substituenten die obengenannten Bedeutungen haben, substituiert ist, $R^{10}$ und $R^{12}$ unabhängig voneinander Niederalkoxy, Hydroxy, Amino, Niederalkylamino, das durch Carboxy, Carbamoyl oder Niederalkoxycarbonyl substituiert ist und das zusätzlich durch Amino, Hydroxy, Carboxy, 2-Amino-ethylthio, 2-Amino-ethoxy und/oder die Sulfogruppe $-SO_3H$ substituiert sein kann, einen Rest der Formel Ig,

$$-X^5-Y^2-X^6-A^1 \qquad (Ig)$$

worin $Y^2$ für unsubstituiertes oder substituiertes Alkylen, worin eine Methylengruppe durch Sauerstoff, Schwefel oder Sulfinyl ersetzt sein kann und das durch Carbonylimino oder Carbonyloxy unterbrochen sein kann, steht, und worin $X^5$, $X^6$ und $A^1$ die obengenannten Bedeutungen haben, oder einen Rest der Formel Ih,

$$\left[-X^5-Y^2-\overset{O}{\overset{\|}{C}}\right]_q-A^2 \qquad \text{(Ih)}$$

worin q, $X^5$, $Y^2$ und $A^2$ die obengenannten Bedeutungen haben, und $R^{11}$ Wasserstoff, Carboxy, Niederalkoxycarbonyl oder Carbamoyl bedeuten, wobei die Verbindungen der Formel I mindestens einen und höchstens drei Reste $A^1$ und/oder $A^2$ aufweisen, oder eines Salzes einer solchen Verbindung mit mindestens einer salzbildenden Gruppe, dadurch gekennzeichnet, dass man

a) zur Herstellung einer Verbindung der Formel I, worin mindestens einer der Reste $R^{10}$ und $R^{12}$ für einen Rest der Formel Ig oder Ih steht, oder eines Salzes einer solchen Verbindung mit mindestens einer salzbildenden Gruppe eine Verbindung der Formel IV,

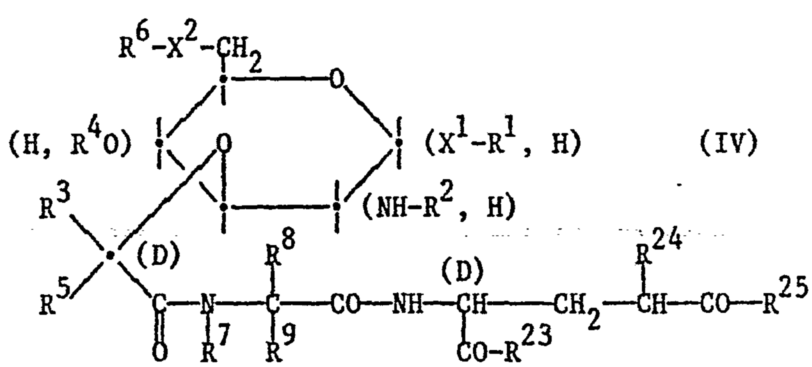

(IV)

(L)

worin mindestens einer der Reste $R^{23}$ und $R^{25}$ für Hydroxy steht und der andere der Reste $R^{23}$ und $R^{25}$ durch eine Carboxylschutzgruppe geschütztes Hydroxy, Niederalkoxy, Amino, Niederalkylamino, das durch Niederalkoxycarbonyl oder geschütztes Carboxy substituiert ist

und das zusätzlich durch Amino, Hydroxy, 2-Amino-ethylthio, 2-Amino-ethoxy, geschütztes Carboxy oder eine gegebenenfalls in geschützter Form vorliegende Sulfogruppe substituiert sein kann, oder einen wie oben definierten Rest der Formel Ig oder Ih bedeutet, $R^{24}$ Wasserstoff, Carbamoyl oder geschütztes Carboxy bedeutet und die übrigen Substituenten die obengenannten Bedeutungen haben, wobei in einer Verbindung der Formel IV vorhandene Carboxy- und Aminogruppen sowie erforderlichenfalls Hydroxy- und andere funktionelle Gruppen mit Ausnahme der an der Reaktion teilnehmenden Gruppe in geschützter Form vorliegen, oder ein reaktionsfähiges Carbonsäurederivat einer Verbindung der Formel IV in einem Schritt oder stufenweise mit einer Verbindung der Formel V,

$$H-\left[-X^5-Y^2-X^7-\right]_r-A^3 \qquad (V)$$

worin $X^5$ und $Y^2$ die obengenannten Bedeutungen haben, $A^3$ die obengenannte Bedeutung von $A^1$ oder $A^2$ hat, $X^7$ die obengenannte Bedeutung von $X^6$ hat, wenn $A^3$ für $A^1$ steht, oder $X^7$ eine Carbonylgruppe bedeutet, wenn $A^3$ für $A^2$ steht, und r für 1 steht, wenn $A^3$ für $A^1$ steht, oder r die obengenannte Bedeutung von q hat, wenn $A^3$ für $A^2$ steht, oder mit einem reaktionsfähigen Derivat einer Verbindung der Formel V umsetzt und vorhandene Schutzgruppen abspaltet, oder

b) zur Herstellung einer Verbindung der Formel I, worin mindestens einer der Reste $R^1$, $R^2$, $R^4$ und $R^6$ für einen Rest der Formel Ia oder Ib steht, oder eines Salzes einer solchen Verbindung mit mindestens einer salzbildenden Gruppe eine Verbindung der Formel VI

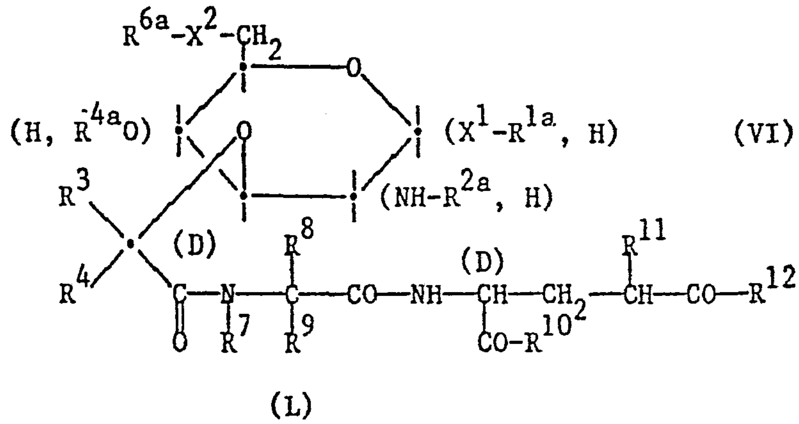

(VI)

worin mindestens einer und höchstens drei der Reste $R^{1a}$, $R^{2a}$, $R^{4a}$ und $R^{6a}$ für Wasserstoff stehen und die anderen der Reste $R^{1a}$, $R^{2a}$, $R^{4a}$ und $R^{6a}$ die obengenannten Bedeutungen der Reste $R^1$, $R^2$, $R^4$ beziehungsweise $R^6$ haben und die übrigen Substituenten die obengenannten Bedeutungen haben, wobei in einer Verbindung der Formel VI vorhandene Hydroxy, Amino oder Mercaptogruppen mit Ausnahme der an der Reaktion teilnehmenden Gruppe(n) sowie erforderlichenfalls auch andere funktionelle Gruppen in geschützter Form vorliegen, oder ein reaktionsfähiges Derivat einer Verbindung der Formel VI in einem Schritt oder stufenweise mit einer Carbonsäure der Formel VII,

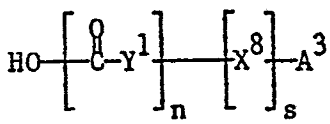

(VII)

worin $Y^1$ und n die obengenannten Bedeutungen haben, $A^3$ die obengenannte Bedeutung von $A^1$ oder $A^2$ hat, $X^8$ die obengenannte Bedeutung von $X^3$ hat, wenn $A^3$ für $A^1$ steht, oder $X^8$ eine Carbonylgruppe bedeutet, wenn $A^3$ für $A^2$ steht, und s die obengenannte Bedeutung von n hat, wenn $A^3$ für $A^1$ steht, oder s für 1 steht, wenn $A^3$ für $A^2$ steht, wobei in einer Verbindung der Formel VII vorhandene funktionelle Gruppen mit Ausnahme der an der Reaktion teilnehmenden Gruppe erforderlichenfalls in geschützter Form vorliegen, oder mit einem reaktionsfähigen Derivat einer Verbindung der Formel VII umsetzt und vorhandene Schutzgruppen abspaltet, oder

0163286

c) zur Herstellung einer Verbindung der Formel I, worin der Rest $R^9$ Niederalkyl bedeutet, das durch einen wie oben definierten Rest der Formel Ic oder Id substituiert ist, oder eines Salzes einer solchen Verbindung mit mindestens einer salzbildenden Gruppe eine Verbindung der Formel I, worin der Rest $R^9$ für durch Hydroxy oder Mercapto substituiertes Niederalkyl steht, wobei in einer Verbindung der Formel I vorhandene Hydroxy-, Mercapto- und Aminogruppen sowie, wenn erwünscht, andere funktionelle Gruppen mit Ausnahme der an der Reaktion teilnehmenden funktionellen Gruppe in geschützter Form vorliegen, oder ein reaktionsfähiges Derivat davon, in einem Schritt oder stufenweise mit einer Carbonsäure der Formel VIII,

$$HO-\left[\overset{O}{\overset{\|}{C}}-Y^1\right]_q-\left[-X^9-\right]_t-A^3 \qquad \text{(VIII)}$$

worin $Y^1$ und q die obengenannten Bedeutungen haben, $A^3$ die obengenannte Bedeutung von $A^1$ oder $A^2$ hat, $X^9$ die obengenannte Bedeutung von $X^3$ hat, wenn $A^3$ für $A^1$ steht, oder $X^9$ eine Carbonylgruppe bedeutet, wenn $A^3$ für $A^2$ steht, und t die obengenannte Bedeutung von q hat, wenn $A^3$ für $A^1$ steht, oder q für 1 steht, wenn $A^3$ für $A^2$ steht, wobei in einer Verbindung der Formel VIII vorhandene funktionelle Gruppen mit Ausnahme der an der Reaktion teilnehmenden Gruppe erforderlichenfalls in geschützter Form vorliegen, oder mit einem reaktionsfähigen Derivat einer Verbindung der Formel VIII umsetzt, und vorhandene Schutzgruppen abspaltet, oder

d) zur Herstellung einer Verbindung der Formel I, worin der Rest $R^9$ durch einen wie oben definierten Rest der Formeln Ie oder If substituiertes Niederalkyl bedeutet, oder eines Salzes einer solchen Verbindung mit mindestens einer salzbildenden Gruppe eine Verbindung der Formel I, worin der Rest $R^9$ durch Carboxy substituiertes Niederalkyl bedeutet, wobei in einer Verbindung der Formel I vorhandene Carboxygruppen sowie, wenn erwünscht, andere funktionelle

Gruppen mit Ausnahme der an der Reaktion teilnehmenden funktionellen Gruppe in geschützter Form vorliegen, oder ein reaktionsfähiges Carbonsäurederivat davon in einem Schritt oder stufenweise mit einer Verbindung der Formel V,

$$H-\left[-X^5-Y^2-X^7-\right]_r-A^3 \qquad (V)$$

worin $X^5$ und $Y^2$ die obengenannten Bedeutungen haben, $A^3$ die obengenannte Bedeutung von $A^1$ oder $A^2$ hat, $X^7$ die obengenannte Bedeutung von $X^6$ hat, wenn $A^3$ für $A^1$ steht, oder $X^7$ eine Carbonylgruppe bedeutet, wenn $A^3$ für $A^2$ steht, und r für 1 steht, wenn $A^3$ für $A^1$ steht, oder r die obengenannte Bedeutung von q hat, wenn $A^3$ für $A^2$ steht, oder mit einem reaktionsfähigen Derivat einer Verbindung der Formel V umsetzt und vorhandene Schutzgruppen abspaltet, oder

e) in einer Verbindung der Formel I, worin die Substituenten die obengenannten Bedeutungen haben, wobei aber mindestens eine funktionelle Gruppe durch eine leicht abspaltbare Schutzgruppe geschützt ist, diese Schutzgruppe(n) abspaltet, oder

f) eine Verbindung der Formel X

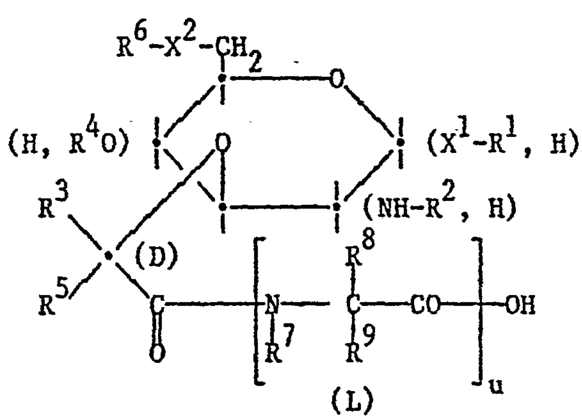

(X)

 

worin der Index u die untengenannte Bedeutung hat und die Substituenten die obengenannten Bedeutungen haben, wobei in einer Verbindung der Formel X vorhandene Amino- und weitere Carboxygruppen sowie erforderlichenfalls weitere funktionelle Gruppen mit Ausnahme der an der Rektion teilnehmenden Gruppe in geschützter Form vorliegen, oder ein reaktionsfähiges Säurederivat einer Verbindung der Formel X mit einer Verbindung der Formel XI,

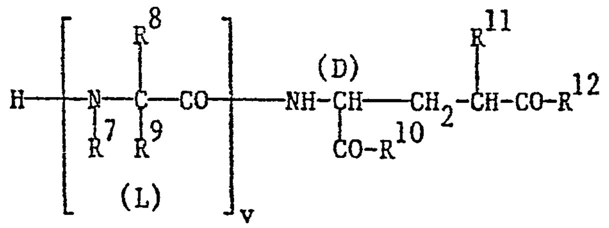

(XI)

worin der Index v für 1 steht, wenn in der Verbindung der Formel X u für 0 steht, oder worin v für 0 steht, wenn u für 1 steht, und die Substituenten die obengenannten Bedeutungen haben, wobei in einer Verbindung der Formel XI vorhandene Carboxyl- und weitere Aminogruppen sowie erforderlichenfalls weitere funktionelle Gruppen mit Ausnahme der an der Rektion teilnehmenden Gruppe in geschützter Form vorliegen, oder mit einem reaktionsfähigen Derivat einer Verbindung der Formel XI umsetzt, und vorhandene Schutzgruppen abspaltet, oder

g) zur Herstellung einer Verbindung der Formel I, worin sich der Zuckerteil von D-Glucose ableitet, $X^1$ und $X^2$ Sauerstoff, $R^1$, $R^4$ und $R^6$ Wasserstoff und $R^2$ unsubstituiertes oder substituiertes Benzoyl bedeuten, in einer Furanoseverbindung der Formel XII,

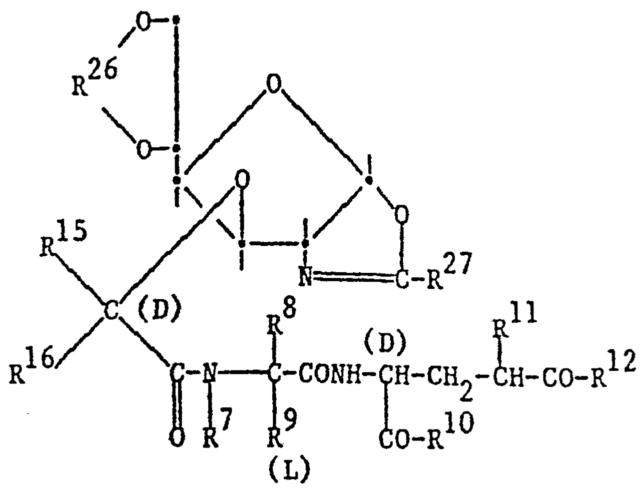

(XII)

worin $R^{26}$ eine bivalente Hydroxyschutzgruppe und $R^{27}$ unsubstituiertes oder substituiertes Phenyl bedeuten und die übrigen Substituenten die obengenannten Bedeutungen haben, den Oxazolin- und den Dioxolanring aufspaltet und nach Ausführung einer der Verfahrensvarianten a-g) zur Herstellung eines Salzes eine erhaltene Verbindung der Formel I mit mindestens einer salzbildenden Gruppe in ein Salz überführt.

2. Verfahren nach Anspruch 1, dadurch gekennzeichnet, dass man die Ausgangsstoffe so wählt, dass man eine Verbindung der Formel I, worin $R^1$, $R^4$ und $R^6$ unabhängig voneinander Wasserstoff, Niederalkanoyl, einen Rest der Formel Ia,

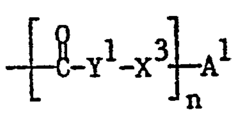

(Ia)

worin n für 0 oder 1, $Y^1$ für unsubstituiertes oder substituiertes Alkylen, das durch Carbonylimino oder Carbonyloxy unterbrochen sein kann, $X^3$ für Sauerstoff oder die Gruppe NH und $A^1$ für durch Aryl, Heteroaryl oder Heteroarylthio substituiertes Niederalkanoyl, das zusätzlich durch einen Ethylenrest, der mit dem obengenannten

Aryl- oder Heteroarylsubstituenten einen fünfgliedrigen Ring bildet, substituiert sein kann, oder für ortho- oder ortho/meta-substituiertes Aroyl stehen, oder einen Rest der Formel Ib,

$$-\left[\overset{O}{\overset{\|}{C}}-Y^1\right]_n-\overset{O}{\overset{\|}{C}}-A^2 \qquad \text{(Ib)}$$

worin n und $Y^1$ die obengenannten Bedeutungen haben und $A^2$ für durch Aryl, Heteroaryl oder Heteroarylthio substituiertes Niederalkoxy steht, $R^2$ unsubstituiertes oder durch Hydroxy substituiertes Niederalkanoyl oder einen der obengenannten Reste der Formeln Ia oder Ib, $R^3$ Wasserstoff, Niederalkyl oder Cycloalkyl und $R^5$ Wasserstoff oder $R^3$ und $R^5$ zusammen Niederalkyliden oder Cycloalkyliden, $R^{10}$ und $R^{12}$ unabhängig voneinander Niederalkoxy, Hydroxy, Amino, Niederalkylamino, das durch Carboxy, Carbamoyl oder Niederalkoxycarbonyl substituiert ist und das zusätzlich durch Amino, Hydroxy, Carboxy, 2-Amino-ethylthio, 2-Amino-ethoxy und/oder die Sulfogruppe $-SO_3H$ substituiert sein kann, einen Rest der Formel Ig,

$$-X^5-Y^2-X^6-A^1 \qquad \text{(Ig)}$$

worin $Y^2$ für unsubstituiertes oder substituiertes Alkylen, worin eine Methylengruppe durch Sauerstoff oder Schwefel ersetzt sein kann und das durch Carbonylimino oder Carbonyloxy unterbrochen sein kann, steht, und worin $X^5$, $X^6$ und $A^1$ die obengenannten Bedeutungen haben, oder einen Rest der Formel Ih,

$$\left[-X^5-Y^2-\overset{O}{\overset{\|}{C}}\right]_q-A^2 \qquad \text{(Ih)}$$

worin q, $X^5$, $Y^2$ und $A^2$ die obengenannten Bedeutungen haben, und $R^{11}$ Wasserstoff, Carboxy oder Carbamoyl bedeuten, und die übrigen Substituenten die in Anspruch 1 genannten Bedeutungen haben, oder

ein Salz einer solchen Verbindung mit mindestens einer salzbildenden Gruppe herstellt.

3. Verfahren nach Anspruch 1 oder 2, dadurch gekennzeichnet, dass man die Ausgangsstoffe so wählt, dass man eine Verbindung der Formel I, worin sich der Zuckerteil von D-Glucose ableitet, oder ein Salz einer solchen Verbindung mit mindestens einer salzbildenden Gruppe herstellt.

4. Verfahren nach einem der Ansprüche 1-3, dadurch gekennzeichnet, dass man die Ausgangsstoffe so wählt, dass man eine Verbindung der Formel I, worin $A^1$ für den Acylrest einer Carbonsäure, ausgewählt aus der Gruppe, bestehend aus 6-Chlor-5-cyclohexyl-indan-1-carbonsäure (Clindanac), 2-[4,5-bis-(4-Methoxy-phenyl)-oxazol-2-yl]-propionsäure, 2-(5-Chlor-4-cyclohexyl-2-hydroxy-phenyl)-esigsäure, 2-[4,5-bis-(4-Methoxy-phenyl)-oxazol-2-yl]-2-methyl-propionsäure, 2-(3-Fluor-4-phenyl-phenyl)-propionsäure (Flurbiprofen), (±)-5-Benzoyl-3H-1,2-dihydrorrolo[a]-pyrrol-1-carbonsäure, 2-[4-(1,3-Dihydro-1-oxo-2H-isoindol-2-yl)-phenyl]-propionsäure (Indoprofen), 2-{2-[(2,6-Dichlor-phenyl)-amino]-phenyl}-essigsäure (Diclofenac), 2-{2-[(2,6-Dichlor-4-fluor-phenyl)-amino]-5-fluor-phenyl}-essigsäure, 2-(2,3-Dimethyl-phenyl)-amino-benzoesäure, 2-[4,5-bis-(4-Methoxy-phenyl)-imidazol-2-yl]-2-methyl-propionsäure, 2-{2-[(2,6-Dichlor-phenyl)-amino]-5-fluor-phenyl}-essigsäure, 2-(3-Benzoyl-phenyl)-propionsäure (Ketoprofen), 2-S-[4,5-bis-(4-Methoxy-phenyl)-thiazol-2-yl]-mercaptossigsäure, 3-S-[4,5-bis-(4-Methoxy-phenyl)-thiazol-2-yl]mercaptopropionsäure, 2-{2-[(2,6-Dichlor-4-fluor-phenyl)-amino]-phenyl}-essigäure, 5-(2,4-Difluor-phenyl)-2-hydroxy-benzoesäure (Diflunisal), 2-(6-Chlor-9H-carbazol-2-yl)-propionsäure (Carprofen), 2-(4-Isobutyl-phenyl)-propionsäure (Ibuprofen), 1-(4-Chlor-benzoyl)-5-methoxy-2-methyl-indol-3-yl-essigsäure (Indomethacin), 2-(6-Methoxy-naphth-2-yl)-propionsäure (Naproxen), 2-[3-Chlor-4-(3-pyrrolin-1-yl)-phenyl]-propionsäure (Pirprofen), 2-(5H-[1]benzopyrano[2,3-b]pyridin-7-yl)-propionsäure (Pranoprofen), 5-(4-Methyl-benzoyl)-1-methyl-pyrrol-2-yl-essigsäure (Tolmetin) und

2-[4,5-bis-(4-Methoxy-phenyl)-oxazol-2-yl]-essigsäure steht, oder ein Salz einer solchen Verbindung mit mindestens einer salzbildenden Gruppe herstellt.

5. Verfahren nach einem der Ansprüche 1-4, dadurch gekennzeichnet, dass man die Ausgangsstoffe so wählt, dass man eine Verbindung der Formel I, worin $A^2$ für substituiertes Niederalkoxy, ausgewählt aus der Gruppe, bestehend aus 2-[4,5-bis-(4-Methoxy-phenyl)-thiazol-2-yl-thio]-ethoxy, 2-[4,5-bis-(4-Methoxy-phenyl)-imidazol-2-yl]-2-methyl-propoxy und 3-[4,5-bis-(4-Methoxy-phenyl)-thiazol-2-yl-thio]-propoxy, steht, oder ein Salz einer solchen Verbindung mit mindestens einer salzbildenden Gruppe herstellt.

6. Verfahren nach einem der Ansprüche 1-5, dadurch gekennzeichnet, dass man die Ausgangsstoffe so wählt, dass man eine Verbindung der Formel I, worin $X^1$ und $X^2$ Sauerstoff, $R^2$ unsubstituiertes oder durch Hydroxy substituiertes $C_{2-4}$-Alkanoyl oder einen Rest der wie oben definierten Formeln Ia oder Ib, $R^3$ Wasserstoff oder Niederalkyl, $R^5$, $R^7$ und $R^8$ Wasserstoff, $R^9$ Niederalkyl, das unsubstituiert oder durch Hydroxy, Mercapto, Methylthio oder einen Rest der wie oben definierten Formeln Ic, Id, Ie oder If substituiert ist, $R^{10}$ Hydroxy oder Amino, $R^{11}$ Wasserstoff, $R^{12}$ Niederalkoxy, Hydroxy, Amino oder einen Rest der wie oben definierten Formeln Ig oder Ih bedeuten, wobei die Verbindungen der Formel I einen Rest $A^1$ oder $A^2$ aufweisen müssen, oder ein Salz einer solchen Verbindung mit mindestens einer salzbildenden Gruppe herstellt.

7. Verfahren nach einem der Ansprüche 1-6, dadurch gekennzeichnet, dass man die Ausgangsstoffe so wählt, dass man eine Verbindung der Formel I, die einen Rest der Formel Ia, Ib, Ic, Id, Ie, If, Ig oder Ih aufweist, worin $Y^1$ beziehungsweise $Y^2$ unsubstituiertes oder durch Hydroxy oder Carboxy substituiertes Alkylen mit bis zu 12 C-Atomen, das durch Carbonylimino oder Carbonyloxy unterbrochen sein kann, bedeuten, oder ein Salz einer solchen Verbindung mit einer salzbildenden Gruppe herstellt.

8. Verfahren nach einem der Ansprüche 1-7, dadurch gekennzeichnet, dass man die Ausgangsstoffe so wählt, dass man eine Verbindung der Formel I, die einen Rest der Formel Ia, Ic, Ie oder Ig aufweist, worin $A^1$ für den Acylrest von Ketoprofen, Naproxen oder Ibuprofen steht, oder ein Salz einer solchen Verbindung mit einer salzbildenden Gruppe herstellt.

9. Verfahren nach einem der Ansprüche 1-7, dadurch gekennzeichnet, dass man die Ausgangsstoffe so wählt, dass man eine Verbindung der Formel I, die einen Rest der Formel Ia, Ic, Ie oder Ig aufweist, worin $A^1$ für den Acylrest von Diclofenac steht, oder ein Salz einer solchen Verbindung mit einer salzbildenden Gruppe herstellt.

10. Verfahren nach einem der Ansprüche 1, 3-5 und 7-9, dadurch gekennzeichnet, dass man die Ausgangsstoffe so wählt, dass man eine Verbindung der Formel I, worin $R^3$ Wasserstoff, Niederalkyl oder, zusammen mit $R^5$, gegebenenfalls durch $C_{1-3}$-Alkyl substituiertes Methyliden oder gegebenenfalls substituiertes Benzyliden bedeutet, oder ein Salz einer solchen Verbindung mit mindestens einer salzbildenden Gruppe herstellt.

11. Verfahren nach einem der Ansprüche 1, 3-5 und 7-9, dadurch gekennzeichnet, dass man die Ausgangsstoffe so wählt, dass man eine Verbindung der Formel I, worin $R^2$ Benzoyl und $R^3$ zusammen mit $R^5$ unsubstituiertes oder durch $C_{1-3}$-Alkyl substituiertes Methyliden oder unsubstituiertes oder durch Halogen substituiertes Benzyliden bedeuten, oder ein Salz einer solchen Verbindung mit mindestens einer salzbildenden Gruppe herstellt.

12. Verfahren nach Anspruch 1, dadurch gekennzeichnet, dass man die Ausgangsstoffe so wählt, dass man eine Verbindung der Formel I, worin sich der Zuckerteil von D-Glucose ableitet, $X^1$ und $X^2$ Sauerstoff, $R^1$ Wasserstoff oder Niederalkanoyl, $R^2$ Niederalkanoyl oder Benzoyl, $R^3$ Wasserstoff oder Niederalkyl oder zusammen mit $R^5$ einen Niederalkylidenrest, der unsubstituiert ist oder durch gegebenenfalls Halogen-substituiertes Phenyl substituiert ist, $R^4$ Wasserstoff

oder Niederalkanoyl, $R^5$ Wasserstoff oder zusammen mit $R^3$ einen Niederalkylidenrest, der unsubstituiert ist oder durch gegebenenfalls Halogen-substituiertes Phenyl substituiert ist, $R^6$ Wasserstoff, Niederalkanoyl oder einen Rest der Formel Ia, worin n für 1, $Y^1$ für unsubstituiertes oder durch Carboxy substituiertes Niederalkyliden, $X^3$ für NH und $A^1$ für 2-{2-[(2,6-Dichlor-phenyl)-amino]-phenyl}-acetyl stehen, $R^7$ und $R^8$ Wasserstoff, $R^9$ Niederalkyl, $R^{10}$ Amino, Niederalkoxy oder einen Rest der Formel Ig, worin $X^5$ für NH, $Y^2$ für einen Niederalkylenrest, der durch Carbonylimino unterbrochen und durch Hydroxy substituiert sein kann, $X^6$ für NH und $A^1$ für 2-(6-Methoxy-naphth-2-yl)-propionyl stehen, $R^{11}$ Wasserstoff oder Niederalkoxycarbonyl und $R^{12}$ Niederalkoxy, Hydroxy, Amino, einen Rest der Formel Ig, worin $X^5$ für NH, $Y^2$ für unsubstituiertes oder durch Hydroxy, Niederalkanoyloxy, Amino, Carboxy und/oder Benzyloxycarbonyl substituiertes $C_{2-10}$-Alkylen, worin eine Methylengruppe durch Sauerstoff, Schwefel, Sulfinyl ($-\overset{O}{\overset{\|}{S}}-$) oder Carbonylimino ($-\overset{O}{\overset{\|}{C}}-NH-$) ersetzt sein kann, $X^6$ für NH oder Sauerstoff und $A^1$ für 2-{2-[(2,6-Dichlor-phenyl)-amino]-phenyl}-acetyl, 1-Benzoyl-5-methoxy-2-methyl-indol-3-yl-acetyl, 2-(6-Methoxy-naphth-2-yl)-propionyl, 2-(4-Isobutyl-phenyl)-propionyl, 2-[3-(Hydroxybenzyl)-phenyl]-propionyl, 2-(3-Benzoyl-phenyl)-propionyl, 2-[3-Chlor-4-(pyrrol-1-yl)-phenyl]-propionyl, 1-(4-Chlor-benzoyl)-5-methoxy-2-methyl-indol-3-yl-acetyl, 2-[3-Chlor-4-(3-pyrrolin-1-yl)-phenyl]-propionyl oder 2-[4,5-bis-(4-Methoxy-phenyl)-oxazol-2-yl]-propionyl stehen, oder einen Rest der Formel Ih bedeuten, worin q für 1, $X^5$ für NH, $Y^2$ für Niederalkylen und $A^2$ für 2-[4,5-bis-(4-Methoxy-phenyl)-thiazol-2-yl-thio]-ethoxy stehen, mit der Massgabe, dass die Verbindung einen und nur einen Rest $A^1$ oder $A^2$ enthält, oder ein pharmazeutisch verwendbares Salz einer solchen Verbindung mit mindestens einer salzbildenden Gruppe herstellt.

13. Verfahren nach Anspruch 1 oder 12, dadurch gekennzeichnet, dass man die Ausgangsstoffe so wählt, dass man eine Verbindung der Formel I, worin $Y^1$ 2-Carboxy-ethyliden oder 2-Methyl-propyliden oder $Y^2$ Di- oder Tetramethylen, Ethyliden, 1-Carboxy-dimethylen, 1-Carboxy-tetramethylen, 1-Carboxy-pentamethylen, 2-Hydroxy-trimethylen,

1-Carboxy-3-oxa-pentamethylen $(-\underset{COOH}{CH}-CH_2-O-CH_2-CH_2-)$, 1-Benzyloxycarbonyl-3-thiapentamethylen, 4-(Ethylidencarbonylimino)-n-butyl $[-CH(CH_3)-CONH-(CH_2)_4-]$, 3-(Ethylidencarbonylimino)-2-hydroxy-propyl, 2-Acetoxy-3-(ethylidencarbonylimino)-propyl, 3-[(4-Amino-pentyliden)-carbonylimino]-2-hydroxy-propyl oder 1-Benzyloxycarbonyl-2-(ethylen-1-sulfinyl)-dimethylen$(-\underset{COO-CH_2-C_6H_5}{CH}-CH_2-\overset{O}{S}-CH_2-CH_2-)$

bedeuten, oder ein pharmazeutisch verwendbares Salz einer solchen Verbindung mit mindestens einer salzbildenden Gruppe herstellt.

14. Verfahren nach Anspruch 1, dadurch gekennzeichnet, dass man die Ausgangsstoffe so wählt, dass man N-Propionyl-desmethylmuramyl-L-alanyl-D-isoglutaminyl-L-alanin-3-{2-[2-(2,6-dichlor-phenyl-amino)-phenyl]-acetylamio}-2-hydroxy-propylamid herstellt.

15. Verfahren nach Anspruch 1, dadurch gekennzeichnet, dass man die Ausgangsstoffe so wählt, dass man 1,4,6-Tri-O-acetyl-N-propionyl-desmethylmuramyl-L-alanyl-D-isoglutaminyl-L-alanin-3-{2-[2-(2,6-dichlor-phenyl-amino)-phenyl]-acetylamino}-2-hydroxy-propylamid herstellt.

16. Verfahren nach Anspruch 1, dadurch gekennzeichnet, dass man die Ausgangsstoffe so wählt, dass man N-(N-Acetyl-muramyl-L-alanyl-D-isoglutaminyl)-S-{2-(2,6-dichlor-phenyl-amino)-phenylacetylamino}-ethyl-L-cysteinbenzylester-sulfoxid herstellt.

FO 7.4/VBU/jt*